# EUROPEAN PATENT SPECIFICATION

(11) **EP 3 443 300 B1**
(45) Date of publication and mention of the grant of the patent: **14.10.2026**
(21) Application number: 17783028.8
(22) Date of filing: 12.04.2017
(51) Int. Cl.: A61B 5/11, G01C 21/16, G01C 21/20, G01S 5/02, H04W 4/02, H04W 4/33

(54) **METHODS, APPARATUS AND SYSTEMS FOR OBJECT TRACKING**
VERFAHREN, VORRICHTUNG UND SYSTEME ZUR OBJEKTVERFOLGUNG
PROCÉDÉS, APPAREIL ET SYSTÈMES POUR LE SUIVI D'OBJETS

(30) Priority: 14.04.2016 US 201662322575 P; 10.05.2016 US 201662334110 P; 18.10.2016 US 201662409796 P
(43) Date of publication of application: 20.02.2019
(73) Proprietor: ORIGIN RESEARCH WIRELESS, INC., Rockville, MD 20852 (US)
(72) Inventor: ZHANG, Feng, Greenbelt, MD 20770 (US); CHEN, Chen, College Park, MD 20740 (US); HAN, Yi, Ellicott City, MD 21042 (US); WANG, Beibei, Clarksville, MD 21029 (US); LAI, Hung-Quoc, Duc, Parkville, MD 21234 (US); WU, Zhung-han, Laurel, MD 20707 (US); CHEN, Chun-I, Brookeville, MD 20833 (US); LIU, K. J. Ray, Potomac, MD 20854 (US)
(74) Representative: Epping - Hermann - Fischer
(86) International application number: PCT/US2017/027131
(87) International publication number: WO 2017/180698

(56) References cited:
- US-A1- 2006 025 229
- US-A1- 2010 109 903
- US-A1- 2013 034 130
- US-A1- 2014 232 380
- US-A1- 2015 234 033
- YI WANG ET AL: "Robust Indoor Human Activity Recognition Using Wireless Signals", SENSORS, vol. 15, no. 7, 15 July 2015 (2015-07-15), pages 17195 - 17208, XP055614919, DOI: 10.3390/s150717195
- WEI WANG ET AL: "Understanding and Modeling of WiFi Signal Based Human Activity Recognition", PROCEEDINGS OF THE 21ST ANNUAL INTERNATIONAL CONFERENCE ON MOBILE COMPUTING AND NETWORKING, MOBICOM '15, 1 January 2015 (2015-01-01), New York, New York, USA, pages 65 - 76, XP055314705, ISBN: 978-1-4503-3619-2, DOI: 10.1145/2789168.2790093
- QIAN KUN ET AL: "PADS: Passive detection of moving targets with dynamic speed using PHY layer information", 2014 20TH IEEE INTERNATIONAL CONFERENCE ON PARALLEL AND DISTRIBUTED SYSTEMS (ICPADS), IEEE, 16 December 2014 (2014-12-16), pages 1 - 8, XP032771421, DOI: 10.1109/PADSW.2014.7097784
- MANIKANTA KOTARU ET AL: "SpotFi", SPECIAL INTEREST GROUP ON DATA COMMUNICATION, ACM, 2 PENN PLAZA, SUITE 701 NEW YORK NY 10121-0701 USA, 17 August 2015 (2015-08-17), pages 269 - 282, XP058071191, ISBN: 978-1-4503-3542-3, DOI: 10.1145/2785956.2787487
- KAISHUN WU ET AL: "CSI-Based Indoor Localization", IEEE TRANSACTIONS ON PARALLEL AND DISTRIBUTED SYSTEMS, IEEE SERVICE CENTER, LOS ALAMITOS, CA, US, vol. 24, no. 7, 1 July 2013 (2013-07-01), pages 1300 - 1309, XP011511152, ISSN: 1045-9219, DOI: 10.1109/TPDS.2012.214
- ZHENG YANG ET AL: "From RSSI to CSI", ACM COMPUTING SURVEYS, ACM, NEW YORK, NY, US, US, vol. 46, no. 2, 27 December 2013 (2013-12-27), pages 1 - 32, XP058036548, ISSN: 0360-0300, DOI: 10.1145/2543581.2543592
- ZHOU ZIMU ET AL: "On Multipath Link Characterization and Adaptation for Device-Free Human Detection", PROCEEDINGS OF THE INTERNATIONAL CONFERENCE ON DISTRIBUTED COMPUTING SYSTEMS, IEEE COMPUTER SOCIETY, US, 29 June 2015 (2015-06-29), pages 389 - 398, XP033180510, ISSN: 1063-6927, [retrieved on 20150722], DOI: 10.1109/ICDCS.2015.47
- WU ZHUNG-HAN ET AL: "A Time-Reversal Paradigm for Indoor Positioning System", IEEE TRANSACTIONS ON VEHICULAR TECHNOLOGY, IEEE SERVICE CENTER, PISCATAWAY, NJ, US, vol. 64, no. 4, 1 April 2015 (2015-04-01), pages 1331 - 1339, XP011578352, ISSN: 0018-9545, [retrieved on 20150414], DOI: 10.1109/TVT.2015.2397437
- KUN QIAN ET AL: "Decimeter level passive tracking with wifi", HOT TOPICS IN WIRELESS, ACM, 2 PENN PLAZA, SUITE 701 NEW YORK NY 10121-0701 USA, 3 October 2016 (2016-10-03), pages 44 - 48, XP058280212, ISBN: 978-1-4503-4251-3, DOI: 10.1145/2980115.2980131

## Description

### TECHNICAL FIELD

The present teaching generally relates to object tracking. More specifically, the present teaching relates to object tracking based on time-reversal technology in a rich-scattering environment, e.g., an indoor environment or urban metropolitan area.

### BACKGROUND

Indoor location-based service is becoming more and more important nowadays. One popular approach is to use dead-reckoning method to estimate the location of a moving object in real time. Usually, the moving direction and the moving distance are estimated by inertia measurement unit (IMU). However, the performance of moving distance estimation in the dead-reckoning based approach is far from satisfactory, which is the main reason that such indoor navigation systems are still not popular now.

Estimating the speed of a moving object in an indoor environment, which can assist the location-based service, is also an open problem and no satisfactory results appear yet. The Doppler effect has been widely applied to different speed estimation systems using sound wave, microwave, or laser light. However, low speed such as human walking speed is very difficult to be estimated using Doppler shift, especially using electromagnetic (EM) waves. This is because the maximum Doppler shift is about ${Δ}_{f} = \frac{v}{c} {f}_{0}$ where *f*₀ is the carrier frequency of the transmitted signal and *v* is the human walking speed. Under normal human walking speed *v*=5.0 km/h and *f*₀ =5.8 GHz, Δ*_{f}* is around 26.85 Hz and it is extremely difficult to estimate this tiny amount with high accuracy. In addition, these methods need line-of-sight condition and perform poorly in a complex indoor environment with rich multipath reflections.

Most of the existing speed estimation methods that work well for outdoor environments fail to offer satisfactory performance for indoor environments, since the direct path signal is disturbed by the multipath signal in indoor environments and the time-of-arrival (or Doppler shift) of the direct path signal cannot be estimated accurately. Then, researchers focus on the estimation of the maximum Doppler frequency which can be used to estimate the moving speed. Various methods have been proposed, such as level crossing rate methods, covariance based methods, and wavelet based methods. However, these estimators provide results that are unsatisfactory because the statistics used in these estimators have a large variance and are location-dependent in practical scenarios. For example, the accuracy of one existing speed estimation method can only differentiate whether a mobile station moves with a fast speed (above30 km/h) or with a slow speed (below 5 km/h).

Another kind of indoor speed estimation method based on the traditional pedestrian dead reckoning algorithm is to use accelerometers to detect steps and to estimate the step length. However, pedestrians often have different stride lengths that may vary up to 40% at the same speed, and 50% with various speeds of the same person. Thus, calibration is required to obtain the average stride lengths for different individuals, which is impractical in real applications and thus has not been widely adopted.
YI WANG ET AL, "Robust Indoor Human Activity Recognition Using Wireless Signals", SENSORS, vol. 15, no. 7, pages 17195 - 17208, doi:10.3390/s150717195, provides robust indoor human activity recognition using wireless signals.
US 2015/234033 describes a method and apparatus for determining the location of a mobile device using multiple wireless access points, each wireless access point comprising multiple antennas. WEI WANG ET AL, "Understanding and Modeling of WiFi Signal Based Human Activity Recognition", PROCEEDINGS OF THE 21ST ANNUAL INTERNATIONAL CONFERENCE ON MOBILE COMPUTING AND NETWORKING, doi:10.1145/2789168.2790093, describes understanding and modeling of WiFi signal based human activity recognition.

### SUMMARY

The present teaching generally relates to object tracking. More specifically, the present teaching relates to object tracking based on time-reversal technology in a rich-scattering environment, e.g., an indoor environment or urban metropolitan area. The invention is defined by the appended claims.

In one example, a method for tracking a movement of an object in real-time is disclosed. The method may be implemented on a machine including at least a processor and a memory communicatively coupled with the processor. The method may comprise: obtaining an initial position of the object prior to a movement of the object; obtaining at least one wireless signal from a multipath channel that is impacted by the movement of the object; extracting a time series of channel state information (CSI) for the multipath channel from the at least one wireless signal; determining a distance of the movement of the object based on the time series of CSI; estimating a direction of the movement of the object; and determining a new position of the object after the movement based on the distance, the direction, and the initial position.

In another example, a method for tracking real-time position of an elevator is disclosed. The method may be implemented on a machine including at least a processor and a memory communicatively coupled with the processor. The method may comprise: obtaining a first output from a measurement unit that is coupled to the elevator such that the measurement unit has a fixed position relative to the elevator, wherein the first output represents a raw estimate of acceleration of the elevator; obtaining a second output from the measurement unit, wherein the second output represents a measurement of gravity at a same location as the elevator; calculating an acceleration of the elevator in vertical direction in a current time slot based on the first output and the second output; obtaining a previous speed of the elevator in vertical direction calculated in a previous time slot; and determining whether the elevator is moving based on the acceleration and the previous speed.

In yet another example, a system for tracking a movement of an object in real-time is disclosed. The system may comprise: a receiver configured for receiving at least one wireless signal from a multipath channel that is impacted by a movement of the object; a processor; and a memory communicatively coupled with the processor. The processor is configured for: obtaining an initial position of the object prior to the movement of the object; extracting a time series of CSI for the multipath channel from the at least one wireless signal; determining a distance of the movement of the object based on the time series of CSI; estimating a direction of the movement of the object; and determining a new position of the object after the movement based on the distance, the direction, and the initial position.

In still another example, a system for tracking real-time position of an elevator is disclosed. The system may comprise: a measurement unit that is coupled to the elevator such that the measurement unit has a fixed position relative to the elevator, wherein the measurement unit is configured for generating a first output representing a raw estimate of acceleration of the elevator, and generating a second output representing a measurement of gravity at a same location as the elevator; a processor; and a memory communicatively coupled with the processor, wherein the processor is configured for: calculating an acceleration of the elevator in vertical direction in a current time slot based on the first output and the second output, obtaining a speed of the elevator in vertical direction calculated in a previous time slot, and determining whether the elevator is moving based on the acceleration and the speed.

In a different example, a system for detecting object motion in a venue is disclosed. The system may comprise: a transmitter configured for transmitting at least one wireless signal; a receiver configured for receiving the at least one wireless signal that can be impacted by object motion in the venue; a processor; and a memory communicatively coupled with the processor. The processor is configured for: extracting one or more time series of CSI from the at least one wireless signal, calculating a statistical value based on the one or more time series of CSI, wherein the statistical value represents a degree of object motion in the venue, and determining whether object motion is present in the venue based on the statistical value.

In a different example, a system for tracking a status of a door of an elevator is disclosed. The system may comprise: a transmitter configured for transmitting at least one wireless signal; a receiver configured for receiving the at least one wireless signal that can be impacted by a status of the door, wherein at least one of the transmitter and the receiver is located within the elevator; a processor; and a memory communicatively coupled with the processor. The processor is configured for: obtaining a time series of signal measurements based on the at least one wireless signal, filtering the time series of signal measurements by mitigating outliers and noisy measurements to generate a plurality of filtered measurement values each of which is associated with a corresponding time slot, and determining whether the door of the elevator is closed or open in each time slot based on the filtered measurement value associated with the time slot and a threshold.

In another example, a method for determining a minimum bandwidth needed for a TR-based system is disclosed. The method may be implemented on a machine including at least a processor and a memory communicatively coupled with the processor. The method may comprise: determining an application associated with the TR-based system, wherein the application is selected from a plurality of applications including at least one of: tracking a movement of an object in real-time, tracking real-time position of an elevator, detecting object motion in a venue, tracking a status of a door of an elevator, and a TR-based communication; when the application is determined to be a TR-based communication, determining the minimum bandwidth needed for the TR-based system based on a bandwidth that maximizes spectral efficiency of the TR-based system; and when the application is determined not to be a TR-based communication, determining the minimum bandwidth needed for the TR-based system based on a quantity of antennas in the TR-based system and based on one or more features related to the application.

Other examples in the present teaching may include systems, methods, medium, devices, servers, and other implementations directed to object tracking based on time-reversal technology in a rich-scattering environment.

Other concepts relate to software for implementing the present teaching on exploring computation, storage, application, or processing of object tracking based on time-reversal technology in a rich-scattering environment. A software product, in accord with this concept, includes at least one machine-readable non-transitory medium and information carried by the medium. The information carried by the medium may be executable program code data, parameters in association with the executable program code, and/or information related to a user, a request, content, or information related to a social group, etc.

Additional novel features will be set forth in part in the description which follows, and in part will become apparent to those skilled in the art upon examination of the following and the accompanying drawings or may be learned by production or operation of the examples. The novel features of the present teachings may be realized and attained by practice or use of various aspects of the methodologies, instrumentalities and combinations set forth in the detailed examples discussed below.

### BRIEF DESCRIPTION OF DRAWINGS

FIG. 1A shows an exemplary application for TR-based object tracking, according to an embodiment of the present teaching;
FIG. 1B shows another exemplary application for TR-based object tracking, according to an embodiment of the present teaching;
FIG. 1C shows an exemplary diagram showing general implementation of the object tracking, according to an embodiment of the present teaching;
FIG. 2 shows an exemplary schematic diagram of the time-reversal transmission scheme, according to an embodiment of the present teaching;
FIG. 3 shows an exemplary spatial Time Reversal Resonating Strength (TRRS) distribution around the focal spot, according to an embodiment of the present teaching;
FIG. 4 shows an exemplary temporal normalized receive signal distribution of the focal spot, according to an embodiment of the present teaching;
FIG. 5 shows a typical indoor environment where the channel impulse responses (CIRs) were collected, according to an embodiment of the present teaching;
FIG. 6 shows an exemplary empirical Cumulative Distribution Function (CDF) of the real and imaginary part of CIR taps, according to an embodiment of the present teaching;
FIG. 7 shows an exemplary exponential decay of the normalized gain of each tap in CIR, according to an embodiment of the present teaching;
FIG. 8 shows an exemplary sample correlation coefficient matrix between different taps from two CIRs with varying distance d, according to an embodiment of the present teaching;
FIG. 9 shows an exemplary TRRS decay with respect to distance to the focal spot, according to an embodiment of the present teaching;
FIG. 10 shows exemplary values of spatial decay deviation metric over 55 different locations when D=2cm and the corresponding empirical CDF, according to an embodiment of the present teaching;
FIG. 11 shows exemplary empirical CDFs of spatial decay deviation metric with various D, according to an embodiment of the present teaching;
FIG. 12 shows an exemplary distribution of estimated distances compared with the actual distance, according to an embodiment of the present teaching;
FIG. 13 shows the average of exemplary TR spatial resonating decay functions with varying effective bandwidth using 802.11n Wi-Fi system, according to an embodiment of the present teaching;
FIG. 14 shows an illustration of variances of the TR spatial resonating decay function with varying effective bandwidth, according to an embodiment of the present teaching;
FIG. 15 shows an illustration of the polar coordinates in the analysis. Each Multipath Component (MPC) is represented by its total traveled distance, direction of arrival, and the power gain, according to an embodiment of the present teaching;
FIG. 16 shows the comparison between the theoretical TRRS decay curve and experiment measurements, according to an embodiment of the present teaching;
FIG. 17 shows an illustration of the TRRS decay over time when the transmitter or the receiver is moving, according to an embodiment of the present teaching;
FIG. 18 shows an illustration of direction estimation based on TRRS, according to an embodiment of the present teaching;
FIG. 19 shows an illustration of rotation estimation based on TRRS from multiple antennas, according to an embodiment of the present teaching;
FIG. 20 shows an illustration of translational displacement estimation based on TRRS from multiple antennas, according to an embodiment of the present teaching;
FIG. 21 is a flow chart showing an exemplary process of object tracking, where the estimation of moving direction is based on IMU, according to an embodiment of the present teaching;
FIG. 22 is a flow chart showing an exemplary process of object tracking, where the estimation of moving direction is based on TRRS decay pattern across different transmitter/receiver (TX/RX) antennas, according to an embodiment of the present teaching;
FIG. 23 shows an exemplary fusion of different sensors' output for moving direction estimation, according to an embodiment of the present teaching;
FIG. 24A shows an illustration of gyroscope output vector projection in the direction of gravity g, according to an embodiment of the present teaching;
FIG. 24B shows an illustration of magnetic sensor output vector projection in horizontal plane, according to an embodiment of the present teaching;
FIG. 25A shows an exemplary algorithm of sensor output fusion for moving direction estimation, according to an embodiment of the present teaching;
FIG. 25B shows an exemplary correlation pattern of outputs from different sensors, according to an embodiment of the present teaching;
FIG. 26 shows an exemplary diagram of connection between various components in an object tracking system with one Origin and one Bot, according to an embodiment of the present teaching;
FIG. 27A shows an exemplary architecture of multiple-Bot tracking using uplink sounding sent from the Bots, according to an embodiment of the present teaching;
FIG. 27B shows an exemplary architecture of multiple-Bot tracking using downlink sounding sent from the Origin, according to an embodiment of the present teaching;
FIG. 28 shows an exemplary diagram of connection between various components in an object tracking system with multiple Origins and multiple Bots, according to an embodiment of the present teaching;
FIG. 29 shows a flowchart of an exemplary software implementation of the object tracking system, according to an embodiment of the present teaching;
FIG. 30 shows the schematic diagram of the elevator tracking algorithm, according to an embodiment of the present teaching;
FIG. 31 shows an exemplary experiment result of elevator tracking module in a typical building, according to an embodiment of the present teaching;

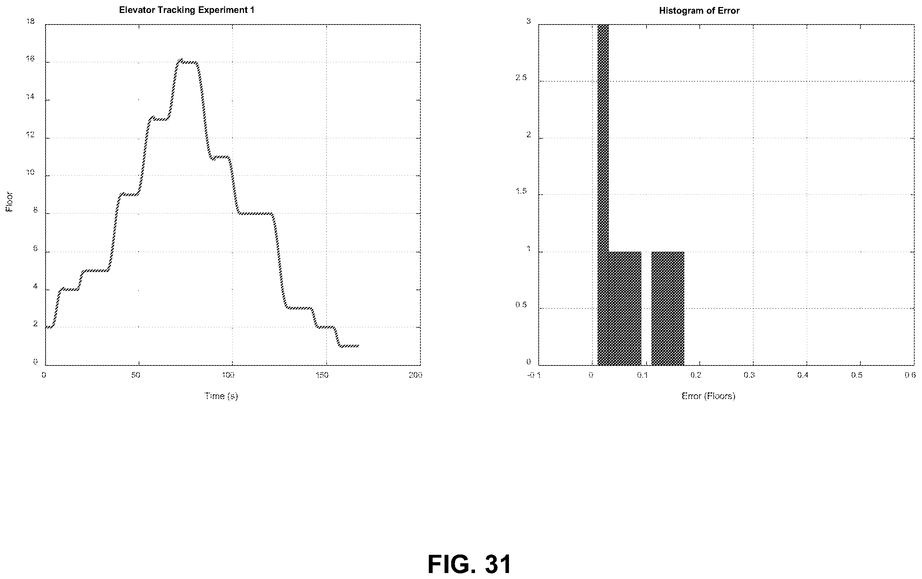

FIG. 31 shows another exemplary experiment result of elevator tracking module in a typical building, according to an embodiment of the present teaching;

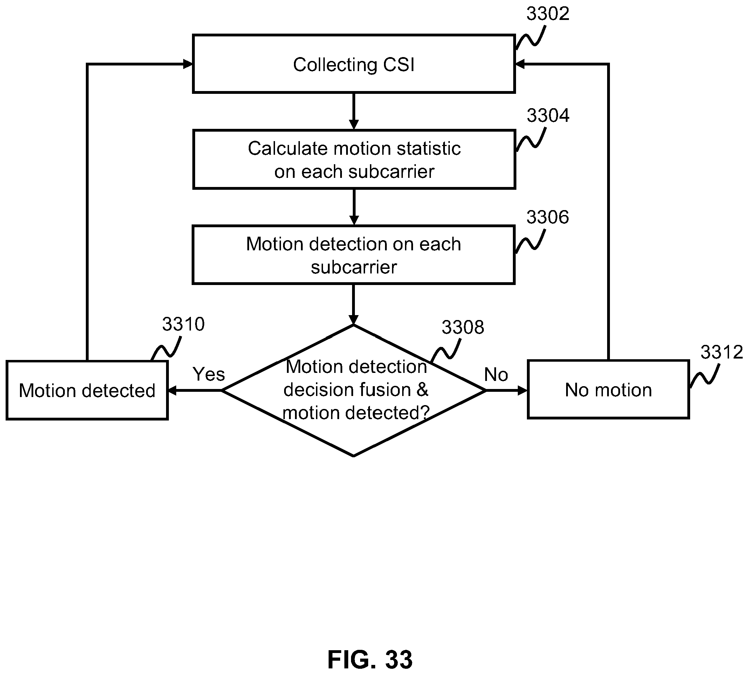

FIG. 33 shows an exemplary schematic diagram of the motion detector, according to an embodiment of the present teaching;

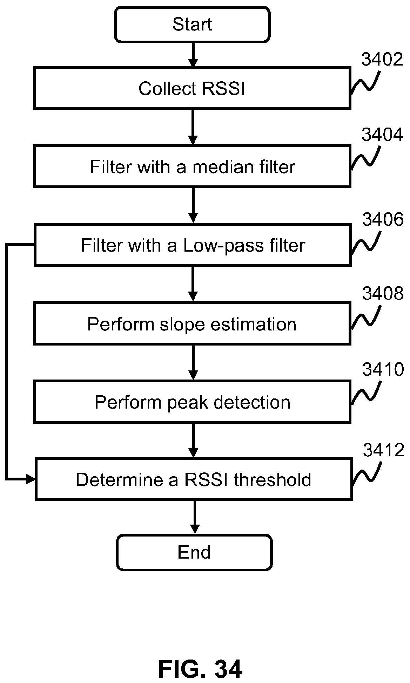

FIG. 34 shows an exemplary schematic diagram of the training procedure for the elevator door detection algorithm, according to an embodiment of the present teaching;

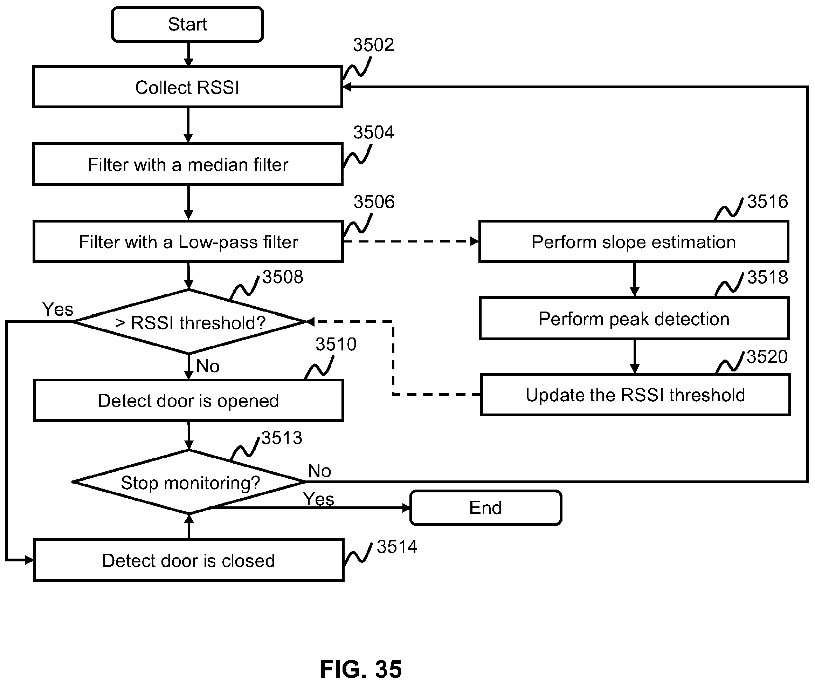

FIG. 35 shows an exemplary schematic diagram of the real-time monitoring procedure for the elevator door detection algorithm, according to an embodiment of the present teaching;

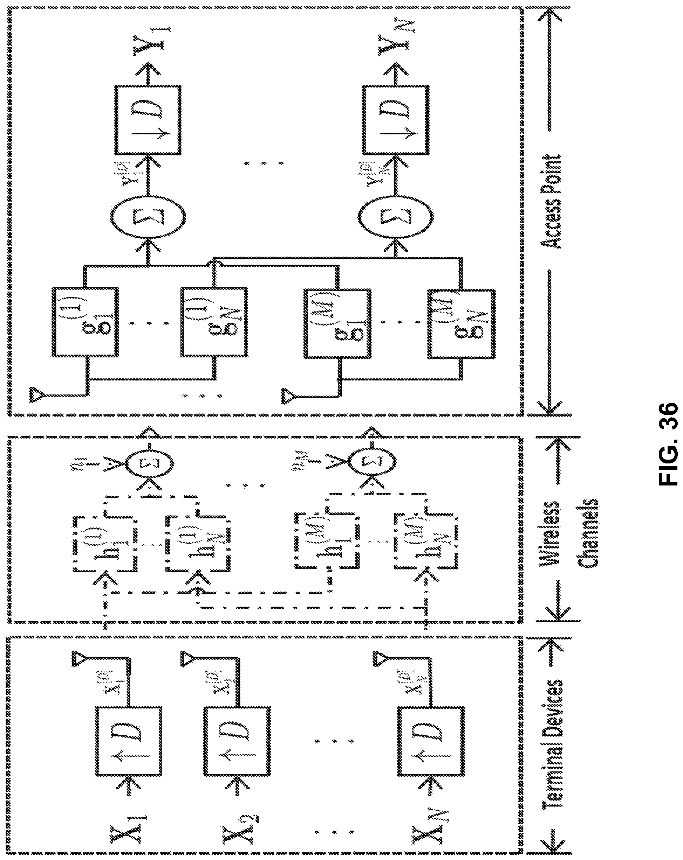

FIG. 36 shows an exemplary diagram of Time-Reversal Division Multiple Access with multiple antennas (TRDMA-MA) uplink system, according to an embodiment of the present teaching;

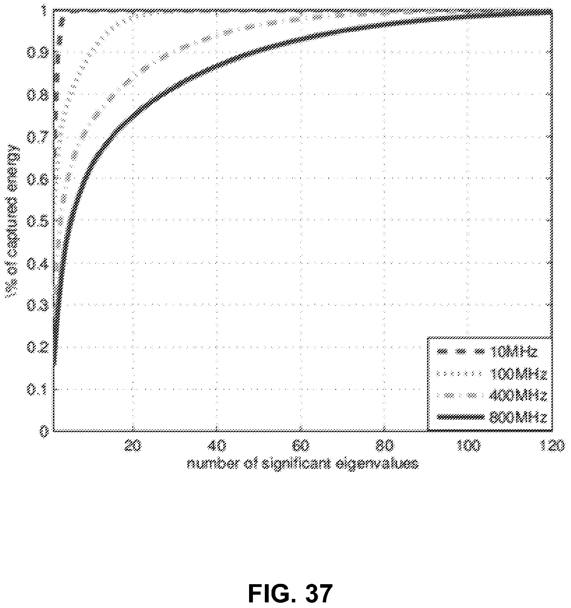

FIG. 37 shows the percentage of captured energy versus the number of significant eigenvalues with a single antenna, according to an embodiment of the present teaching;

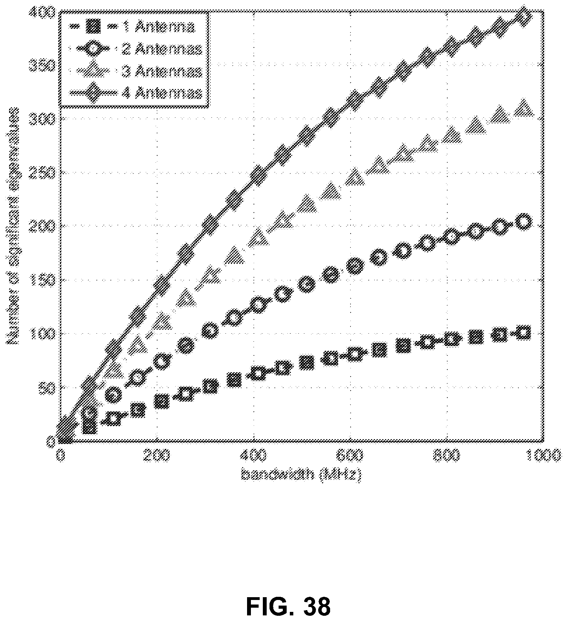

FIG. 38 shows the number of significant eigenvalues with varying bandwidth, according to an embodiment of the present teaching;

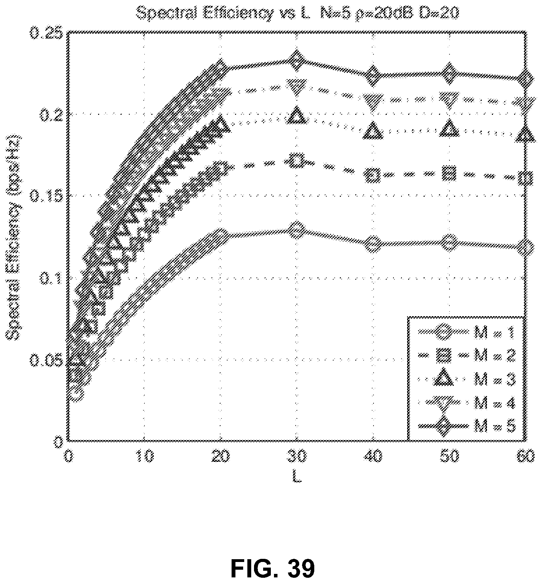

FIG. 39 shows the spectral efficiency of an individual user vs L, under basic TR waveforming with the number of users N = 5, varying M, ρ=20dB and D=20, according to an embodiment of the present teaching;

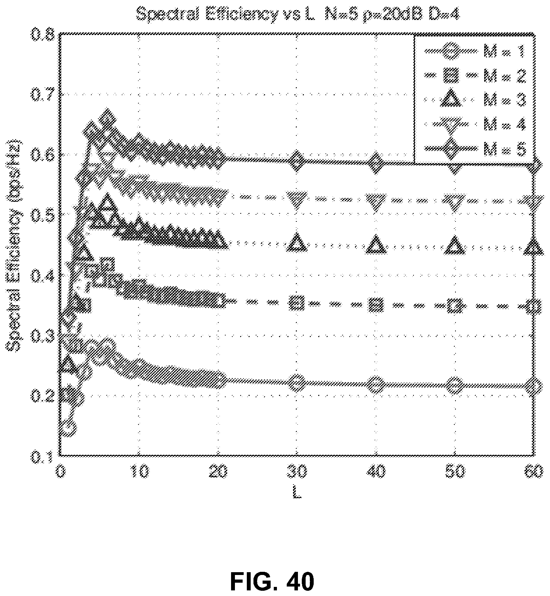

FIG. 40 shows the spectral efficiency of an individual user vs L, under basic TR waveforming with the number of users N = 5, varying M, ρ=20dB and D=4 , according to an embodiment of the present teaching;

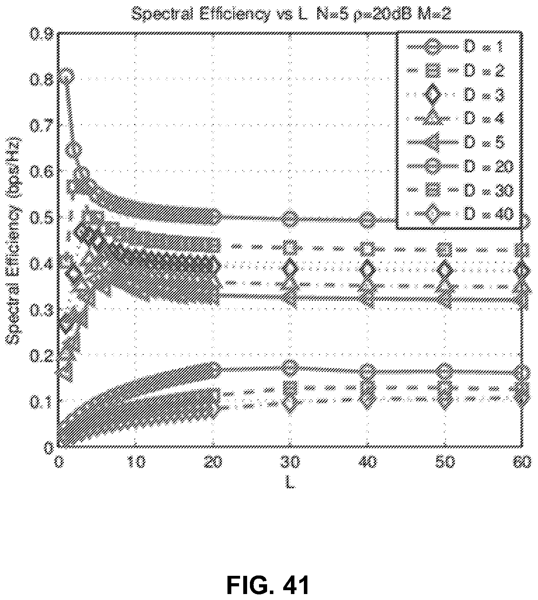

FIG. 41 shows the spectral efficiency of an individual user under basic TR waveforming with N = 5, M = 2 and varying D, according to an embodiment of the present teaching;

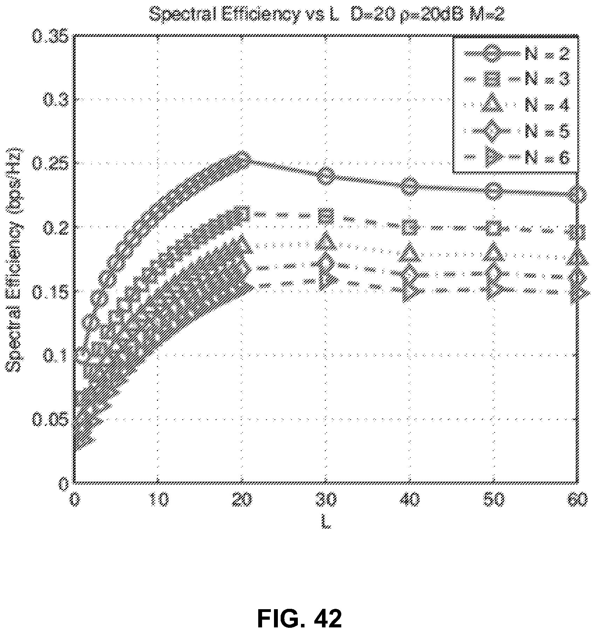

FIG. 42 shows the spectral efficiency of an individual user under Zero Forcing (ZF) waveforming with M = 2, D=20 and varying N, according to an embodiment of the present teaching;

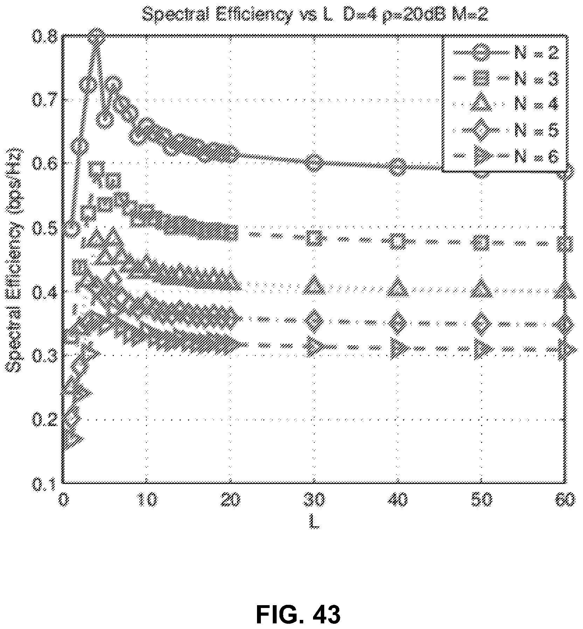

FIG. 43 shows the spectral efficiency of an individual user under ZF waveforming with M = 2, D=4 and varying N, according to an embodiment of the present teaching;

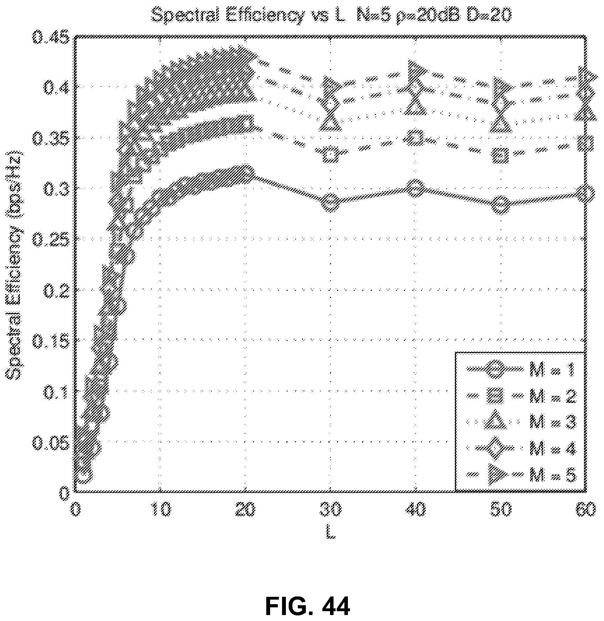

FIG. 44 shows the spectral efficiency of an individual user vs. L, under ZF waveforming with N=5, ρ=20dB, and D=20, according to an embodiment of the present teaching;

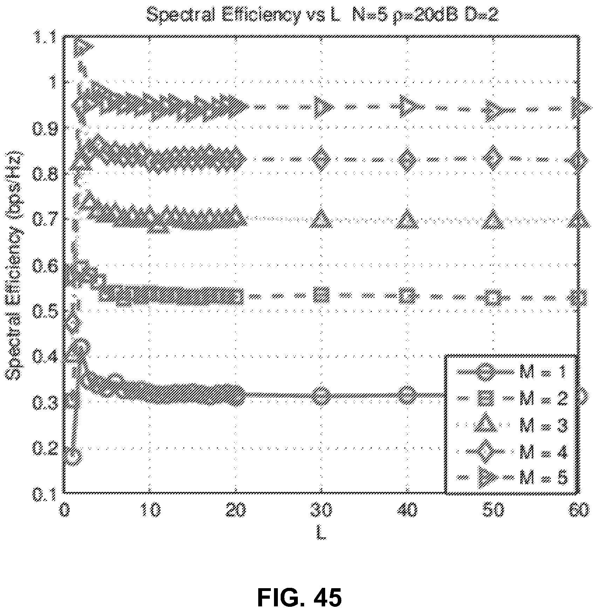

FIG. 45 shows the spectral efficiency of an individual user vs L, under ZF waveforming with N=5, ρ=20dB, and D=2, according to an embodiment of the present teaching;

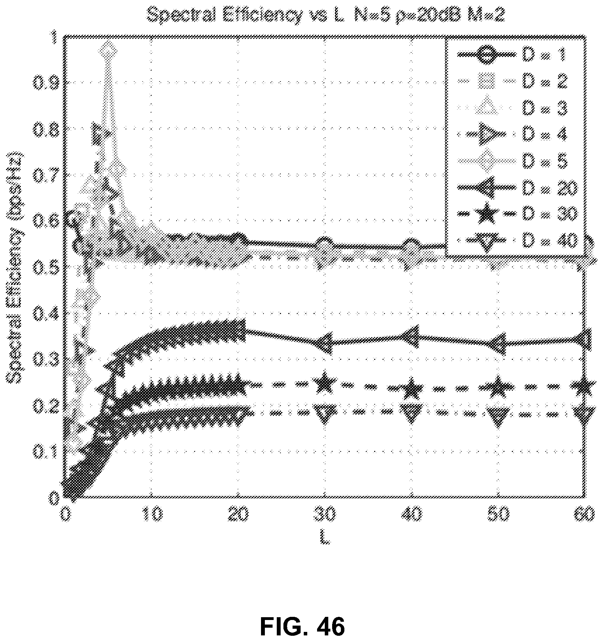

FIG. 46 shows the spectral efficiency of an individual user under ZF waveforming with N = 5, M = 2 and varying D, according to an embodiment of the present teaching;

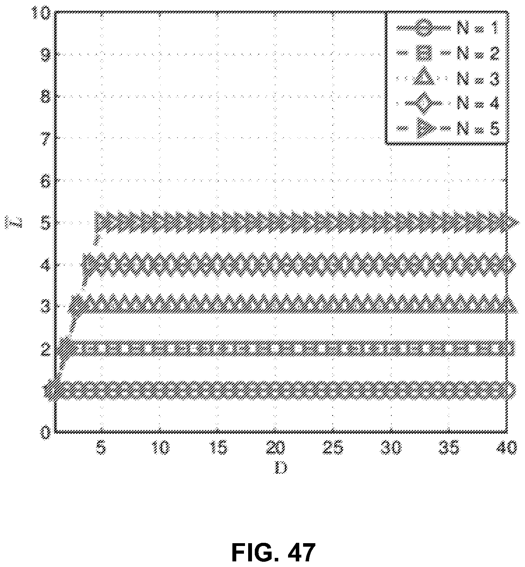

FIG. 47 shows the sub-optimal L with varying D and N, according to an embodiment of the present teaching;

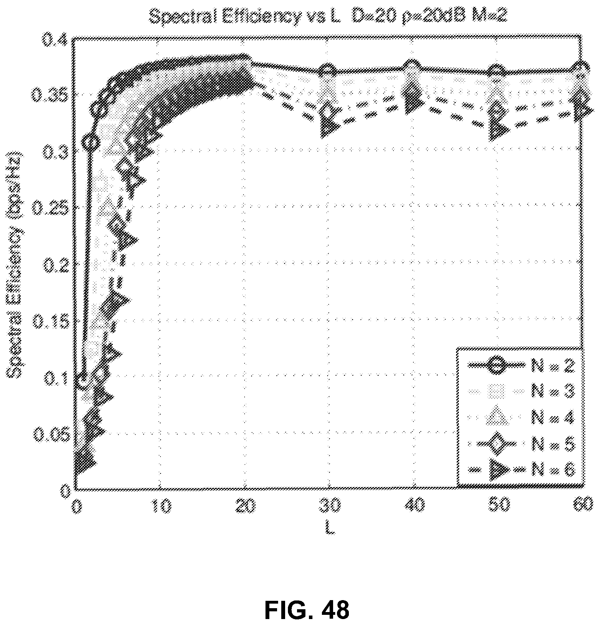

FIG. 48 shows the spectral efficiency of an individual user with M = 2, D=20 and varying N, according to an embodiment of the present teaching; and

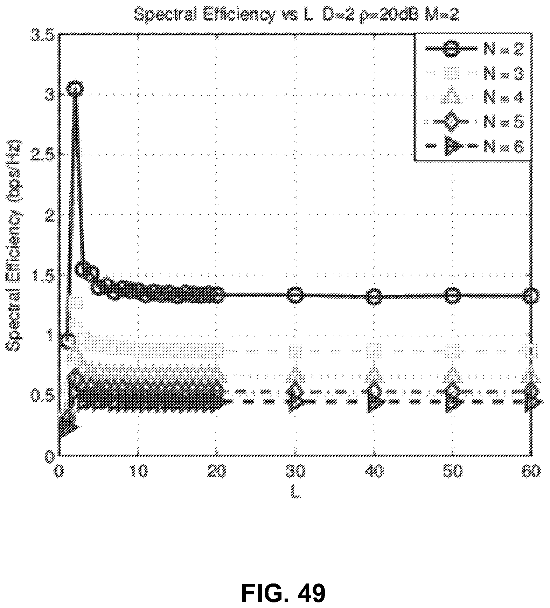

FIG. 49 shows the spectral efficiency of an individual user with M = 2, D=2 and varying N.

### DETAILED DESCRIPTION

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and/or circuitry have been described at a relatively high-level, without detail, in order to avoid unnecessarily obscuring aspects of the present teachings.

The present teaching discloses an object tracking system, Time-Reversal Indoor Tracking System (TRITS), that can track the real-time location of a moving object based on a special property caused by time-reversal resonating/focusing effect in a rich-multipath environment. The present teaching discloses a new discovery that due to the sum of many multiple signal paths, the energy distribution of the time-reversal focusing effect exhibits a stationary but location-independent property, which can be used to estimate the speed of a moving object in a typical real-world indoor environment. Then, based on the accurate estimation of moving speed of an object, the present teaching discloses an object tracking system by combining the speed estimation and the estimation of moving direction, the latter of which can be obtained from IMU.

In one example, a method for tracking a movement of an object in real-time is disclosed. The method may be implemented on a machine including at least a processor and a memory communicatively coupled with the processor. The method may comprise: obtaining an initial position of the object prior to a movement of the object; obtaining at least one wireless signal from a multipath channel that is impacted by the movement of the object; extracting a time series of channel state information (CSI) for the multipath channel from the at least one wireless signal; determining a distance of the movement of the object based on the time series of CSI; estimating a direction of the movement of the object; and determining a new position of the object after the movement based on the distance, the direction, and the initial position. During the movement, the object may carry at least one of: a transmitter that transmits the at least one wireless signal; a receiver that receives the at least one wireless signal; and a sensor configured for direction estimation.

In one embodiment, determining the distance of the movement of the object comprises: cleaning a phase offset of each of the time series of CSI; calculating a similarity score based on each pair of consecutive CSIs among the time series of CSI to obtain a plurality of calculated similarity scores, wherein each of the plurality of calculated similarity scores indicates a degree of similarity between a corresponding pair of CSIs; computing an average similarity score based on the plurality of calculated similarity scores, wherein the average similarity score indicates a degree of spatial resonating decay associated with the movement of the object; and comparing the average similarity score with a reference decay curve to obtain an estimated distance. In one embodiment, determining the distance of the movement of the object further comprises: calculating an additional similarity score based on a first CSI and a last CSI in the time series of CSI; comparing the additional similarity score with a pre-determined threshold; determining the distance of the movement of the object to be zero when the additional similarity score exceeds the pre-determined threshold; and determining the distance of the movement of the object to be the estimated distance when the additional similarity score does not exceed the pre-determined threshold. The similarity score may be calculated based on at least one of: a TRRS, a cross-correlation, an auto-correlation, an inner product of two vectors, a similarity score, a distance score, a phase correction, a timing correction, a timing compensation, and a phase offset compensation, of a pair of CSIs.

In another embodiment, determining the distance of the movement of the object comprises: cleaning a phase offset of each of the time series of CSI, wherein the time series of CSI are extracted according to a sampling period; calculating a similarity score between a most recent CSI in the time series of CSI and each of previous CSIs in the time series of CSI to obtain a time series of similarity scores, wherein each of the time series of similarity scores indicates a degree of similarity between the most recent CSI and a corresponding previous CSI; determining a curve based on the time series of similarity scores; identifying a feature point on the curve; estimating a time period corresponding to the feature point on the curve; estimating a speed of the movement during the time period; and obtaining an estimated distance of the movement of the object based on the speed and the sampling period. Determining the distance of the movement of the object may further comprises: calculating an additional similarity score based on a first CSI and a last CSI in the time series of CSI; comparing the additional similarity score with a pre-determined threshold; determining the distance of the movement of the object to be zero when the additional similarity score exceeds the pre-determined threshold; and determining the distance of the movement of the object to be the estimated distance when the additional similarity score does not exceed the pre-determined threshold. The similarity score may be calculated based on at least one of: a TRRS, a cross-correlation, an auto-correlation, an inner product of two vectors, a similarity score, a distance score, a phase correction, a timing correction, a timing compensation, and a phase offset compensation, of a pair of CSIs. The feature point on the curve may be identified based on at least one of: a first local peak on the curve, one or more other local peaks on the curve, a first local bottom on the curve, one or more other local bottoms on the curve, and a point having a pre-determined relationship with a local peak or a local bottom on the curve. In one example, the feature point on the curve is identified based on a first local peak on the curve, and the time period corresponding to the first local peak is estimate based on a similarity score corresponding to the first local peak and two adjacent similarity scores among the time series of similarity scores.

In one embodiment, estimating the direction of the movement of the object comprises: obtaining a gravity direction of the object from a first sensor; obtaining rotational information of the object from a second sensor; determining a coordinate rotation velocity based on the gravity direction and the rotational information; obtaining a sensor reading interval of the second sensor; calculating a direction change based on the coordinate rotation velocity and the sensor reading interval; and estimating the direction of the movement based on the direction change and a previously estimated direction. Obtaining rotational information of the object from a second sensor may comprise obtaining angular velocity of the object from a gyroscope.

In another embodiment, estimating the direction of the movement of the object comprises: obtaining a first moving distance from a first location straightly to a second location; obtaining a second moving distance from the second location straightly to a third location; obtaining a third moving distance from the first location straightly to the third location, wherein at least one of the first, second and third moving distances is determined based on the time series of CSI; and estimating the direction of the movement of the object based on the first, second and third moving distances according to trigonometry. In another embodiment, estimating the direction of the movement of the object comprises: obtaining a plurality of average decay curves of spatial resonating strength within a time window on a plurality of antennas; determining at least one pattern based on the plurality of average decay curves; and estimating the direction of the movement of the object based on the at least one pattern.

In various embodiments, the at least one wireless signal is received by a receiver through a network that is at least one of: Internet, an Internet-protocol network, and another multiple access network; and the receiver is associated with a physical layer of at least one of: a wireless PAN, IEEE 802.15.1 (Bluetooth), a wireless LAN, IEEE 802.11 (Wi-Fi), a wireless MAN, IEEE 802.16 (WiMax), WiBro, HiperMAN, mobile WAN, GSM, GPRS, EDGE, HSCSD, iDEN, D-AMPS, IS-95, PDC, CSD, PHS, WiDEN, CDMA2000, UMTS, 3GSM, CDMA, TDMA, FDMA, W-CDMA, HSDPA, W-CDMA, FOMA, 1xEV-DO, IS-856, TD-SCDMA, GAN, UMA, HSUPA, LTE, 2.5G, 3G, 3.5G, 3.9G, 4G, 5G, 6G, 7G and beyond, another wireless system and another mobile system.

In another example, a method for tracking real-time position of an elevator is disclosed. The method may be implemented on a machine including at least a processor and a memory communicatively coupled with the processor. The method may comprise: obtaining a first output from a measurement unit that is coupled to the elevator such that the measurement unit has a fixed position relative to the elevator, wherein the first output represents a raw estimate of acceleration of the elevator; obtaining a second output from the measurement unit, wherein the second output represents a measurement of gravity at a same location as the elevator; calculating an acceleration of the elevator in vertical direction in a current time slot based on the first output and the second output; obtaining a previous speed of the elevator in vertical direction calculated in a previous time slot; and determining whether the elevator is moving based on the acceleration and the previous speed. In one embodiment, the method further comprises calibrating the measurement unit by: collecting readings of the measurement unit for a time period to estimate a reading bias; and calculating the reading bias based on an average of the readings, wherein the reading bias is subtracted from each output of the measurement unit before calculating the acceleration or a speed of the elevator.

In one embodiment, determining whether the elevator is moving comprises: comparing the acceleration with a first threshold; comparing the previous speed with a second threshold; when either the acceleration exceeds the first threshold or the previous speed exceeds the second threshold, determining that the elevator is moving, and comparing the previous speed with a third threshold; and when neither the acceleration exceeds the first threshold nor the previous speed exceeds the second threshold, determining that the elevator is not moving, setting a speed of the elevator to be zero, and estimating a current position of the elevator. The method may further comprise: when the previous speed exceeds the third threshold, generating an alarm indicating that the elevator is experiencing an abnormal fall; and when the previous speed does not exceed the third threshold, generating an updated speed of the elevator based on the previous speed and the acceleration, generating an updated moving distance of the elevator based on the updated speed, and generating an updated position of the elevator by adding the updated moving distance to a previously estimated position of the elevator.

Estimating the current position of the elevator may comprise: rounding off an estimate of the current position to a nearest height of floors; determining a rounding off error based on the rounding off; comparing the rounding off error with a fourth threshold; when the rounding off error exceeds the fourth threshold, generating a report indicating that the elevator stops at an abnormal position; and when the rounding off error does not exceed the fourth threshold, determining whether the acceleration is smaller than a fifth threshold, and updating an estimation of a reading bias of the measurement unit when the acceleration is smaller than a fifth threshold. The measurement unit may include at least one of: an inertial measurement unit (IMU), an accelerometer, and a gyroscope.

In a different example, a system for detecting object motion in a venue is disclosed. The system may comprise: a transmitter configured for transmitting at least one wireless signal; a receiver configured for receiving the at least one wireless signal that can be impacted by object motion in the venue; a processor; and a memory communicatively coupled with the processor. The processor is configured for: extracting one or more time series of CSI from the at least one wireless signal, calculating a statistical value based on the one or more time series of CSI, wherein the statistical value represents a degree of object motion in the venue, and determining whether object motion is present in the venue based on the statistical value. In one embodiment, the statistical value may be calculated based on at least one of: a real part of a CSI of the one or more series of CSI, an imaginary part of the CSI, CSI amplitude of the CSI, a square of the CSI amplitude, another function of the CSI amplitude, and a sample auto-correlation coefficient derived from a function of the one or more series of CSI.

In one embodiment, the at least one wireless signal includes a plurality of subcarriers; and calculating the statistical value comprises: calculating a time series of CSI for each of the plurality of subcarriers, calculating a sub-statistic based on each time series of CSI to generate a plurality of sub-statistics, and calculating the statistical value based on the plurality of sub-statistics. Whether object motion is present in the venue may be determined based on at least one of: a majority vote for fusing all decisions about whether object motion is present from the plurality of sub-statistics; and a comparison between a statistical combination of the plurality of sub-statistics and a threshold.

In a different example, a system for tracking a status of a door of an elevator is disclosed. The system may comprise: a transmitter configured for transmitting at least one wireless signal; a receiver configured for receiving the at least one wireless signal that can be impacted by a status of the door, wherein at least one of the transmitter and the receiver is located within the elevator; a processor; and a memory communicatively coupled with the processor. The processor is configured for: obtaining a time series of signal measurements based on the at least one wireless signal, filtering the time series of signal measurements by mitigating outliers and noisy measurements to generate a plurality of filtered measurement values each of which is associated with a corresponding time slot, and determining whether the door of the elevator is closed or open in each time slot based on the filtered measurement value associated with the time slot and a threshold. In one embodiment, the processor is further configured for: obtaining a first time series of signal measurements based on a first plurality of wireless signals received when the door is known being opened; obtaining a second time series of signal measurements based on a second plurality of wireless signals received when the door is known being closed; determining a change pattern in the first and second time series of signal measurements during changes of the status of the door; and calculating the threshold based on the change pattern. The processor may be further configured for: updating the threshold based on at least one of slope estimation and peak detection performed when determining the change pattern. For example, each of the time series of signal measurements may be based on a function of received signal power of the at least one wireless signal, and the function can be determined based on at least one of: received signal strength indicator (RSSI), received channel power indicator (RCPI), reference signal received power (RSRP), reference signal received quality (RSRQ), signal-to-noise ratio (SNR), and signal-to-interference-and-noise ratio (SINR).

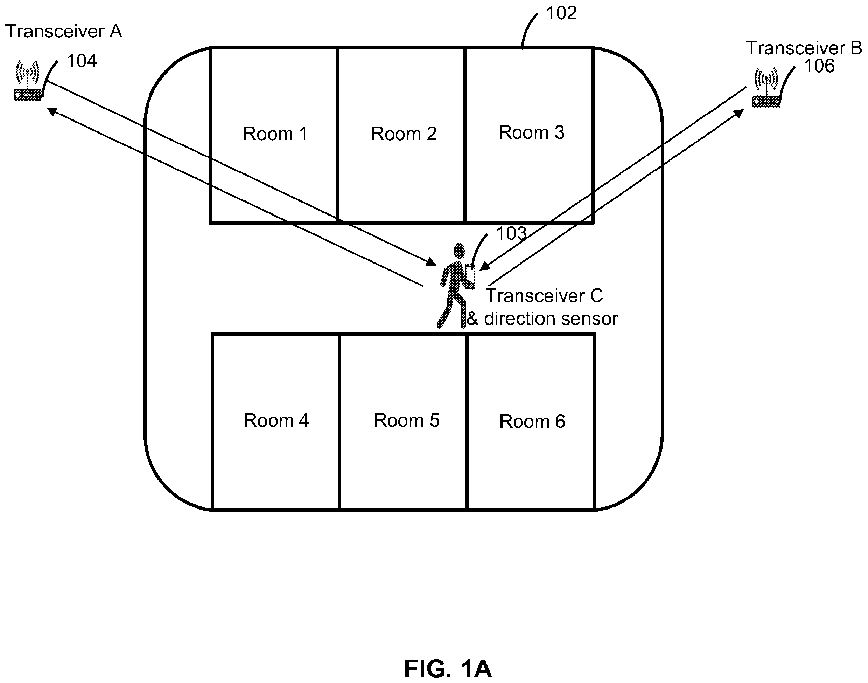

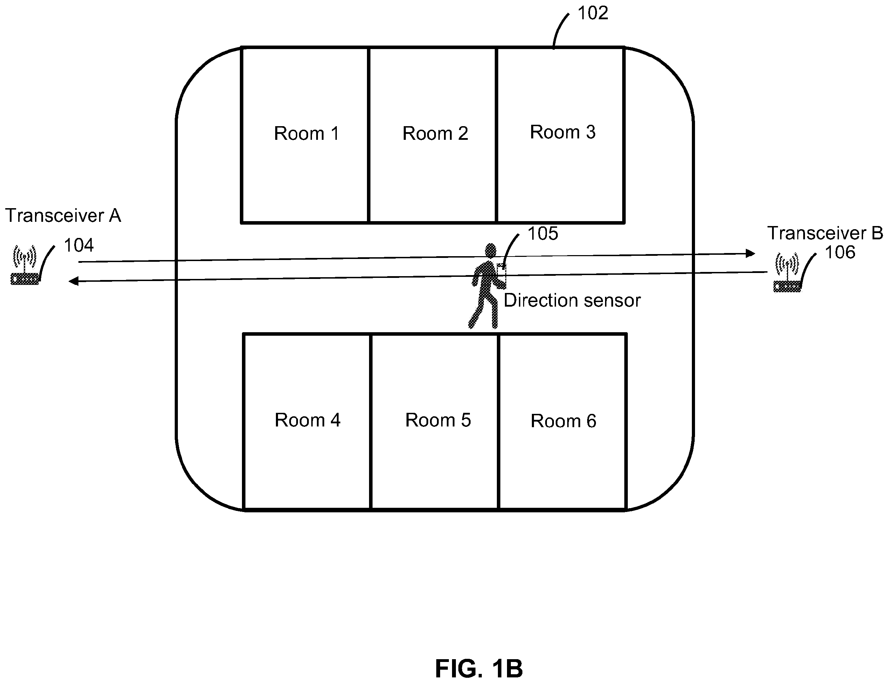

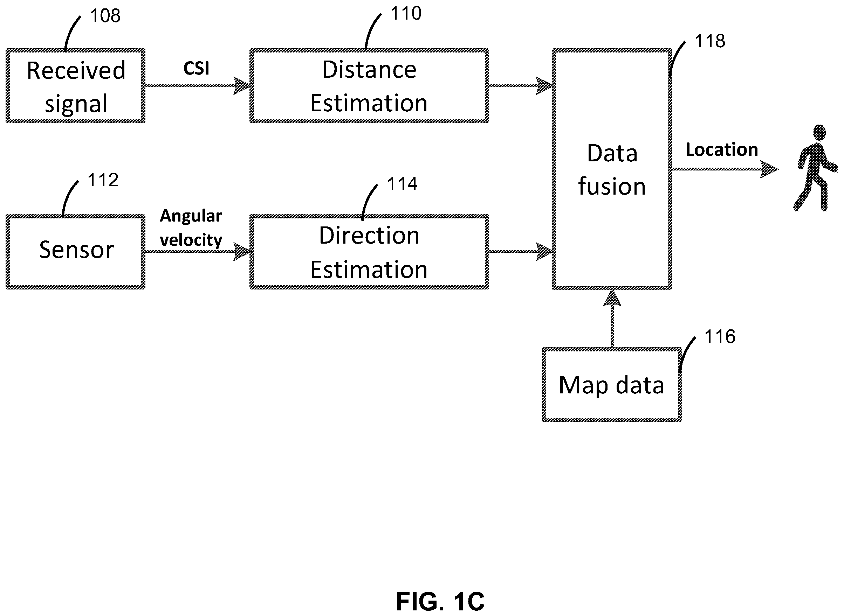

Two exemplary diagrams of the system are shown in FIG. 1A and FIG. 1B, and a flowchart of the system is shown in FIG. 1C. In FIG. 1A, the object/person moving inside a venue 102 carries a transceiver C 103 which keeps sending channel sounding signals to other transceivers, for example, transceiver A 104 and transceiver B 106. The transceivers A 104 and B 106 can estimate the channel state information (CSI) and calculate speed/moving distance of the object/person. The transceiver C 103 can be equipped with other sensors (e.g., Inertial Measurement Unit (IMU)) that estimate the angular velocity. By integrating the angular velocity, the angle change or change in the moving direction of the object/human can be estimated. In FIG. 1B, the object/person only carriers a sensor 105 for moving direction estimation. The wireless channel between transceiver A 104 and transceiver B 106 gets affected by the movement of the object/person, so based on the CSI estimate of the affected wireless channel, the speed/moving distance of the object/person can also be estimated. Assuming the initial starting point is known, by fusing 118 the distance estimation 110 and direction estimation 114 (or perhaps as well as combining information about the floor plan/path 116) the real-time location of the moving object/human can be obtained. Some experimental results showed that the accuracy of the object tracking system can achieve within 1 meter, if information about the floor plan of the venue in which the object is moving is incorporated. Note that the present teaching can also work for tracking an object moving in outdoor environment, if there is rich multipath propagation of the radio frequency (RF) signals.

In one embodiment, consider a transmitter and receiver pair placed in a typical indoor environment. The transmitter transmits wireless signals continuously with a nearly uniform transmission interval and the receiver estimates the corresponding channel state information (CSI). Given the start point (or initial location, initial position) of the transmitter, the goal of the disclosed TRITS is to track the location of the transmitter in real time. TRITS is not limited to tracking moving objects in indoors only and it can work well as long as the system operates on a large enough bandwidth to resolve enough multipath components (MPC) existing in rich-scattering environments, for example in indoor or urban metropolitan areas. In one embodiment, the disclosed system can use TRITS to represent the object tracking system. But this does not mean the system can only work in indoor.

In the following, as an example, the disclosed system can operate TRITS on commercial Wi-Fi platform and track the location of a transmitter as an example. TRITS utilizes the idea of dead-reckoning to localize the transmitter, that is, TRITS calculates the transmitter's current position by using a previously determined position. Mathematically, it can be written as $x \left(t\right) = x \left(t-1\right) + Δ \left(t\right) ,$ (1) where x(t) stands for the location of the transmitter at time *t* and *Δ*(*t*) can be expressed as *d*(*t*)**1**(*t*), where *d*(*t*)*=*|Δ(*t*)| and **1**(*t*) is the unit vector pointing to the direction of Δ(t). Therefore, TRITS includes two main modules: moving distance estimation for each time slot d(t) and the moving direction estimation **1̂**(t). The main innovation of TRITS is that it utilizes the time-reversal spatial resonating phenomenon to estimate the moving distance of the transmitter.

The present teaching discloses two moving distance estimation methods and two moving direction estimation methods. Then, using equation (1), the location of the moving object can be tracked in real time. In the following, the distance estimation module and the direction estimation module will be introduced.

### Distance Estimation Based on Statistical Behavior of TR Resonance

The first method for moving distance estimation is based on the statistical behavior of time-reversal resonating strength (TRRS). TRRS, which will be defined later, can be viewed as a similarity score between two CSIs. It can be found that the TRRS values between one CSI collected at one particular location and CSIs collected at surrounding locations exhibit a certain statistical pattern, i.e., there exists a mapping between distance and TRRS value.

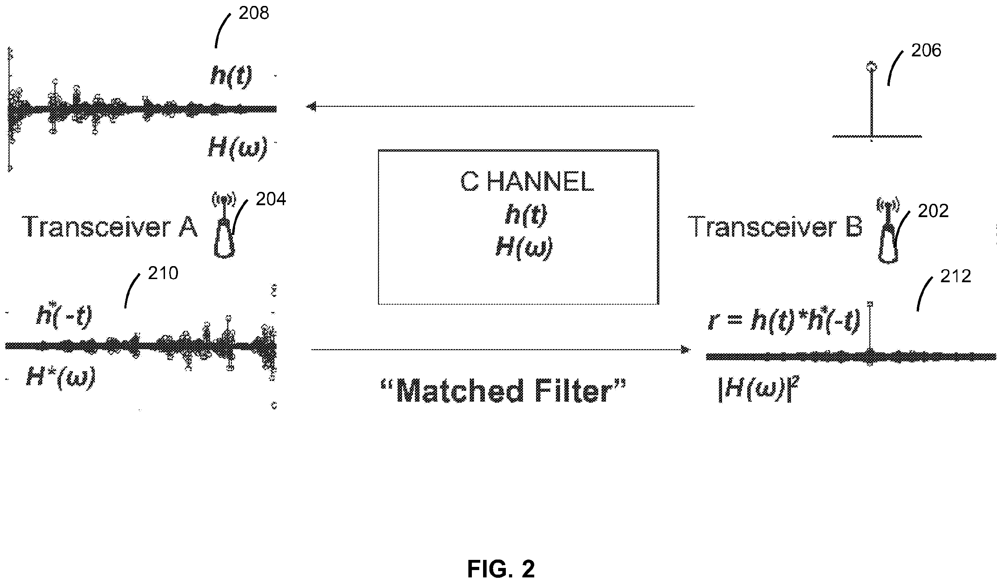

In one embodiment, consider a wireless transmitter and receiver each equipped with a single omnidirectional antenna in a rich-scattering environment. The channel impulse response (CIR) from transmit point T to receive point R is expressed as *h*(*τ*; *T* → *R*) where T and R denotes the coordinates of the transmitter and receiver respectively, and *τ* stands for the delay of multipath components of the measured CIR. Consider a stationary indoor rich-scattering environment, i.e., all CIRs can be considered as time-invariant signals. Therefore, the CIR is determined by the receiver coordinate given a fixed transmitter coordinate. In a TR communication system, the receiver (transceiver B 202 in FIG. 2) first transmits a delta-like pilot impulse 206 and the transmitter at T (transceiver A 204) captures the CIR (208) from *R*₀ to T. Using a delta-like pilot impulse is just an example and other types of channel probing signal, e.g., pseudo random sequence or a sequence of pulses, can also be adopted using methods disclosed in U.S. Patent Application No. 15/041,677, filed February 11, 2016, entitled "Handshaking Protocol for Time-Reversal System," and U.S. Patent Application No. 15/284,496, filed October 3, 2016, entitled "Time-Reversal Communication Systems,". After that, the transmitter at T (transceiver A 204) simply transmits back the reversed and conjugated version of the captured CIR 210, i.e., as illustrated in FIG. 2, and the received signal 212 at any receive point R (transceiver B 202) can be expressed as $s \left(τ; R\right) = h \left(τ;T\to R\right) ⋆ h * \left(-τ;{R}_{0}\to T\right) ,$ (2) where * denotes the convolution operation and the superscript * denotes complex conjugation. In the following, *R*₀ is called the *focal point.* Assuming that the channel reciprocity holds, i.e., the forward and backward channels are identical, the disclosed system can have *h*(τ; *T* → *R*) *= h*(τ; *R* → T), which has already been validated experimentally.

One can study the characteristics of TR resonating effects by investigating s(τ;R) from the space-time perspective. In practice, the sampling frequency of the wireless transceivers is fixed which limits the resolution of the CIRs. A large bandwidth, denoted by B, enhances the resolvability of the CIR profile and thus increases the number of components of *h*(τ; *T* → *R*). Due to the sampling process in wireless communications, the disclosed system can discretize *h*(*τ*; *T* → *R*) into *h*(*k*; *T* → *R*), with k∈ {0,1, ···, L-1} and L is the maximum number of taps given a bandwidth of B. Assuming that the transmitter coordinate is fixed, one can rewrite *s*(*k*; *R*) as $s \left(τ; R\right) = {\sum }_{l = 0}^{L - 1} h \left(k; R\right) h * \left(l-k;{R}_{0}\right) ,$ (3) where *h**(*l - k; R*₀) = 0 when (l-k) ∉ {0,1, ..., L-1}, and k∈{-(L-1), ..., (L-1)}.

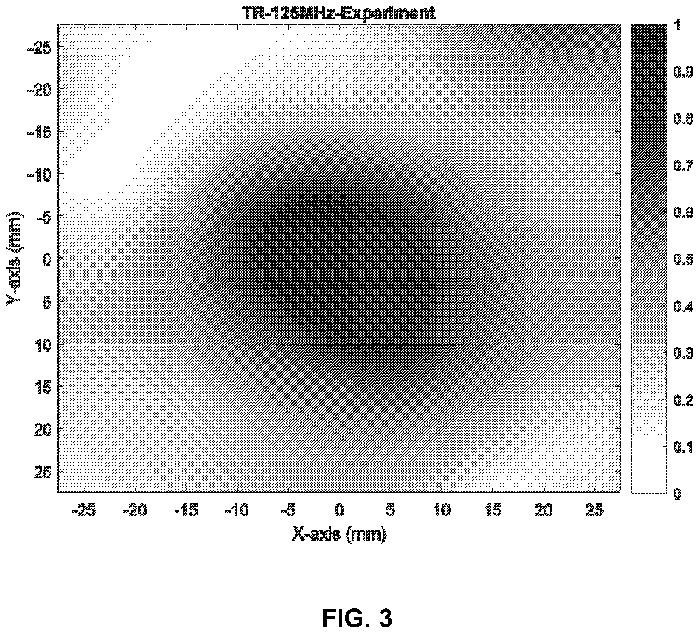

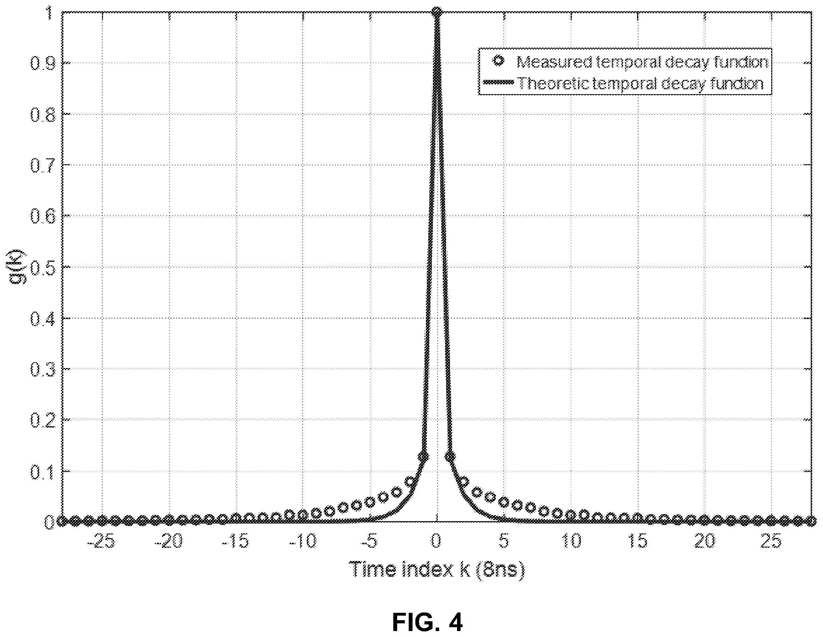

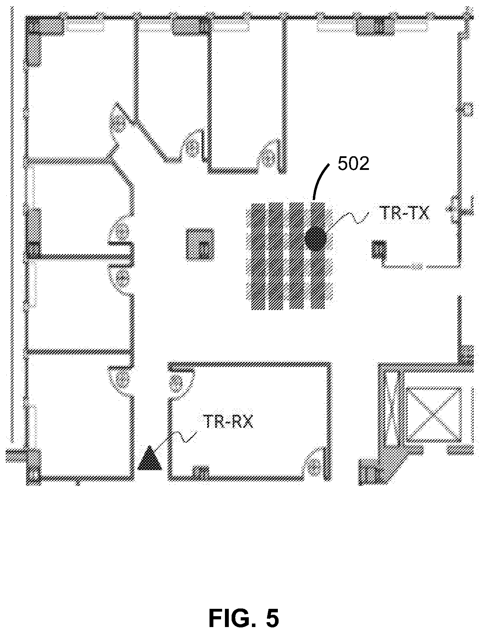

The TR resonating effect occurs at the specific time k=0 and at the particular location *R*₀, where all the multipath components add up coherently. For k≠0, the taps add up incoherently and thus the magnitude of received signal is much smaller. Consequently, the energy of the received signal is highly concentrated around k=0 (temporal focusing) and *R*₀ (spatial focusing), which is called the TR focusing/resonating effect. Using CIR measurements taken in a typical indoor environment as shown in FIG. 5, FIG. 3 shows the spatial TRRS distribution around the focal spot and FIG. 4 shows the temporal normalized receive signal distribution of the focal spot. It can be seen that the normalized receive energy both focused in both time and spatial domains.

One can define the TR resonating strength (TRRS) between the CIR at the focal point *R*₀ and the CIR at another point R as a normalized version of the energy of the received signal at R if the reversed and conjugated version of *h*(*k*; *R*₀) were transmitted: $η \left({R}_{0}, R\right) = {\left|\frac{s \left(0; R\right)}{\sqrt{{\sum }_{l = 0}^{L - 1} {\left|h\left(k; {R}_{0}\right)\right|}^{2}} \sqrt{{\sum }_{l = 0}^{L - 1} {\left|h\left(k; R\right)\right|}^{2}}}\right|}^{2} .$ (4)

It can be seen from FIG. 3 that the TRRS distribution/decay pattern along the straight lines originated from the focal spot to outside the focal spot is independent of the direction. Actually, a very interesting phenomenon associated with the TR spatial resonating effect is that the variations of the spatial resonating decay patterns around different focal points tend to be very small, as long as the distance from the focal point is small. One can name this physical phenomenon as the "TR spatial hardening effect".

One can use two TR prototypes: "Prototype I" and "Prototype II" to validate the "TR spatial hardening effect". Prototype I is implemented on the specially designed hardware that operates at 5GHz ISM band with a bandwidth of 125MHz. One can set the maximum number of multipath components as L=30, which is sufficient to capture most the total channel energy in a typical indoor environment. For Prototype II, one can obtain the channel frequency response (CFR) from the Wi-Fi devices equipped with multiple antennas. For each transmitting-receiving antenna pair, the CFRs are reported on 114 usable subcarriers out of the 128 subcarriers under 40MHz bandwidth using 802.11n. The CFRs can be transformed into CIRs via discrete Fourier transform. For each prototype, the receiver is placed on the channel probing table 502 with 5mm measurement resolution, as shown in FIG. 5.

As shown in FIG. 3 and FIG. 4, the TR resonating effect occurs temporally and spatially. The obtained spatial resonating decay function is almost decreasing uniformly along all directions away from the focal point. If this phenomenon is also uniform over a large area and the spatial resonating decay patterns around different focal points have similar decaying rate, then the decaying in the spatial resonating strength can be utilized as a metric of distance, which can be further utilized for speed estimation given that the time difference between two CIRs is fixed. Next, one can investigate the stationarity of TR resonating effect within a certain area.

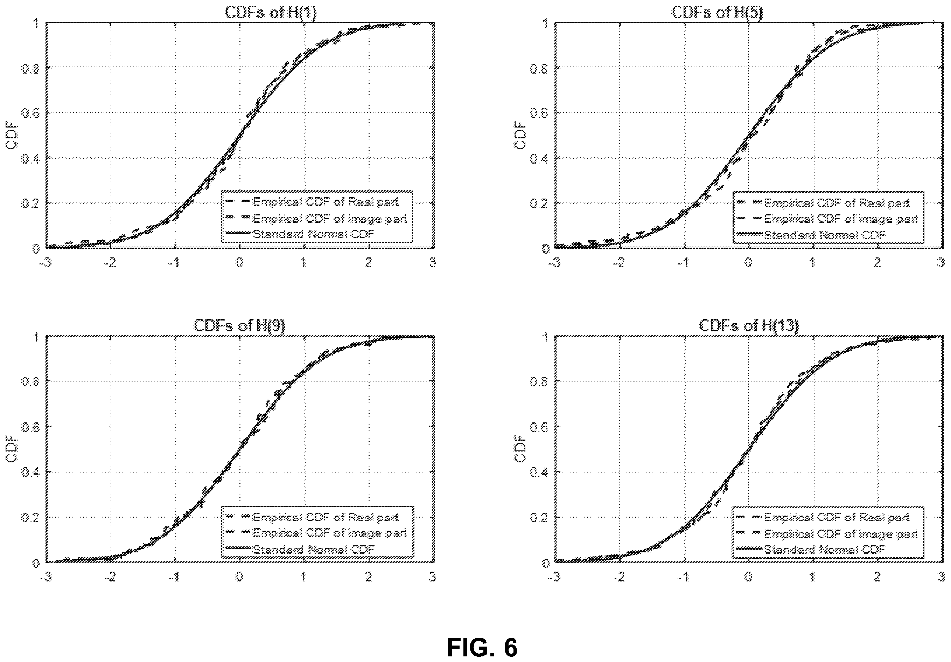

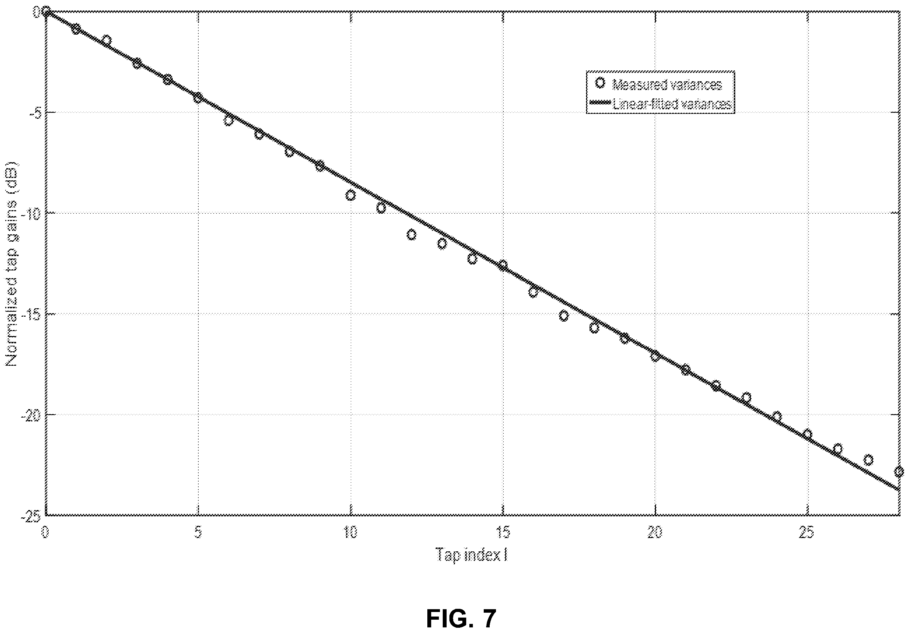

In the following, one can use h(R) to denote the CIR at location R. One can measure the CIRs of 55 different locations on a designed channel probing table using Prototype I. The distance between any two locations exceeds 20cm. For each location, one can measure CIRs from 20 equally-spaced sub-locations along a 10cm line away from that location with 0.5cm measurement resolution. In total, one can obtain 1100 CIRs in this experiment. Now one can treat the CIR as a random vector denoted as H and thus h(R) can be treated as the realization of H at location R. Let (H, *H_{d}*) denote the pair of CIR random vectors with distance d apart from each other, and a realization of (H, *H_{d}*) at location (R, R + Δ) can be denoted as (h(R), h(R + Δ))) with |Δ|=d. When |Δ| is sufficiently large, the two components contained in the tuple are modeled as two complex random vectors independent and identically distributed (i.i.d.).
One can first study the statistical characteristics of each tap of H where H(l) stands for the l-th tap of the random vector H. Let Re(·) and Im(·) denote the real and imaginary parts of a complex number respectively. Then one can choose the CIRs from the points at least 5cm apart from each other and compute the sample correlation coefficient between the real and imaginary parts of each H(l). Empirical cumulative distribution functions (CDF) of some components of H are shown in FIG. 6 with the real and imaginary parts separately. One can also apply the Kolmogorov-Smirnov test (K-S test) to Re(H(l)) and Im(H(l)) for ∀ l=0, 1,..., L-1. All the K-S tests fail to reject the null hypothesis at the 5% significance level, where the null hypothesis is that the distributions of both Re(H(l)) and Im(H(l)) for ∀ l=0,...L-1 are normal. Therefore, the real part and imaginary part of H(l), ∀l can be assumed as *i.i.d. Gaussian random variables.* In addition, the variances of H(l) exponentially decay with the tap index l for some constant rate α. If one can normalize the variances with respect to that of the first tap, then the linearly-fitted and normalized variance of each tap is shown in FIG. 7 in a dB scale with α=0.1952. Note that this exponential decay phenomenon is consistent with classical results taken in UWB channels.

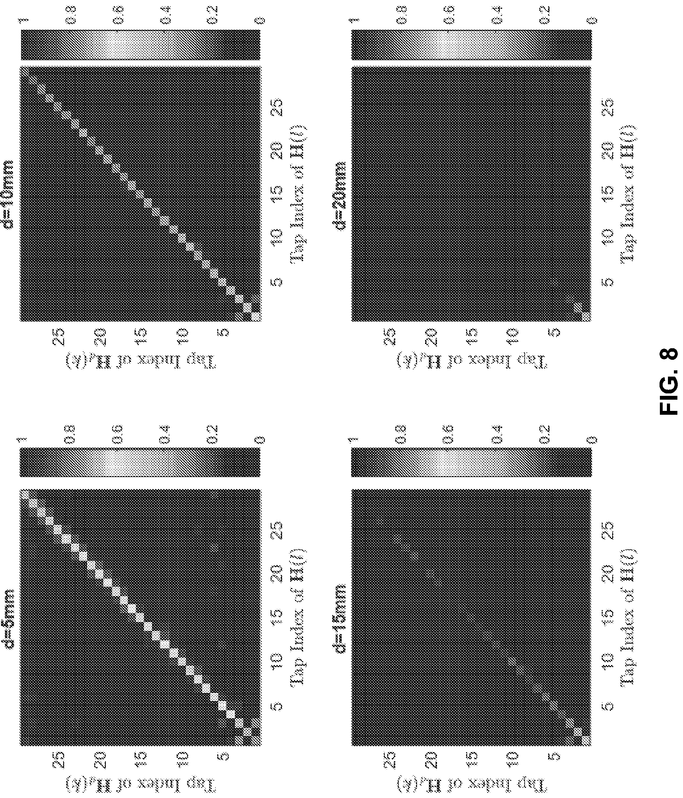

Then one can study the relationship among the different taps in H using the metric of sample correlation coefficient, as shown in FIG. 8. The result shows that H(l) and H(k) are nearly *statistically uncorrelated* for ∀l≠k. Since both H(l) and H(k) (l≠k) are Gaussian random variables, they can be treated as independent random variables. Define the temporal resonating decay function as $g \left(k; {R}_{0}\right) = {\left|\frac{s \left(k; R\right)}{s \left(0; {R}_{0}\right)}\right|}^{2}$. If the TR transmission scheme is applied, assuming the independence among the different taps in H, then based on the previous assumptions, one can compute the theoretical average TR temporal resonating decay function g̅(·) by taking the expectation of the TR temporal resonating decay g(·). In the following, one can approximate g̅(k) by taking the expectations of the numerator and denominator of g(k) separately. Under the assumptions that H(l) is a complex Gaussian random variable for ∀l=0,1,...,L-1, and H(l) and H(k) are independent for ∀l≠k, for k=1,...,L-1 one can have $\overline{g} \left(k\right) = \frac{{e}^{- αk} \left(1-{e}^{- α}\right) \left(1-{e}^{- 2 α \left(L-k\right)}\right)}{\left(1-{e}^{- α}\right) \left(1-{e}^{- 2 αL}\right) + \left(1+{e}^{- α}\right) {\left(1-{e}^{- αL}\right)}^{2}} .$ (5)

It can be seen that g̅(k) is even symmetric in k, i.e., g̅(k)=g̅(-k), and one can have g̅(0)=1. The comparison between the theoretical and measured temporal resonating decay functions are shown in FIG. 4. Overall, the theoretical result matches the empirical data very well especially when k is close to 0. One can observe that the average of temporal resonating decay curve is a strictly monotone decreasing function in |k| and the decaying slope is very steep when |k| is small.

For the spatial resonating decay function, it is just a linear function of the squared magnitude of correlation coefficient 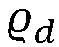 *,* which capture the correlations between two CIR with distance d. With the previous assumptions, one can derive the average of TRRS spatial decay function f(d) as 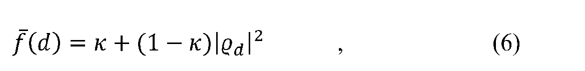 where $κ = \left(\frac{1 - {e}^{- 2 αL}}{1 - {e}^{- 2 α}}\right) / \left(\frac{1 - {e}^{- 2 αL}}{1 - {e}^{- 2 α}}+{\left(\frac{1 - {e}^{- αL}}{1 - {e}^{- 2 α}}\right)}^{2}\right)$.

When the locations corresponding to the two CIRs are very far away and the length of a CIR is very large, the average spatial resonating decay function *f̅*(*d*) converges to $\frac{1 - {e}^{- α}}{2}$, which indicates that the limit of average spatial resonating decay function is determined by α, when the system is placed in a rich-scattering environment and has a large enough bandwidth. With a large α, *f̅*(*d*) approaches to 0 when the two points are sufficiently far away from each other, e.g. 3cm in an experiment setting. Under these conditions, time-reversal transmission techniques can separate the two receive points perfectly in terms of receive power.

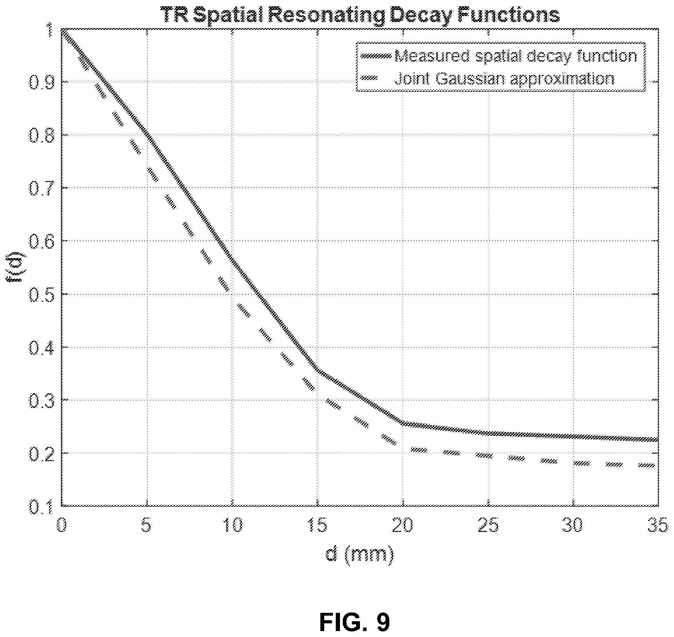

In the experiment, one can discard the first three taps and thus the assumption for the correlation coefficients holds, and the magnitude of correlation coefficient 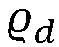 is obtained by taking average over that of all the taps. The results of TR spatial resonating decay functions obtained from the measurements are shown in FIG. 9. The two curves have similar shapes for small distance d<1cm and the joint Gaussian approximation model agrees with the measured one very well, which also justifies the validity of our channel model regarding to spatial resonating effect.

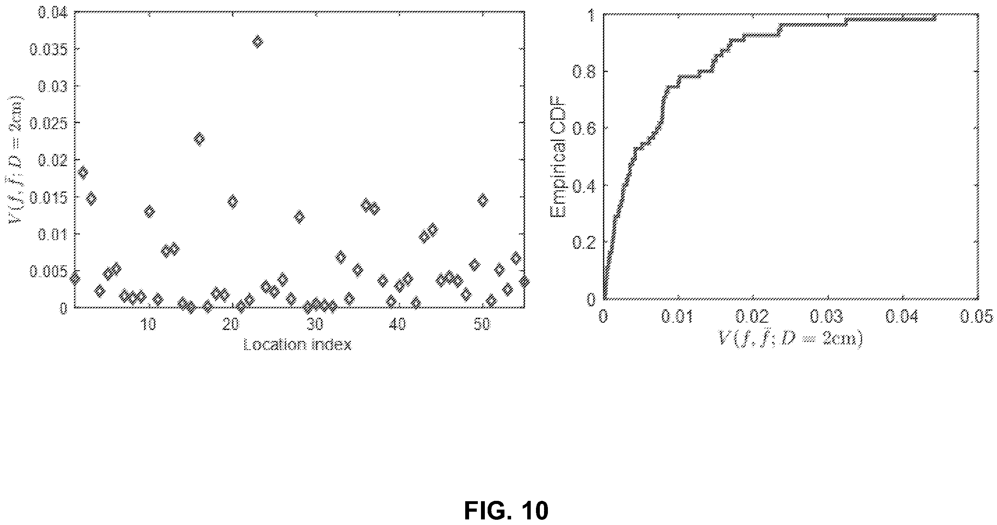

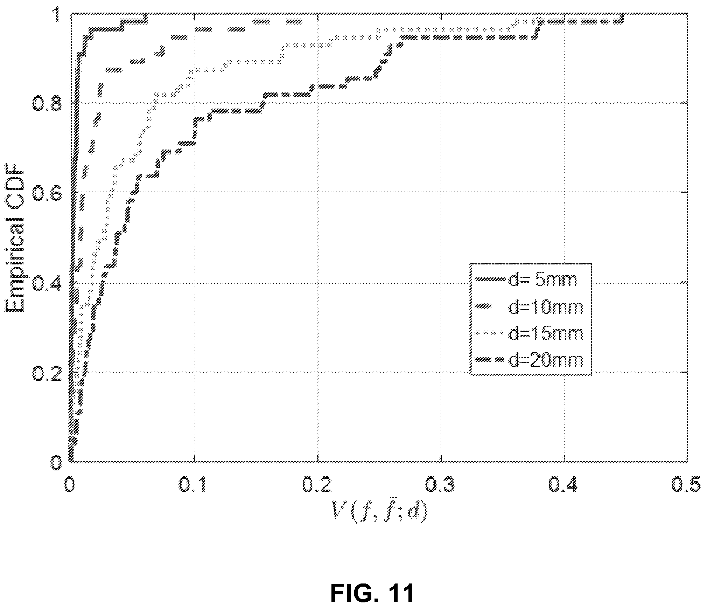

Then, one can verify the stationarity of the spatial resonating decay function over a certain area. To quantify the deviation of the realizations of the decay function from the average decay function, one can define the spatial decay deviation metric as $V \left(f, \overline{f}, D\right) = \frac{{\sum }_{d \leq D} {\left|f\left(d\right)-\overline{f}\left(d\right)\right|}^{2}}{{\sum }_{d \leq D} {\left|\overline{f}\left(d\right)\right|}^{2}} ,$ (7) which measures the normalized deviations between each realization and the average spatial resonating decay functions. Since one can measured 55 different locations in total and one can obtain a single realization of the spatial decay function for each location, the spatial decay variation metric can be computed correspondingly and the result is shown in FIG. 10 for D=2cm. For various D, one can compute the spatial decay deviation metric in FIG. 11.

As one can see from FIG. 11, over 90% of the realizations of spatial resonating decay function have a deviation smaller than 0.02. When the distance is 5mm, more than 90% of realizations are within a variation level of 0.6%. This implies that the defined spatial resonating decay function can be treated as a *stationary feature* over a certain area. It is very interesting to note that although the indoor environment is very complex to model, the spatial resonating decay function exhibits deterministic-like behaviors which may be a consequence of the law of large numbers. Since TR transmission schemes harvest many multipath components existing in the environment, the spatial decay function can be treated as an average of all the random factors.

Under the joint Gaussian approximation model in the above, the l-th taps of two CIRs H(l) and *H_{d}*(*l*) are highly correlated when the distance d is small and one can derive the theoretical variance of the received signal given the CIR at the focal spot h as 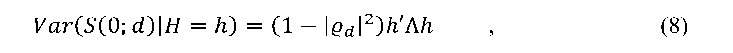 where A is a diagonal matrix with elements {1 *e^{-α} ... e*^{*-*(*L*-1)*α*}} in the diagonal. For the points that are very close to the focal point, the variance of the received signal is small since the magnitude of correlation coefficient is close to 1. Therefore, under the joint Gaussian approximation model, the TR transmission scheme hardens the gain of the received signal near the focal point and one can call it TR spatial hardening effect.

From the TR spatial hardening effect, the gain of received signal or the TR spatial resonating decay function is quite stable especially for small distance d apart from the focal point. Thus, one can translate the reduction of spatial resonating decay into distance d with small errors due to the small variations nearby the focal point. If the transmission interval is a uniform and known Δt, then one can estimate the speed using linear interpolation. Choose two small distances nearby the focal point *d*₁=5mm and *d*₂=10mm, and their average spatial decays are *f̅*(*d*₁) and *f̅*(*d*₂)*,* respectively. For a short period of time, one can get a series of CIRs and one can estimate the spatial decay between adjacent CIRs as f. One can assume that the point (vΔt,f) lies on the line formed by the two points (*d*₁, *f̅*(*d*₁)) and (*d*₂, *f̅*(*d*₂)). Thus, one can estimate the speed as $\hat{v} = \left(\frac{\left({d}_{2}-{d}_{1}\right) \left(\overline{f}\left({d}_{1}\right)-f\right)}{\overline{f} \left({d}_{1}\right) - \overline{f} \left({d}_{2}\right)}+{d}_{1}\right) / Δ t .$ (9) From the result in FIG. 11, one can know that if the channel probing rate is high enough so that one can measure the spatial decay within d=5mm, then the speed estimation can be very accurate. For example, when the channel probing rate is 100Hz, i.e., 100 CIRs within 1 second, and the walking speed is 1.2m/s, then the distance d between two adjacent CIRs is 1.2m/100=1.2cm and the variance of estimation error can be predicted accordingly. On the other hand, the channel probing rate is around 250Hz in a typical LTE system which translate to d=4.8mm for walking speed 1.2m/s, and high accuracy can be expected. To combat the deviations of the spatial resonating decay functions, one may need more CIR samples to average out this effect especially when the channel probing rate is not high enough. In addition, since the spatial resonating decay should be small when the transmitter does not move, one may need to detect if the object is moving or not based on the decay of TRRS within a period. Algorithm 1 summarizes the first method for moving distance estimation.

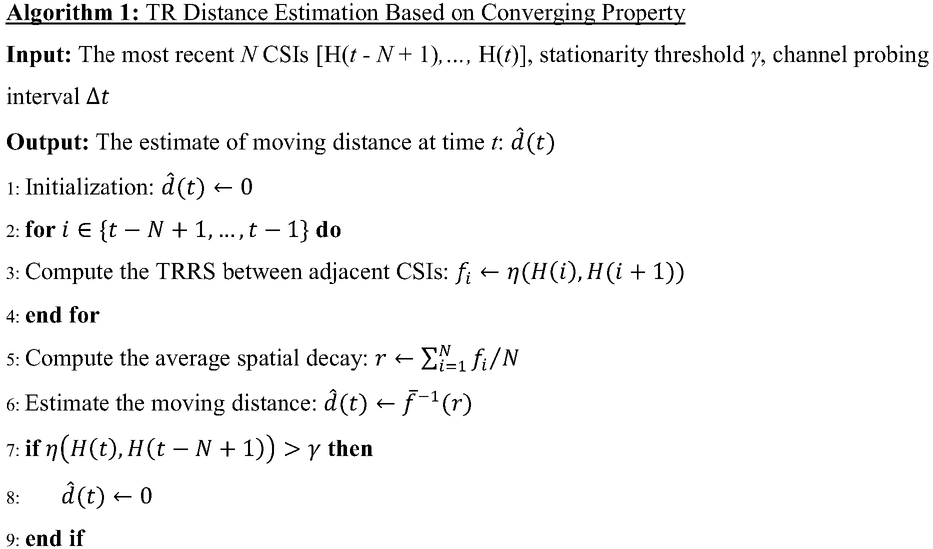

In Algorithm 1, the average of the TRRS decay between adjacent CSIs is estimated within the CSI buffer, and then the estimation of moving distance can be obtained by referring to the pre-measured TRRS decay curve with respect to distance. Specifically, one can use linear interpolation as shown in equation (9) to estimate the moving distance.

At last, the TRRS between the newly coming CSI and the earliest CSI in the buffer is computed to check if the object is moving or not. A very large TRRS value indicates that the two CSIs are highly similar and the object moves a so small distance within the duration of the CSI buffer that the object can be treated as not moving. Empirical measurements show that the distance can be within 5 mm when the TRRS is above 0.9. For a CSI buffer with 0.2-second duration, the speed can be as low as 0.025 m/s, which may be due to the noise of the CSI measurements and should be neglected in real applications.

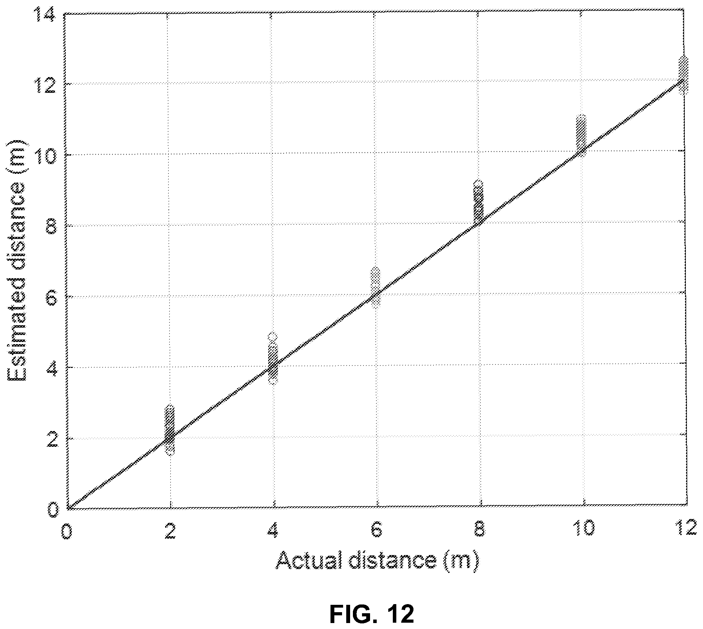

In the following, one can evaluate the performance of distance estimation. One can put the TR transmitter and receiver in a non-line-of-sight scenario in a typical office environment. A person carries the transmitter and moves with a known distance: 2m, 4m, 6m, 8m, 10m and 12m, respectively. For each specific known distance, the experiment is repeated 20 times with different paths and the walking speed does not need to be constant. The channel probing rate of our prototype is set to be 100Hz and the size of the averaging window is N=60. The result is shown in FIG. 12, where the small circles represent the estimated distance values.

The estimation is in general very accurate. There are some variances and biases in the estimation. The variances of errors come from the variance of the spatial resonating decay function especially when the channel probing rate is not high enough or the walking speed is large. When the size of the window is very large, one can do the averaging operation better but the speed must be constant during the window period, which is not the case in practice. In addition, a large size of the window can also delay the speed estimation of the current time. Thus, choosing the optimal length of the window is dependent on different application scenarios.

Since the only thing that one may need in the speed estimation algorithm is the CIRs and time-reversal resonating effect does not really happen physically, the TR-based object tracking is universal to other platforms as long as accurate CIRs between the transmitter and receiver can be obtained. For example, using Prototype II with 802.11n Wi-Fi with 3×3 MIMO configuration, the channel frequency responses (CFRs) can be obtained from each link. The raw CFRs can be sanitized to compensate symbol timing offsets, carrier frequency offsets and sampling frequency offsets and so on, using methods disclosed in PCT application PCT/US2016/066015, filed December 9, 2016, entitled "Method, Apparatus, and Systems for Wireless Event Detection and Monitoring," and PCT application PCT/US2017/015909, filed January 31, 2017, entitled "Methods, Devices, Servers, Apparatus, and Systems for Wireless Internet of Things Applications".. Then the corresponding CIRs can be derived by performing Discrete Time Fourier Transform (DTFT) to CFRs. Let *hᵢ*(*R*) denote the CIR of the i-th link at location R. If there are D links available in total, then the spatial resonating decay function between the focal point *R*₀ and the point R nearby the focal point is defined similarly as $f \left(R; {R}_{0}\right) = {\left|\frac{{\sum }_{i = 1}^{D} \left|{s}_{i}\left(0; R\right)\right|}{{\sum }_{i = 1}^{D} \left|{s}_{i}\left(0; {R}_{0}\right)\right|}\right|}^{2} ,$ (10) where *sᵢ*(0; *R*) stands for the received signal at time slot 0 and location R from link i when the transmitted signal is the time-reversed and conjugated version of *hᵢ*(*R*). The received signals from different links cannot be added up directly because that each link has its own RF chain and has different initial RF oscillator phase offsets. Thus, one can take the absolute value of the received signals before adding them together and normalize this summation such that *f*(*R*₀; *R*₀) = 1.

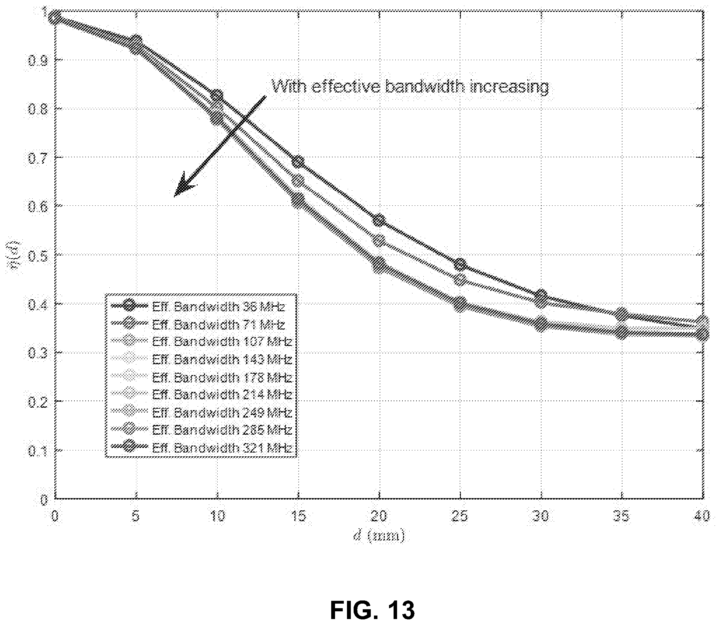

The spatial resonating decay function is affected by the system bandwidth. In the following, one can study the spatial resonating decay function with varying bandwidths. If one can utilize all the CFRs from the usable subcarriers, one can achieve an effective bandwidth B which is calculated as $B = \frac{{DN}_{u} W}{N} ,$ (11) where D is the number of links used, *Nᵤ* is the number of usable subcarriers out of N subcarriers for each link, and W is the bandwidth of each link. In Prototype II, the bandwidth for each link is set to be W=40MHz and then the effective bandwidth for each link is 114/128*40=35.625MHz. Since one can choose different number of links when computing the TR spatial resonating decay function, one can measure f(d) for different locations and the average of f(d) can be computed for varying effective bandwidth, as in FIG. 13. As one can see from the result, the average of TR resonating decay functions overlap with each other when the effective bandwidth is larger than 107MHz. One thing to note is that due to the combination scheme in equation (11), *f̅*(*d*) converges to 0.33 for larger distance d while in Prototype I *f̅*(*d*) converges to 0.22.

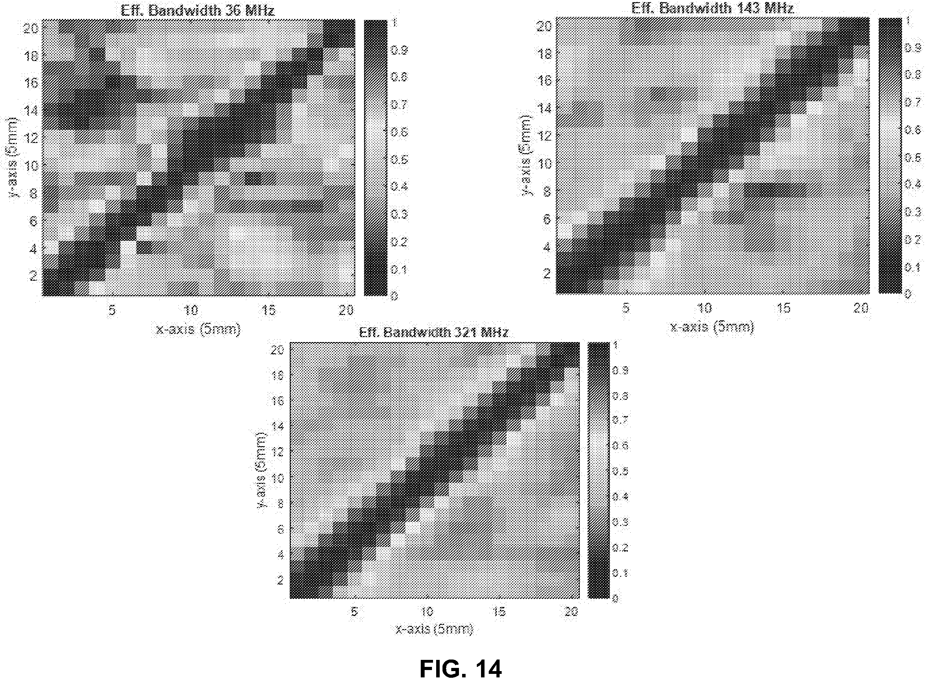

Although *f̅*(*d*) converges to the same value for larger effective bandwidth, the variances of f(d) can be smaller with larger effective bandwidth which is verified as follows. One can measure the CFRs of a square area with dimension 10cm×10cm and for each location one can measure one realization of CFR. The resolution of the measurement is 5mm, i.e., the minimum distance between two adjacent points is 5mm. First, one can choose the focal spots as the points with equal x-axis and y-axis coordinates. Then, the TR spatial resonating decay function for each focal spot is computed between the CSI of that focal spot and the CSI of the points with the same y-axis coordinates as that focal spot. FIG. 14 shows the corresponding result. When the effective bandwidth is small, the variations of f(d) is large; when the effective bandwidth is large, f(d) is more deterministic-like and is not location-dependent, which is ideal for object tracking.

### Distance Estimation based on the Ripple Property of TRRS Decay

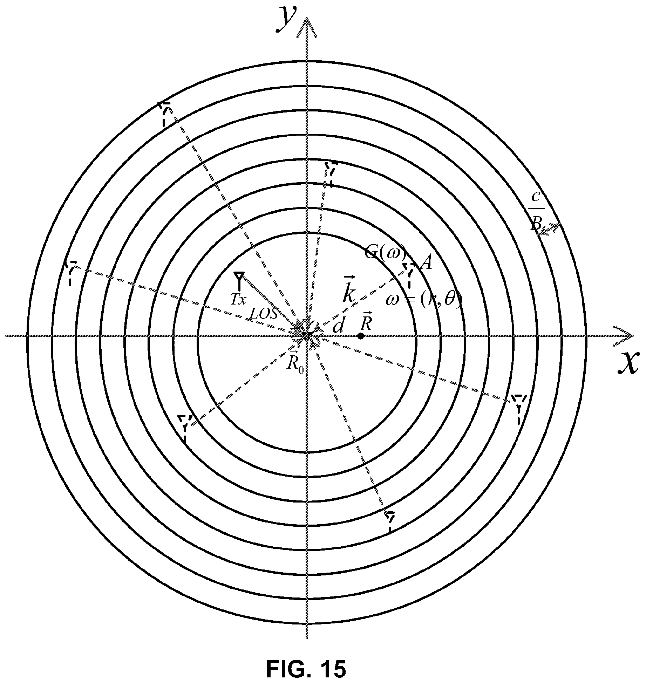

The second distance estimation method is based on the ripple property of TRRS decay. For a system with bandwidth B, two multipath components (MPCs) would be divided into different taps of the measured CIR if the difference of their time of arrival is larger than the sampling period 1/B, that is, any two MPCs with a difference of their traveled distances larger than c/B can be separated, as shown in FIG. 15. With a sufficiently large system bandwidth, the distance resolution c/B of the system is so small that all of the MPCs with significant energy can be separated in the spatial domain, i.e., each significant MPC can be represented by a single tap of a measured CIR. Assume that the distribution of the energy of each MPC is uniform in direction θ. Then the energy of MPCs coming from different directions would be approximately the same when the number of MPCs is large. Therefore, the received signal s(0;R) can be approximated as $\begin{matrix}s \left(0; R\right) = {\sum }_{ω ∈ Ω} {\left|G\left(ω\right)\right|}^{2} {e}^{- i \vec{k} ⋅ \left(\vec{R}-{\vec{R}}_{0}\right)} \\ = {\int }_{0}^{2 π} P \left(θ\right) {e}^{- ikdcos \left(θ\right)} dθ \\ = {PJ}_{0} \left(kd\right) ,\end{matrix}$ (12) where the coordinate system in FIG. 15 is used, Ω stands for the set of all significant MPCs, *J*₀(*kd*) is the 0-th-order Bessel function of the first kind, *k* is the wave vector with amplitude k=c/*f*₀, and the Euclidean distance between *̅R̅*̅ and *̅R̅*̅₀ is d. Here one can use a continuous integral to approximate the discrete sum and *P*(*θ*) = *P* denotes the density of the energy of MPCs coming from direction *θ*. For *̅R̅*̅ = *̅R̅*̅₀*,* it degenerates to the case of d=0 and thus s(0; *̅R̅*̅₀) ≈ *P*. At the same time, the numerator of the TRRS is approximately ${P}^{2} {J}_{0}^{2} \left(kd\right)$ as discussed above. As a result, the defined TRRS can be approximated as $η \left({R}_{0}, R\right) \approx {J}_{0}^{2} \left(kd\right) .$ (13)

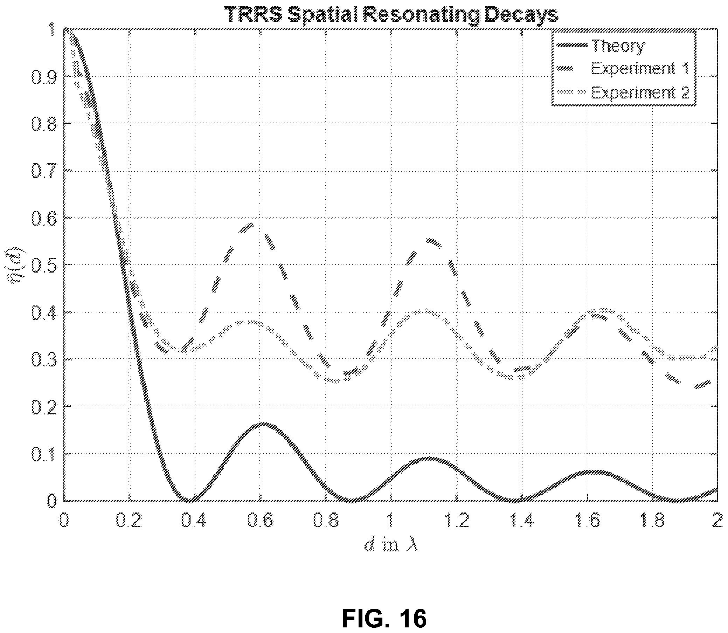

Since the theoretical approximation of the TRRS distribution only depends on the distance between two points, one can use $η \left(d\right) \approx {J}_{0}^{2} \left(kd\right)$ to stand for the approximation of TRRS between two points with distance d. The comparison between the above theoretic curve and the experiment measurements are shown in FIG. 16, which can validate (18). It may be seen that the peaks of the three curves appear at the same *d* values, meaning the ripples have similar shapes, so one can use such ripple property to estimate the moving distance.

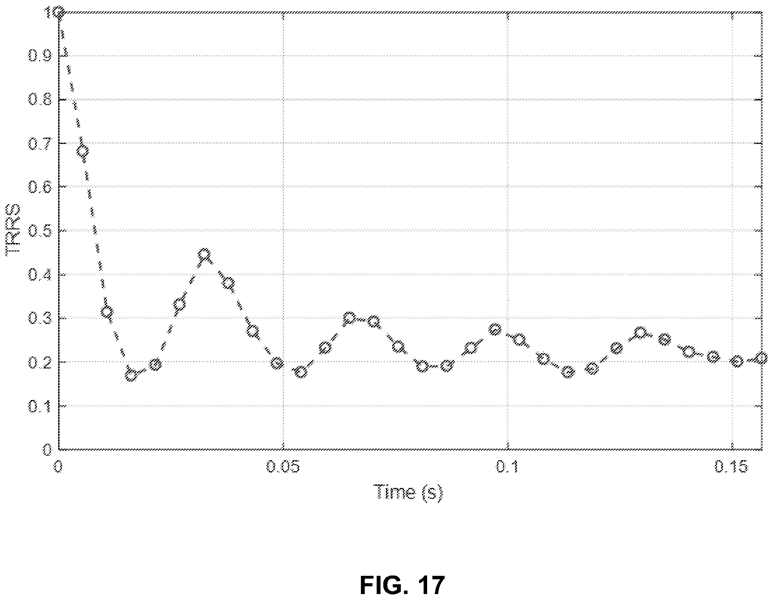

Since the shape of the TRRS distribution function $η \left(d\right) \approx {J}_{0}^{2} \left(kd\right)$ is only determined by the wave number k which is independent of specific locations, it can be utilized as an intrinsic ruler to measure distance in space. In one embodiment, consider that one receiver moves along a straight line with a constant speed v starting from location *R*₀, and one transmitter keeps transmitting the TR waveform (i.e., time-reversed and conjugated version of the received signal) corresponding to *R*₀ at regular intervals. Then, the TRRS measured at the receiver is just a sampled version of *η*(*d*), which would also exhibit the Bessel-function-like pattern, as illustrated in FIG. 17.

Take the first local peak of *η*(*d*) for example. The corresponding theoretical distance *d*₁ is about 0.61λ. To estimate the moving speed, one may only need to estimate how much time *t̂* it takes for the TR receiver to reach the first local peak starting from point *R*₀. One can use a quadratic curve to approximate the shape of the first local peak. Combining the knowledge of the timestamps of each CIR measurement *t̂* is estimated by the vertex of the quadratic curve. Therefore, one can obtain the speed estimation as $\hat{v} = \frac{0.61 λ}{\hat{t}}$. One thing to note is that if the rate of CIR measurement is fast enough, the assumption that the moving speed is constant within the duration of the measurement of the TRRS distribution function is reasonable in practice. For example, in FIG. 17 the duration is about 0.16 seconds.

Multiple realizations of the TRRS distribution function measured at adjacent time slots can be combined to increase the accuracy of the estimation of *t̂*. For the i-th measurement, first find the data points near the first local peak (*t_{i,j}, y_{i,j}*), i=1,...,N, j=1,2,3. Then fit the data points with a quadratic regression model ${y}_{i , j} = α + β {t}_{i , j} + {γt}_{i , j}^{2} + {e}_{i , j}$, and thus estimation of the elapsed time is $\hat{t} = - \frac{{\hat{β}}_{LS}}{2 {\hat{γ}}_{LS}}$, where *β̂_{LS}* and *γ̂_{LS}* are the least-square estimators of *β* and *γ*, respectively. Different reference points can be used as well, such as the first local valley, the second local peak and so on, to increase the accuracy of estimation. Algorithm 2 summarizes the second method for moving distance estimation.

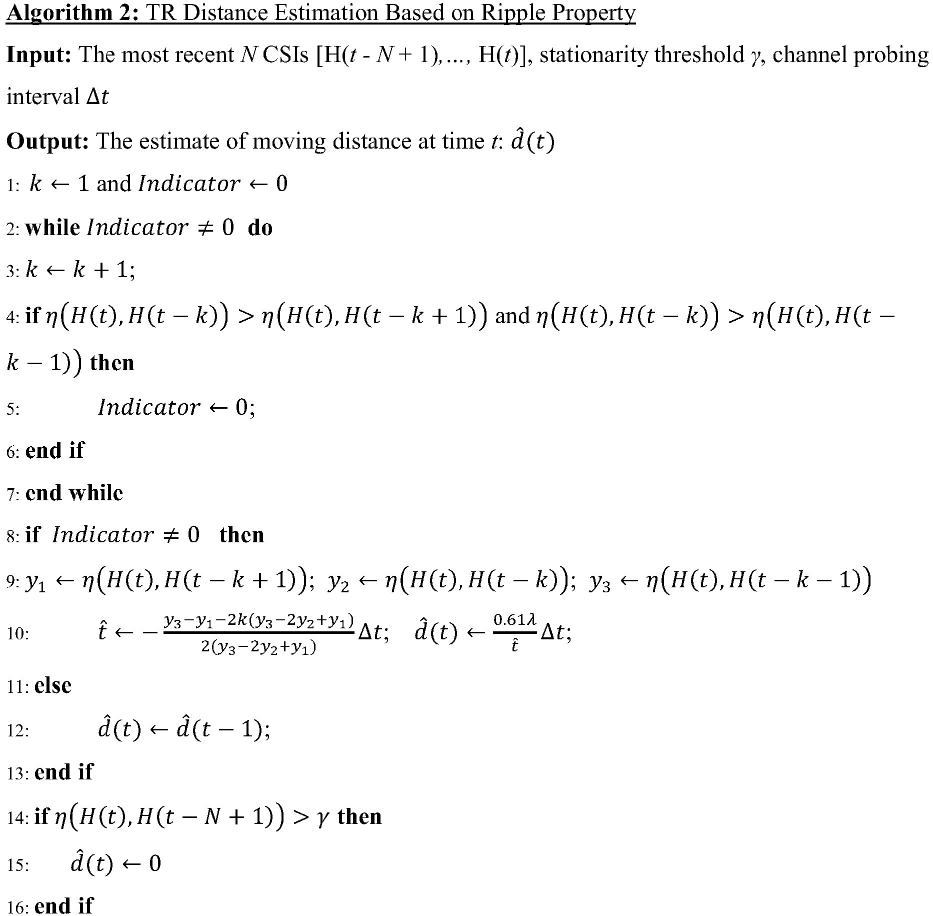

In Algorithm 2, one can choose the newly collected CSI *H(t)* as a reference and compute the TRRS between *H(t)* and previously collected CSIs in the CSI buffer. As mentioned previously in Algorithm 1, if the TRRS values between adjacent CSIs are above some threshold, the object can be viewed as not moving. If the object is determined to have moved, the TRRS value *η*(*H*(*t*), *H*(*t - k* + 1)) would decay as *k* increases and exhibit the pattern as described by equation (13). Based on the ripple property, one can know that the first local peak always corresponds to a distance of around 0.61*λ* from the original starting point. If one can know the time duration it would take the object to move to the position corresponding to the first local peak, one can get the estimated moving speed. To improve the estimation accuracy of the position of the first local peak of the TRRS decay, one can use a quadratic curve to approximate the TRRS distribution nearby the first local peak. Then, a better estimation of the time duration *t̂* that the object needs to move a distance of 0.61*λ* can be obtained. Since the CSIs are collected in every *Δt,* the distance *d̂*(*t*) the object moves within the sample period from *t -* 1 to *t* can be estimated as in line 10 of Algorithm 2. Again, for the similar reason as Algorithm 1, if the TRRS value between the newest CSI and the oldest CSI in the CSI buffer is very large, then the distance estimation is set to be 0.

In the above method of object tracking, at least one of the transmitter and the receiver is carried by the moving object/person and the method can be viewed as active tracking, as shown in FIG. 1A. In another embodiment, as shown in FIG. 1B, the moving object/person only needs to carry a sensor for direction estimation and the transmitter and the receiver are at fixed locations. Since the multipath channel depends on the scatterers in between them, the moving object/person has a large enough surface and can be viewed as a mass of scatterers moving at the same speed. In this manner, the multipath channel between the transmitter and receiver is affected by the movement of the object/person and the CSI obtained from the received signal at the receiver demonstrates a pattern reflecting some feature about the movement, for example, the moving speed.

In one embodiment, one can assume that each scatterer has a rough surface and the incoming wireless signals are re-radiated in numerous directions with a uniform distribution; assume that the *i*-th scatterer in the environment is moving towards some direction with a certain speed v; and let *E̅ᵢ*(*t*) denote the change of the received electric field at the receiver. Based on the property of channel reciprocity, if the receiver were transmitting a wireless signal, the electromagnetic (EM) waves would follow exactly the same paths between the *i*-th scatterer and the receiver. Therefore, *E̅ᵢ*(*t*) is equal to the vector summation of all the arriving EM waves which are also distributed uniformly in the direction of arrivals. According to the statistical theory of EM waves in cavities, the autocorrelation function (ACF) of |*̅E̅*̅ᵢ(t)|² would follow: ${ρ}_{EE} \left(vt\right) = \frac{2 {ρ}_{t}^{2} \left(vt\right) + {ρ}_{l}^{2} \left(vt\right)}{3}$, where ${ρ}_{t} \left(vt\right) = \frac{3}{2} \left[\frac{sin \left(kvt\right)}{kvt}-\frac{1}{{\left(kvt\right)}^{2}}\left(\frac{sin \left(kvt\right)}{kvt}-cos\left(kvt\right)\right)\right] , {ρ}_{l} \left(vt\right) = \frac{3}{{\left(kvt\right)}^{2}} \left[\frac{sin \left(kvt\right)}{kvt}-cos\left(kvt\right)\right] , k = \frac{2 π}{λ}$ , and *λ* is the wavelength of the transmitted signal. Therefore, by examining the ACF of the received signal at the receiver which exhibits similar pattern of the ACF of |*E̅ᵢ*(t)|², one can estimate the speed of the moving object in a passive way. Although TRRS is not directly used in the passive speed estimation, the ACF can also be viewed as a measurement of correlation between different CSIs collected at different points along the moving path.

Assuming the most recent *N* CSIs estimated from the received signal are [H(*t - N +* 1), ..., H(t)] with N as the time window length, one can define the ACF as a function about the square of the CSI amplitude, which does not require phase offset cleaning. One can use sample mean to approximate the expectation operation in the ACF, i.e., using CSIs between adjacent CSI pairs to get sample mean of the ACF with time lag 1, using CSIs between {H(i),H(i-2)}, for i=t-N+3,...,t, to get sample mean of the ACF with time lag 2, and so on. It can be shown that the ACF function also demonstrates a ripple property.

Then, one can find a feature point on the ACF curve that is related to the movement pattern, e.g., the first local peak. One can use the first peak ACF value and the two adjacent ACF values to estimate the time corresponding to the first local peak and obtain the speed assuming the speed is uniform during the time to reach the first local peak. Then, one can obtain the moving distance using the estimated speed and the sampling period. If the ACF value between the CSIs at the beginning and the end of the time window is above a threshold, the moving distance may be estimated to be 0; otherwise, the estimated distance may equal to the product of the estimated speed and the sampling period.

### Direction Estimation

In the following, one can introduce two direction estimation methods as the second module of TRITS, that is, the moving direction estimation module. The first one utilizes the inertial measurement unit (IMU) while the second one utilizes the TRRS decay function η(*d*) to estimate the moving direction of the transmitter.

Since one usually only cares about the change of moving direction in the *x-y* plane which is orthogonal to the direction of gravity g, one can project the rotations from *x, y* and z-axis onto g, where g is measured by the on-chip coordinate system. The rotations can be obtained from the readings of the gyroscope. Thus, the moving direction at time t can be estimated as $\hat{θ} \left(t\right) = \hat{θ} \left(t-1\right) + Δ {tω}^{T} g / \left‖g\right‖ ,$ (14) where ***ω*** is the readings of the gyroscope, Δ*t* is the sampling period and ***g*** is the readings of the gravity sensor. Algorithm 3 summarizes the direction estimation method based on IMU as follows.

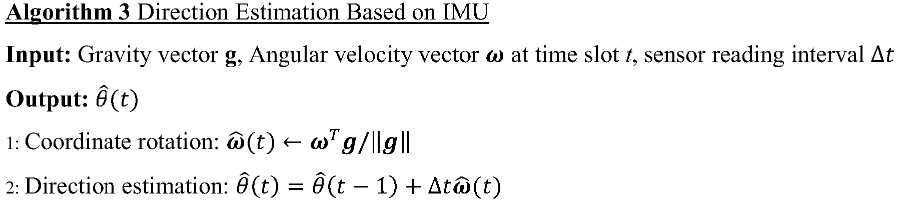

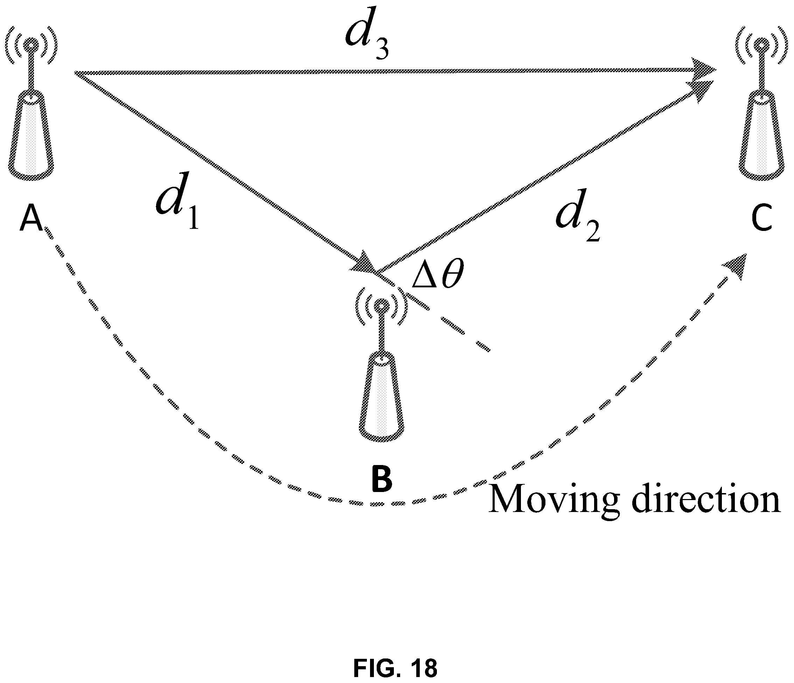

For the second method of moving direction estimation, assume that the receiver moves from location A 1802 to location B 1804 and then location C 1806 as shown in FIG. 18. The three locations stand for the location of the transmitter for three consecutive CSIs. Assume that the channel probing rate is fast enough such that the distances among these three locations *dᵢ* are small enough and the one-to-one mapping relationship between distance and TRRS value still holds. Then the change of the angle of the moving direction can be estimated by the Cosine Rule as $Δθ = π - arccos \left(\frac{{d}_{1}^{2} - {d}_{2}^{2} - {d}_{3}^{2}}{2 {d}_{1} {d}_{2}}\right) ,$ (15) where *dᵢ*'s are obtained by taking the inverse of the TRRS decay function. Therefore, the estimate of moving direction is *θ̂*(t) = *θ̂*(t - 1) + Δθ.

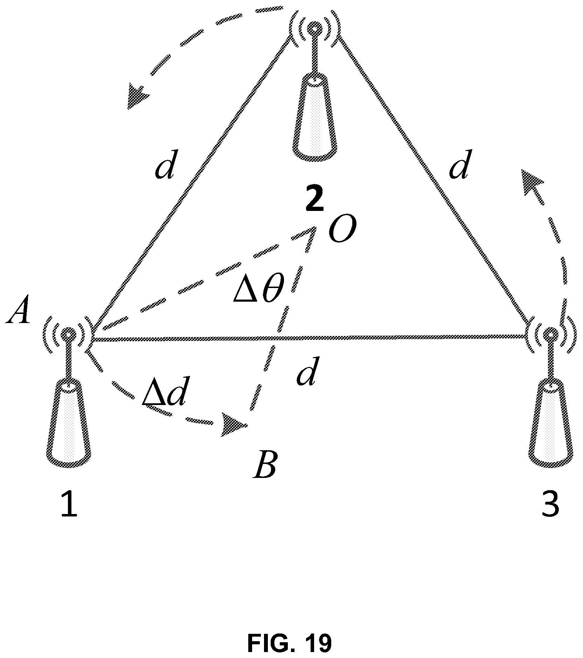

When the transmitter is equipped with multiple antennas located close to each other, for example, as shown in FIG. 19, the rotation of the transmitter can be computed by $Δθ \approx \frac{\sqrt{3} Δ d}{d} ,$ (16) where Δθ is obtained from the TRRS decay for antenna 1 moving from A to B and assume that Δ*d* is small enough which is the case when the channel probing rate is high enough. The direction of the rotation can be determined by computing the TRRS among different antennas. For example, if the TRRS between the CSI measured by antenna 3 at time t and the CSI measured by antenna 1 at time t+1 increases, then the rotation is counter-clockwise. The accuracy of the estimation of Δθ can be improved by averaging the estimation from different antenna selections.

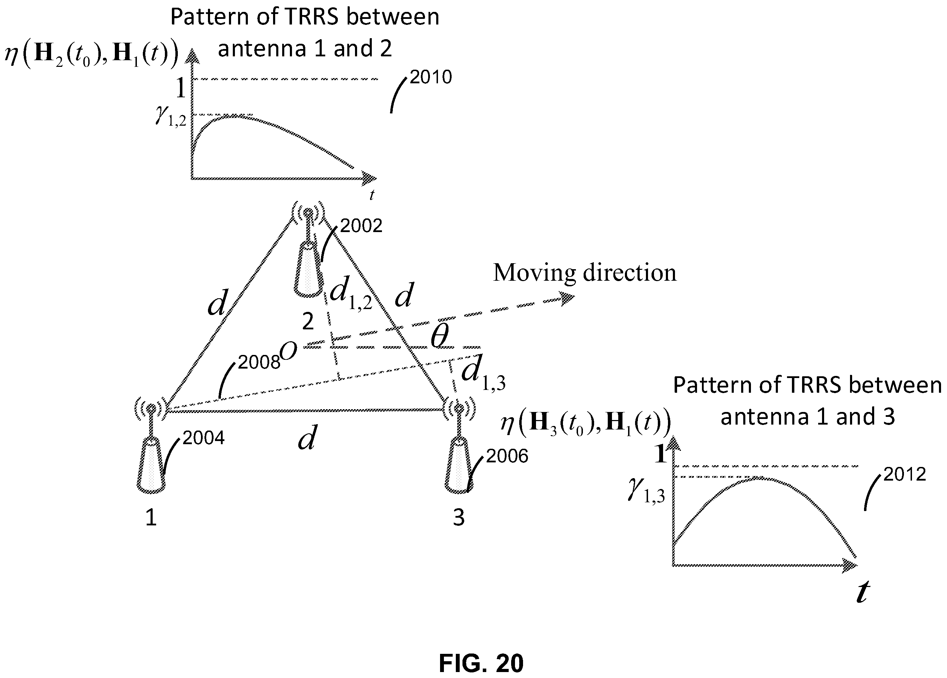

The moving direction relative to the TR device can also be estimated as follows. See FIG. 20 as an illustration where the three antennas 2002, 2004, and 2006 are located at the vertexes of an equilateral triangle. In this example, one can use *Hᵢ* to denote the CSI obtained from the channel probing signal sent from the transmitter to the i-th receive antenna on the receiver and use η(*Hᵢ*(*t*₀), *Hⱼ*(t)) to denote the TRRS between *Hᵢ* measured at time *t*₀ and *Hⱼ* measured at time t. For a specific moving direction as shown in FIG. 20, the function *η*(*Hᵢ*(*t*₀), *Hⱼ*(t)) exhibits distinct patterns when t ≤ *t*₀, which are shown in the figure as well. Since antenna 1 2004 will pass the route 2008 that is first close to antenna 2 and 3 and then is far away from the antennas, *η*(*H*₂(*t*₀), *H*₁(*t*)) and η(*H*₃(*t*₀), *H*₁(*t*)) exhibit the patterns 2010 and 2012 as shown in the figure. Note that the locations and the number of antennas are not restricted and they can be placed in other geometric shapes. Through the peak value of function η(*Hᵢ*(*t*₀), *Hⱼ*(*t*)), the minimum distance between antenna i and j along the moving direction can be determined. For instance, when η(*H*₂(*t*₀), *H*₁(t)) reaches the local maximum *γ*_{1,2}, antenna 1's current location is at distance *d*_{1,2} away from antenna 2's initial location, and *d*_{1,2} can be estimated through the location-TRRS mapping. The moving direction relative to the TR device can be estimated as *θ̂* = arcsin(*d*_{1,3}/*d*) or $\hat{θ} = \frac{π}{3} - arcsin \left({d}_{1 , 2}/d\right)$ in the example, where *d*_{1,3} and *d*_{1,2} are obtained from the TRRS decay value *γ*_{1,2} and *γ*_{1,3}, which are the maximum TRRS values of η(*H*₂(*t*₀), *H*₁(t)) and η(*H*₃(*t*₀), *H*₁(t)), respectively, as shown in the FIG. 20. One inherent assumption is that the channel probing rate of the system is high enough so that *γ*_{1,2} and *γ*_{1,3} are accurate enough.

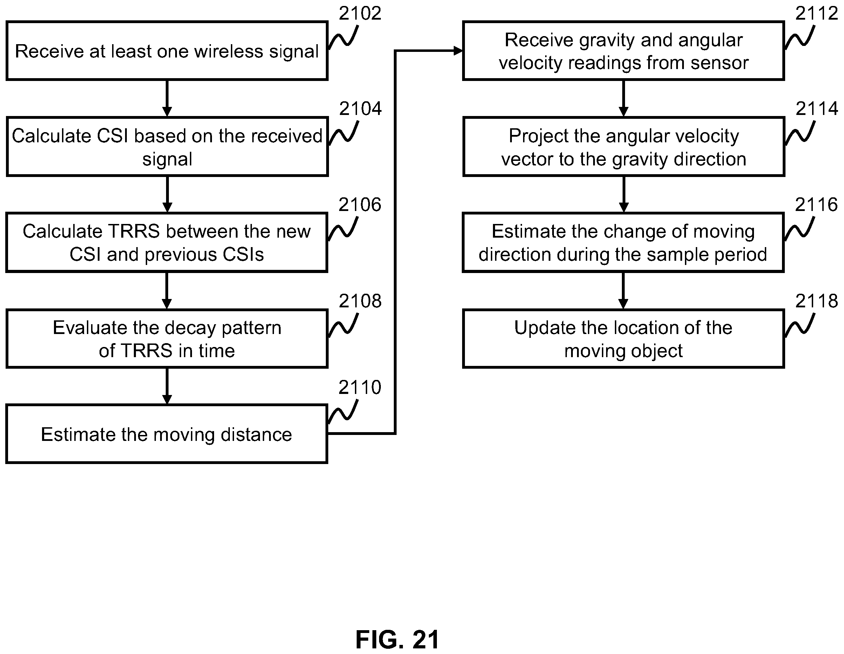

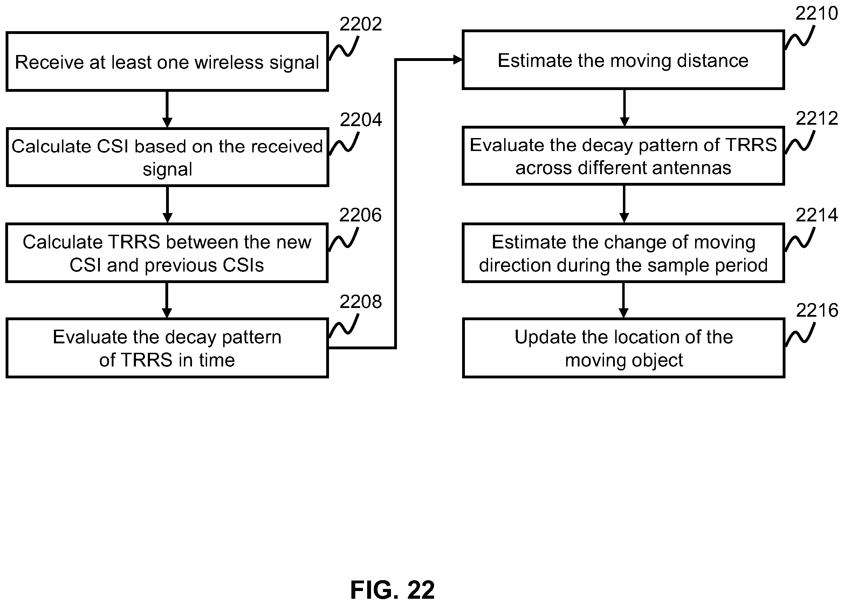

In one embodiment, a flow chart showing a process of the disclosed object tracking is shown in FIG. 21. A transmitter carried by the moving object transmits at least one wireless signal to a receiver 2102. At least one CSI can be estimated based on the received signal, and the phase offsets in the CSIs can be cleaned 2104. The TRRS values between the most recent CSI and previously collected CSIs in a time window can be calculated 2106, which demonstrate some decay pattern of the TRRS in time 2108. TRRS values in multiple such time windows can be averaged to get a smoothed decay pattern. Based on the converging property of the TR resonating effect (according to Algorithm 1) or the ripple property (according to Algorithm 2), the moving distance of the object can be estimated 2110. From the direction sensor (e.g. IMU) attached to the moving object, the angular velocity and gravity information can be read 2112. The angular velocity can be projected 2114 to the gravity direction, and the change of the moving direction can be estimated 2116 according to Algorithm 3. Finally, the location of the moving object is updated 2118 based on the estimated moving distance and direction. In another embodiment, a flow chart showing another process of the disclosed object tracking is shown in FIG. 22, where the moving direction is estimated based on the decay pattern of the TRRS across different antennas (2212 and 2214).

In another embodiment, the moving speed of the object can be estimated without attaching the transmitter to the object. The motion of the object will affect the CSI characteristics, where the CSIs are obtained based on the channel sounding signals sent from a transmitter at a fixed location to a receiver at another fixed location. Other functions about the CSIs can be used to extract the time-varying pattern of the CSIs, such as an autocorrelation function of the CSIs, an amplitude function of the CSIs, a phase function of the CSIs, etc.

### Direction Estimation using Different Types of Sensors

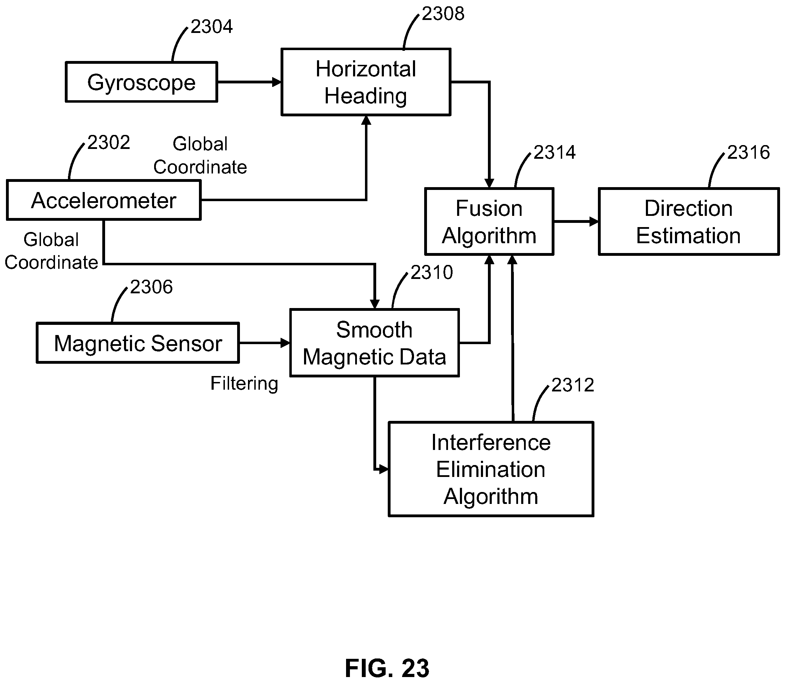

The other types of sensor outputs can also be used to improve the accuracy of direction estimation. One such example is shown in FIG. 23 which takes advantage of complementary features of different sensors and determines the moving direction using fused sensor outputs. From the accelerometer, one can know 2302 the global coordinate and the direction of gravity *g*. The gyroscope sensor 2304 can be projected in the direction of *g* and the horizontal heading 2308 can be obtained. Based on the global coordinate from the accelerometer, the magnetic sensor output 2306 can also be projected in the horizontal plane and then filtered to get the smoothed magnetic sensor data 2310. Interference elimination algorithm2312 can be designed to alleviate the impact of interfering magnetic source. Then the processed data from two types of sensors (gyroscope and magnetic sensor) can be fused 2314 to estimate the moving direction 2316.

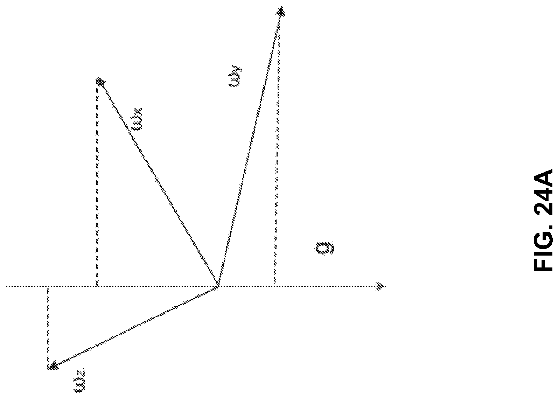

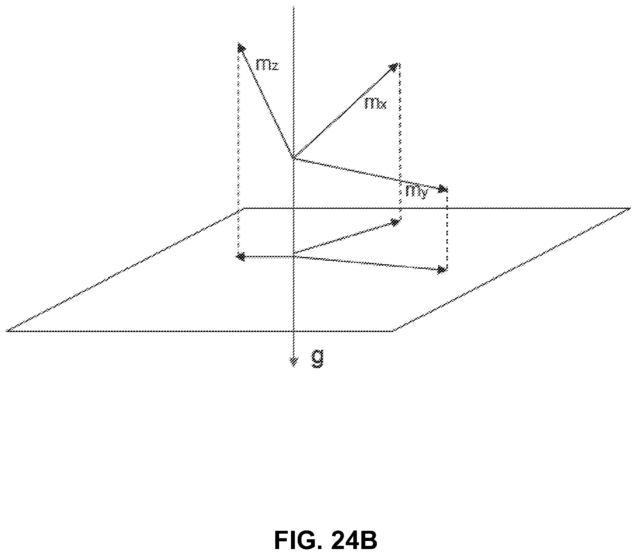

As shown in FIG. 24A, the gyroscope sensor output vector can be projected in the direction of gravity *g* as *ω_{gz} = ωₓ · g + ω_{y} · g + ω_{z} · g.* As shown in FIG. 24B, the magnetic strength vector can also be projected in horizontal plane.

The objective of projecting the magnetic strength vector in horizontal plane is to get the global horizontal component of the magnetic strength vector and compare it with the global axis to get the global direction of moving.

Since the coordinate system available in this problem is the local coordinate system of the sensor, firstly one may need to express three axes of the global coordinate system in the local coordinate system. Then one may need to project the magnetic strength vector onto the global horizontal plane. Finally, one can compare the horizontal component of the vector with the global axis to determine the heading direction. One assumption is that the heading direction is fixed with the horizontal component of local x-axis, i.e., the difference between heading direction and object moving direction does not change with time.

Some notations are as follows.

| |
|---|
| Local axis: *xₗ, yₗ, zₗ* |
| Global axis: *x_{g}*, *y_{g}*, *z_{g}* |
| Magnetic strength: ${{m}_{ι}}^{⇀}$ |
| (local axis component), ${{m}_{h}}^{⇀}$ |
| (Global horizontal component), ${{m}_{ih}}^{⇀}$ |
| (Global horizontal component on local axis), ${{m}_{iz}}^{⇀}$ |
| (Global vertical component on local axis) |

Detailed description of projecting the magnetic strength vector in horizontal plane is as follows:
(1) Find the global coordinate axis
   The z-axis *z_{g}* is given by the accelerometer (gravity). The x-axis *x_{g}* is obtained by subtracting its global vertical component via *x_{g} = xₗ - x_{lzg}*). Having *x_{g}* and *z_{g},* by orthogonality one can have ${{y}^{⇀}}_{g} = {{z}^{⇀}}_{g} \times {{x}^{⇀}}_{g}$.
(2) Project magnetic vector
   By subtracting the global vertical components of the magnetic strength vector, one can get the global horizontal component of the magnetic strength vector: ${{m}_{h}}^{⇀} = {\sum }_{i = x , y , z} {{m}_{ih}}^{⇀} = {\sum }_{i = x , y , z} {{m}_{i}}^{⇀} - {{m}_{iz}}^{⇀}$ , where ${{m}_{iz}}^{⇀} = {{m}_{i}}^{⇀} ⋅ \frac{{g}^{⇀}}{\left|{g}^{⇀}\right|}$.
(3) Obtain the direction
After having the ${{x}^{⇀}}_{g} , {{y}^{⇀}}_{g}$ and ${{m}_{h}}^{⇀}$, one can could apply ${θ}_{1} = {cos}^{- 1} \left({{m}_{h}}^{⇀}⋅{{x}^{⇀}}_{g}\right)$ and ${θ}_{2} = {cos}^{- 1} \left({{m}_{h}}^{⇀}⋅{{z}^{⇀}}_{g}\right)$ to determine the direction ${{x}^{⇀}}_{g}$ of heading.

It can be found that various types of sensors may have complementary features as shown in the table below. Therefore, one can fuse different types of sensor outputs to improve direction estimation accuracy.

| | Advantage | Disadvantage |
|---|---|---|
| Gyroscope | No external disturbances | Relative azimuth drift |
| | Short term stable accuracy | Error accumulation |
| Magnetic Sensor | Absolute azimuth | Unpredictable external disturbances |
| | Long term stable accuracy | |

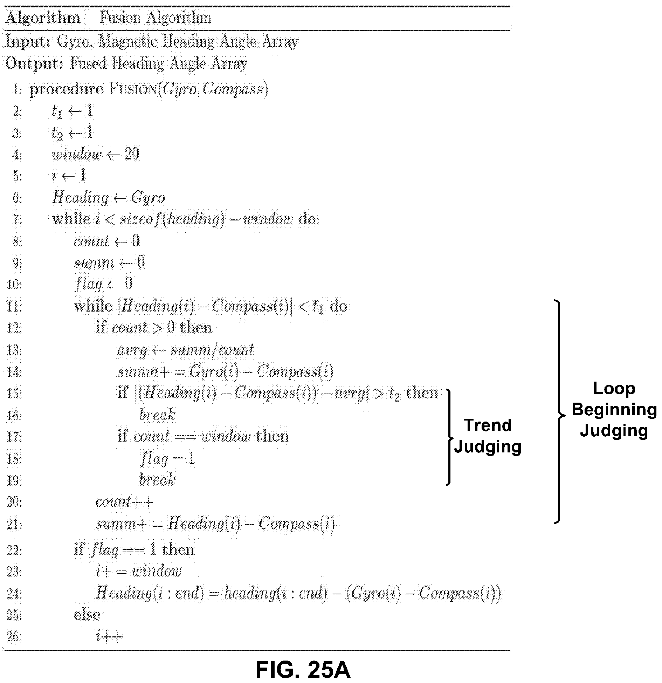

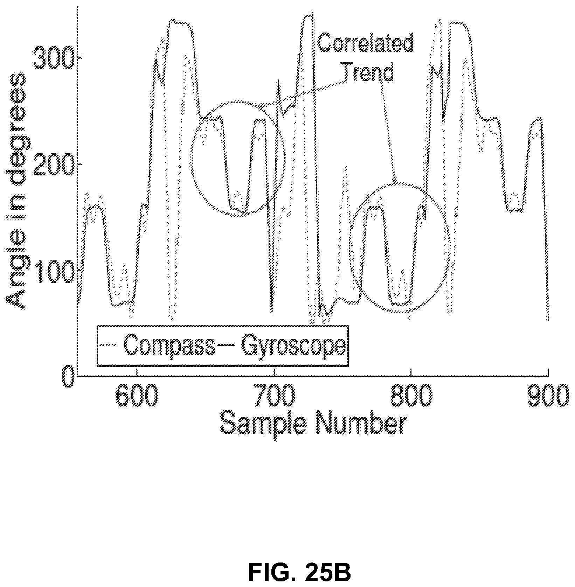

One example of sensor fusion is shown in FIG. 25A and FIG. 25B. The idea is to adjust gyroscope to magnetic sensor reading when 1) the difference of the two readings are within a certain range and 2) the trend of the two readings cohere to each other, e.g., as shown in FIG. 25B. In the algorithm, ***t1*** is readings difference threshold (Loop begin judgement), ***t2*** is the trend judging threshold, and ***window*** is trend judging period length. Line 11 judges the difference between the two readings and decides whether to begin the loop. When the loop begins, ***avrg*** is the average difference between the two readings since the loop begins. If the difference between the two readings is within a certain range (***t2***) around the ***avrg,*** the algorithm concludes that the trend continues. The ***count*** accumulates when the current sample point is still within the trend. When ***count*** reaches the ***window,*** the heading data is adjusted to the compass reading.

### An Example Implementation of the Object Tracking System

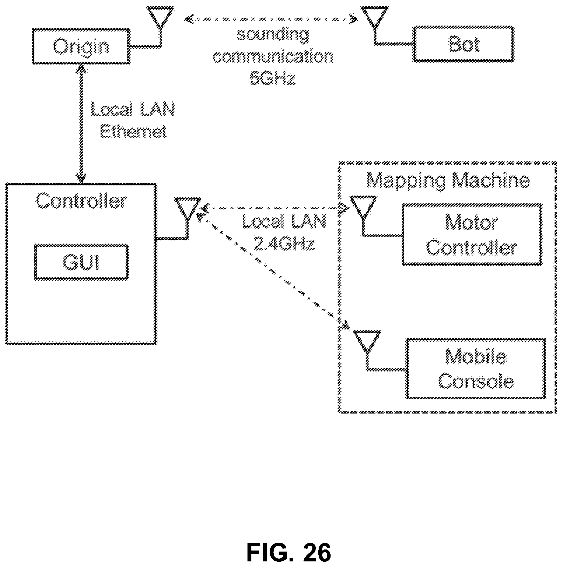

An exemplary functional block diagram of an implementation of the tracking system is shown in FIG. 26. The example tracking system is composed of Origin subsystem, Bot subsystem, Controller subsystem, and mapping machine subsystem.

**Origin Subsystem:** The Origin Subsystem is one or more stationary transceivers (each an "Origin") that communicate directly with and control the Bot Subsystem using identifiers that are specific to each Bot and collect multipath radio signatures that are specific to location of the Origin and the location of each Bot. The Origin Subsystem sends the collected signatures to the Controller Subsystem, which processes the signatures to track Bots.

**Bot Subsystem:** The Bot Subsystem is one or more mobile transceivers tags (each a "Bot") that communicate directly with and are under the control of an Origin. A Bot is tracked using identifiers that are specific to the Bot and multipath radio signatures that are specific to the location of the Bot and the location of the Origin.

**Mapping Subsystem:** The Mapping Subsystem is composed of a 3D mapping table, a motor controller, and a mobile console. The motor controller is capable of moving the 3D mapping table, which carries a Bot over the whole area of Virtual Checkpoints (each a "VC") at a configured speed. The Controller Subsystem controls the whole mapping process including the motor controller and the Origin Subsystem to collect multipath radio signatures from the Bot at the VC. The mobile console enables the remote control of the Controller Subsystem in the mapping process.

**Controller Subsystem:** The Controller Subsystem is a computer system that controls the Origin Subsystem, the Bot Subsystem (through the Origin Subsystem), and the Mapping Subsystem during the mapping process and the tracking process. It includes a graphical user interface ("GUI") for interacting within the System and reporting real-time Bot location, history, and regions for which Bots have permission to operate ("Locate Area Privileges"). The Controller Subsystem also sets and updates Location Area Privileges for each Bot. The Controller Subsystem may include at least one computer running a Windows 10+ operating system and may also include other computing resources/or processors.

In one embodiment, the connections between these components are as follows. The Origin Subsystem communicates with the Bot Subsystems wirelessly via 5GHz Wi-Fi channels, which are compliant with all applicable FCC rules and regulations. The Origin Subsystem and the Controller Subsystem shall communicate via Ethernet. The Mapping Subsystem is connected to the Controller Subsystem through 2.4GHz wireless LAN network. The Controller Subsystem collects the multipath radio signatures from a Bot on one or multiple VCs offline, which are later used to track the Bots online.

In one embodiment, the functionalities of these components are as follows. The Bot subsystem can ping/beacon channel probe signal to the Origin Subsystem based on the command sent by Origin Subsystem. The probe signal contains both the necessary signal to estimate CSI and heading/direction information provided by the Bot Subsystem in the data payload. The Origin Subsystem, controlled by the Controller Subsystem, can deliver the command signal to the Bot Subsystem. Moreover, it is capable to receive the channel probe signal from the Bot Subsystem. After receiving the channel probe signal, Origin Subsystem can derive the CSI and heading information for the Bot Subsystem at the current location, which is then provided to the Controller Subsystem. The Controller Subsystem is the controller of the whole system, which can be a PC station with certain computation and communication capabilities. The Controller Subsystem can control the Origin Subsystem and thus the Bot Subsystem. Moreover, the Mapping Machine Subsystem is also controlled by the Controller Subsystem in terms of the motion. The Controller Subsystem, based on the CSI and heading/direction information sent by Origin Subsystem, can report the location of Bot Subsystem in real-time. A GUI is included in the Controller Subsystem providing the operator with the map information and Virtual Checkpoint configurations. The privileged area of Bot Subsystem is configured in the GUI as well. When the Bot Subsystem enters the area of privilege, an alarm will be triggered in the GUI.

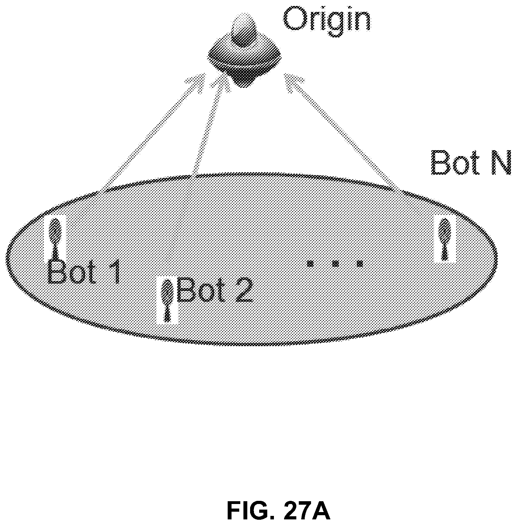

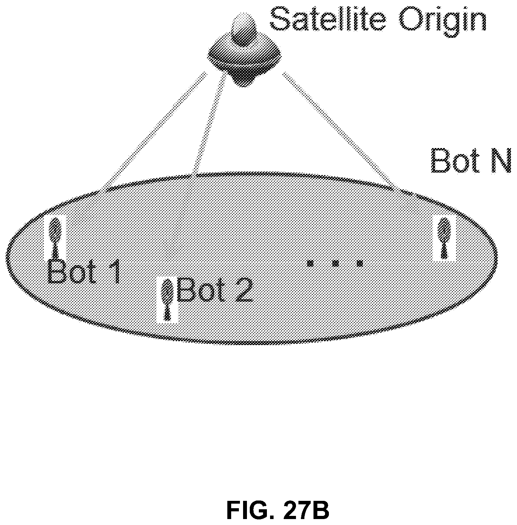

In another embodiment, an exemplary tracking system with multiple object (Bots) to be tracked is shown in FIG. 27A. The Bots take turns to transmit channel sounding signal to the Origin. In other words, they are time-sharing with each other, and it might be difficult to maintain a high sounding rate from the Bots to the Origin, if there is a very large number of Bots. Alternatively, the system architecture can be based on downlink, as shown in FIG. 27B, where the Bots estimate the CSI based on the sounding signal sent from the Origin to the Bots. After that, each Bot computes its coordinates and feedbacks such information to the Origin at a much lower rate compared with channel sounding rate. In such a way, the architecture in FIG. 27B theoretically can support an unlimited number of Bots simultaneously.

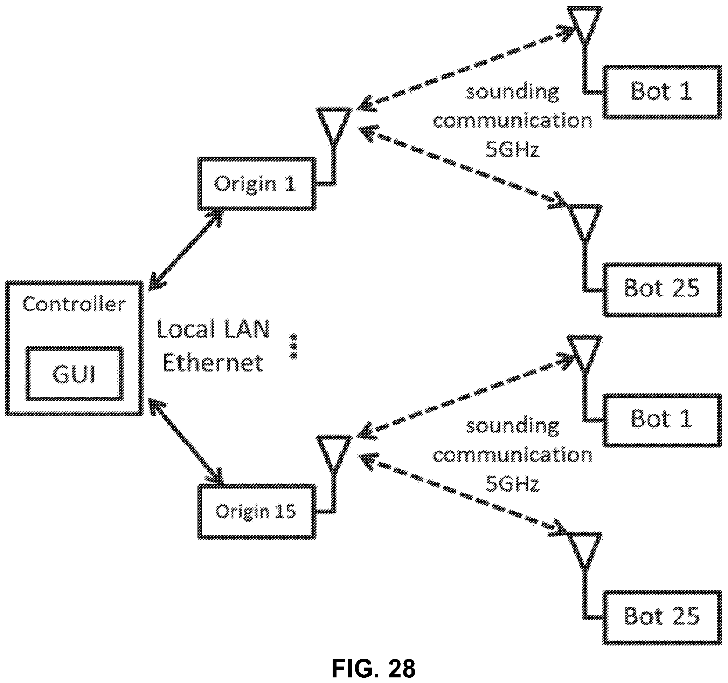

An exemplary functional block diagram corresponding to the architecture in FIG. 27B is shown in FIG. 28. The Origin subsystem broadcasts sounding signal and communicates with the Bot Subsystem wirelessly via Wi-Fi channels, which are compliant with all applicable FCC regulations. The Origin Subsystem and the Controller Subsystem may communicate via Ethernet. Through the Origin, the Controller subsystem collects Bots coordinates, which are used to track Bots in real time.

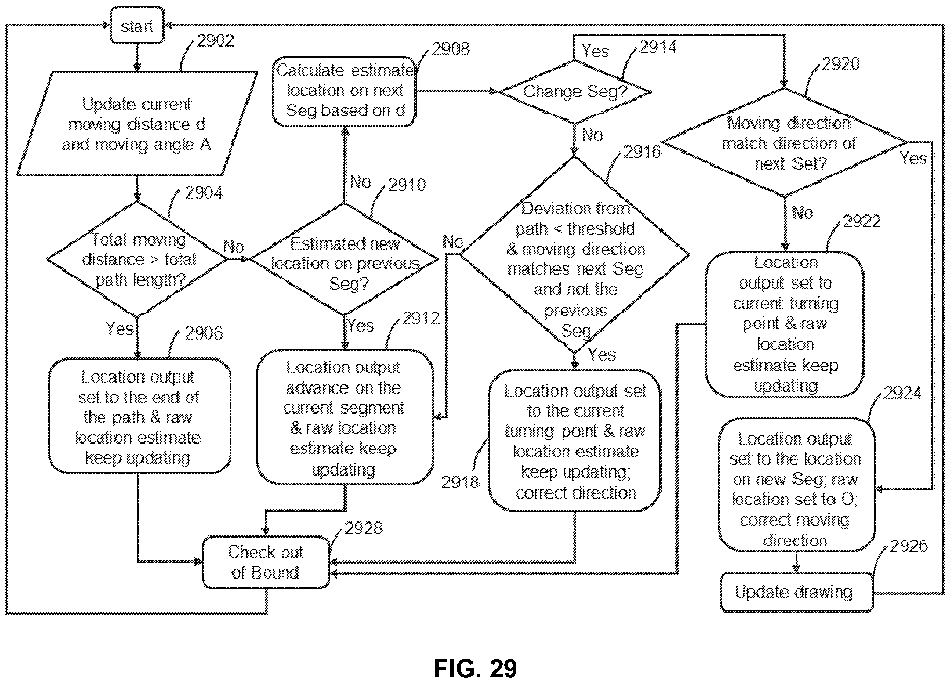

An exemplary software implementation is shown in the flow chart in FIG. 29. Note that the path information is assumed to be known before tracking the object, which can assist in determining the object location. For example, the turning points on the path divide the path into several segments. If the object makes a turn but its trajectory deviates from the direction of the new segment, the location error can be corrected by "mapping" the raw object location to the correct direction on the new segment. The notations used in FIG. 29 are listed as follows.
- d: current moving distance derived from the system
- A: current moving angle
- D: accumulated distance from the starting point
- End: ending distance of the path
- Seg: path segment of the object, which is determined by the turning points on the path
- O: mapped estimated location of object on the path, meaning the location output after incorporating the path information
- <>: estimated location of object in the free space which can be viewed as raw location estimate

Given the current moving distance derived from TR Machine (d) and current moving angle (A), the total distance travelled by the object (D+d) is compared with the total distance of the whole path (2904). If (D+d) is larger than the length of whole path, then location output O is placed at the end of path, while the raw location estimate <> keeps updating (2906). If (D+d) is smaller than the length of whole path, and the estimated new location is still on the previous segment of the path (2910), then the location output O advances to the new location on the current segment and the raw location estimate <> keeps updating (2912). If the estimated location exceeds the current segment of the path, the accumulated (D+d) is updated (2908). Based on the accumulated length of each segment of paths and (D+d), the system will determine whether O arrives at a new segment or still remains at the previous segment (2914). If O arrives at a new segment, the system will evaluate whether the moving direction matches the direction of the new segment (2920). If the direction matches the new segment, O is placed on the new segment, <> gets updated and moving direction is corrected to the new segment (2924). Then, the drawing of the output trajectory is updated (2926). Otherwise, O will stay at the end of last segment, while <> keeps updating (2922). If (D+d) demonstrates that O is still on the current segment of path (evaluation of 2914 is No), the system will evaluate the following conditions. If (D+d) is within a threshold distance (e.g., 2 meters but other values can be adopted too) from the end of current segment, and the direction matches the new segment instead of the previous segment (evaluation of 2916 is Yes), then O is set to the beginning of new segment while <> keeps updating, and direction is corrected (2918). Otherwise, O advances on the current segment and <> keeps updating (2912). The raw location estimate and the location output are checked from time to time to see if the object location deviates too much from the path (2928).

*Calibration:* When the environment fails to have enough scatterers or the antennas of the tracking device are blocked by surrounding obstacles, such as human body, backpack and clothing, then calibration procedure may be needed to compensate this disadvantage. The system should be carried to go through the path with a near constant speed at first. The total time that the device is moving can be computed by taking the difference between the stopping timestamp and starting timestamp of the motion. The actual average moving speed is calculated as dividing the total length of the fixed path by the total time. The path is divided into N segments with equal length, where N is proportional to the total length of the path. For each segment, the scaling factor is defined as the ratio between the actual average moving speed and the estimated average moving speed of that segment, where the estimated moving speed is computed as dividing the estimated length of that segment by the time spent on that segment. The scaling factor of each segment is saved as a vector corresponding to the fixed path. After the calibration procedure, the estimated distance is multiplied by the scaling factor of the segment that the device is on, which can be obtained by previous location estimations.

The mapping subsystem can be implemented based on the method disclosed in U.S. patent application 14/605,611, titled "WIRELESS POSITIONING SYSTEMS," filed on January 26, 2015, and PCT application PCT/US2015/041037, titled "WIRELESS POSITIONING SYSTEMS," filed on July 17, 2015. Virtual checkpoints (VCs) can be deployed in areas of interest. The CSIs collected from the VCs are stored in a database. If the CSI collected in real-time is matched to some CSI in the database, based on the location information associated with the matched CSI in the database, one can know the location of the Bot. The VCs can help correct erroneous estimation of the real-time location of a Bot. Note that map/floor plan/path information of the area/path that the Bot will be travelling through can also help correct erroneous location estimation. For example, if one can know the Bot will make a 90 degree turn from the pre-defined path but the estimated trajectory of the Bot is that it makes a 120 degree turn, one can correct the Bot's moving trajectory to the actual path and avoid error accumulation.

### An Example Application of Object Tracking: Smart Elevator

One application of the object tracking is the elevator monitoring system. There is no satisfactory solution for monitoring the states of a running elevator. For example, it is difficult to know whether an elevator is running well, working properly, or is going to need maintenance immediately. Although a lot of elevators are equipped with monitoring cameras, the above problems are still unsolved because image/video processing of the images/videos taken in the elevator is very complicated and need large operating bandwidth. Using the object tracking system disclosed in the present teaching, one can monitor the working status of an elevator using a smart elevator system and support the following functionalities, including 1) fine-grained elevator positioning, 2) emergency detection, e.g., can detect the elevator stop due to malfunction, and 3) elevator door open/close detection.

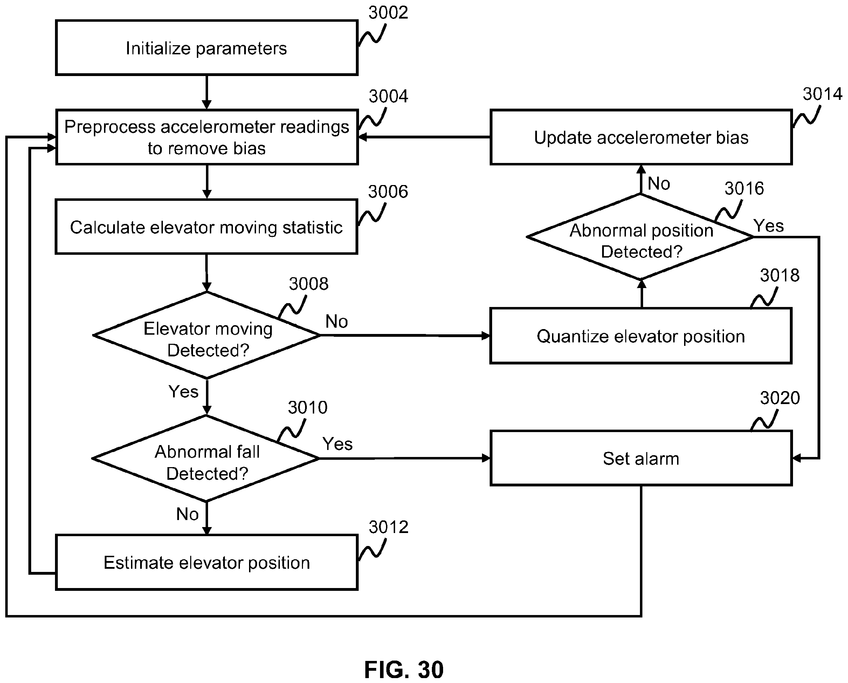

The main components of a smart elevator system include the following three parts. The first part of the smart elevator system is the elevator tracking module, which monitors the position of an elevator in real time by using an inertial measurement unit (IMU), or more specifically, an accelerometer. The algorithm for the tracking module is summarized in Algorithm 4 below and illustrated in FIG. 30. Some notations and their meanings are listed as follows.

| |
|---|
| *k:* time index |
| ***b***[*k*]: bias of accelerometer readings |
| ***b̅***[*k*]: initial bias estimation of accelerometer readings |
| *r*[*k*]: the estimated position of elevator |
| *t*[*k*]: time interval between two readings |
| *v*[*k*]: the estimated velocity of elevator |
| Δ*v*[*k*]: compensation of velocity |
| *h:* the height between adjacent floors |
| ***a**_{raw}*[*k*]: raw accelerometer reading |
| ***a***[*k*]: the accelerometer reading subtracted by the estimated bias |
| ***g***[*k*]: the gravity reading |
| *α*: forgetting factor for updating the bias estimation |
| *m*[*k*]: elevator moving statistic |
| Δ*k:* window length for computing *m*[*k*] |
| *η*₁, *η*₂, *η*₃, and *η*₄ are the preset thresholds |
| *ε:* the tolerable error ratio w.r.t. *h* |

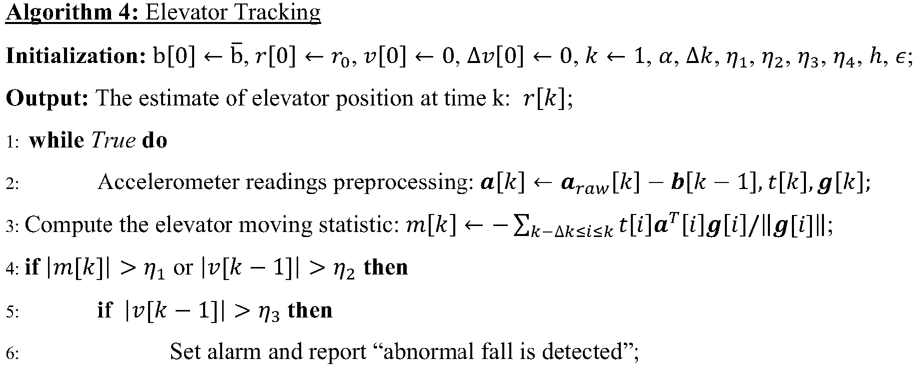

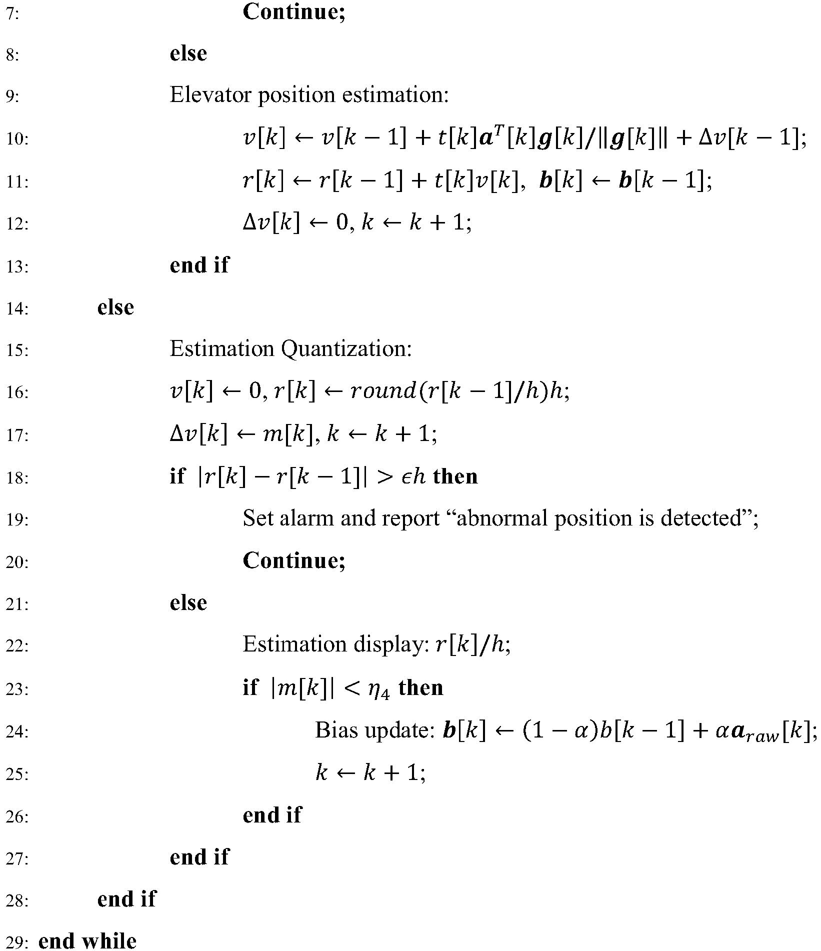

First, since accelerometer has a bias, before running the elevator tracking algorithm, one may need to have an initial bias estimation **b̅** by averaging the readings of the accelerometer for a period, e.g., 10 seconds (3002). Then, one can subtract the estimated bias from the new raw readings of the accelerometer and get an approximate estimation of the acceleration of the elevator at the current time k (3004). One can define the elevator moving statistic as *the change of speed due to acceleration* within a period (3006), which is an important metric in the determination of the motion status of an elevator. Since only the speed of the elevator along the vertical direction plays a role in the estimation of the position of an elevator, the elevator moving statistic at time slot k is computed as $m \left[k\right] = - {\sum }_{k - Δ k \leq i \leq k} t \left[i\right] a {\left[i\right]}^{T} g \left[i\right] / \left‖g\left[i\right]\right‖ ,$ (17) where Δ*k* stands for the length of the time window to compute the moving statistic, *t[i]* is the time difference between the i-th sample and (i-1)-th sample of the readings, ***a***[*i*] is the accelerometer reading after the subtraction of the estimated bias, ***g***[*i*] is the measurement of gravity obtained from accelerometer, and ∥***g***[*i*]∥ stands for the 2-norm of the gravity. A minus sign is also added to *m[k]* due to the reason that one can set the upward direction as the positive direction, which is the opposite direction of the gravity.

Since *m[k]* represents the change of the elevator speed in vertical direction, one can derive the elevator speed in vertical direction. Then one can determine if the elevator is moving or not by examining the magnitude of *m[k]* and *v*[*k*-1] (3008), which is the estimated speed of the elevator in the previous time slot k-1. If either of them is above the preset thresholds, then the algorithm sets the status of the elevator as moving. The algorithm also tracks the estimated speed of the elevator. If the estimated speed is too fast, then the algorithm would set an alarm (3020) to inform users that the elevator may experience an abnormal fall (3010). Otherwise, the estimated speed is updated by integrating the acceleration of the elevator. The moving distance is updated by integrating the estimated speed, and the new estimated position is updated by adding the moving distance to the previous estimated position (3012). At this stage, the bias estimation is not updated. Since the elevator moving statistic would incur a delay of moving detection, the algorithm compensates the speed estimation by adding Δ*v*[*k* - 1], which measures the loss of the speed due to the delay of moving detection.

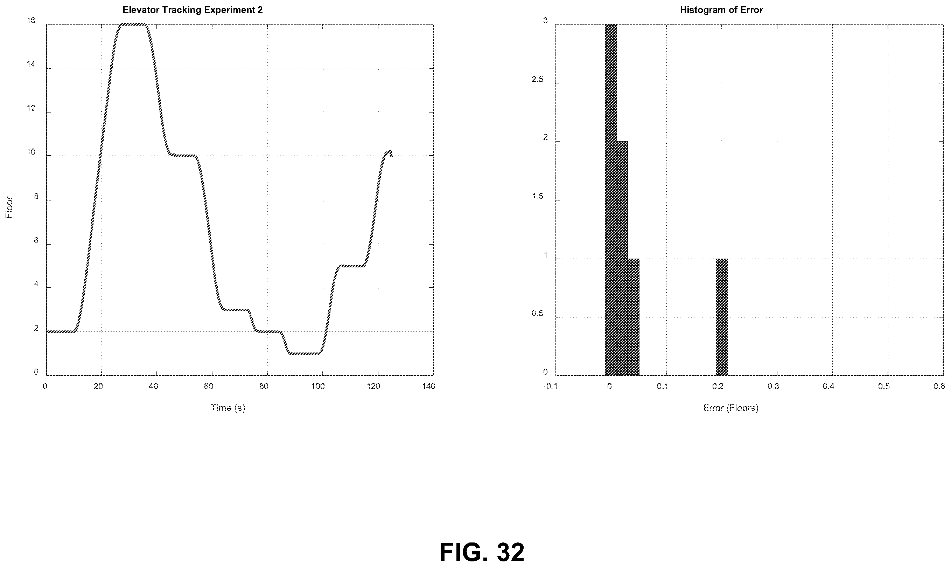

When the system detects that the elevator is not moving, it can set the current estimated speed to be 0, correct the position estimation by rounding off it to the nearest height of the floors as long as the quantization error is within certain range, and update Δ*v*[*k* - 1] by setting it to be the moving statistic (3018). Then, according to the algorithm, the system can check the rounding off error (3016). If the error is larger than a preset threshold, then the elevator may stop at an abnormal position, e.g., somewhere in between two adjacent floors. If the error is tolerable, then the system would check the moving statistic again and updates the estimation of bias when the amplitude of *m*[*k*] is small enough (3014). One can conduct experiments to verify the proposed algorithm in a typical building with 16 floors. The experiment results are shown in FIG. 31 and FIG. 32. The error of the elevator tracking algorithm is within 0.2 height of floor.

The second part of the smart elevator system is the human motion detector module. The system is equipped with a wireless transmitter (TX) and a wireless receiver (RX). The transmitter keeps transmitting wireless signals to the receiver. One can utilize the channel state information (CSI) between them to detect if there are any human beings inside the elevator in real time. The algorithm for the motion detection module is summarized in Algorithm 5 below and illustrated in FIG. 33.

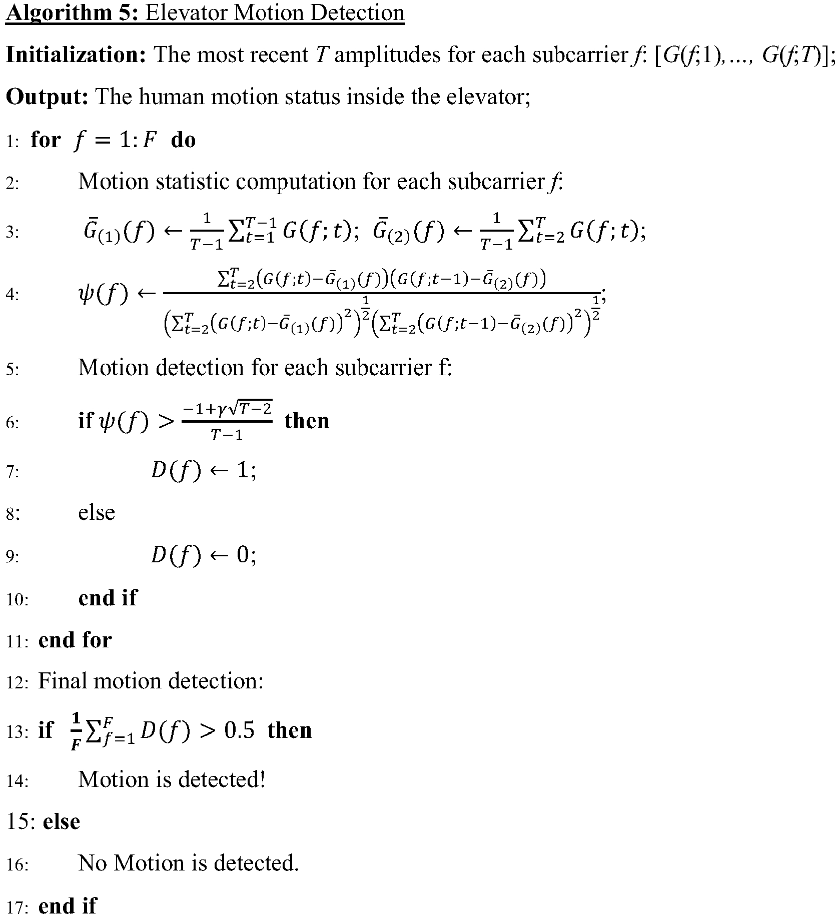

*G*(*f;t*) denotes the CSI amplitude of subcarrier *f* at time slot *t* (3302). For a system with *M* by *N* antenna configuration, the total number of subcarriers is *F=MNL,* where *L* is the number subcarrier for each antenna pair. For each subcarrier *f,* one can compute the *motion statistic as the first order of the sample auto-correlation coefficient* (3304), as shown in FIG. 33, where *T* is the length of the time window to compute the motion statistic. The physical meaning of the motion statistic is that the higher the motion statistic, the stronger the motion is. On each subcarrier, there is a motion statistic calculated for detecting a human motion, e.g., when its sample auto-correlation coefficient is larger than a predefined threshold (3306). A majority vote is applied to fuse all the decisions from *F* subcarriers of the system (3308). When more than half of the total number of the available subcarriers detect the human motion inside the elevator, then the system detects the motion (3010); otherwise, no motion is detected (3312). In another embodiment, *G(f*;*t)* can be defined as another function of the CSI on subcarrier *f* at time slot *t,* for example, (CSI amplitude)^2, (CSI amplitude)^4, real/imaginary part of the CSI after phase offset cleaning. The motion static can also be defined as the sample auto-correlation coefficient with another order, if the order is less than a quarter of the time window length T. Other decision fusion rule such as weighted combining of the individual decision can also be adopted.

The third part of the smart elevator system is the elevator door detector module. This module leverages the fact that the received signal strength indicators (RSSIs) on the receiver side differ under the elevator door open status and the elevator door closed status. Depending on the structure, material of the elevator and the mounting positions of the devices in the elevator, the impact of elevator door being open and closed on the RSSI changes also differ from case to case. Thus, the elevator door detector module requires a training process after the devices are mounted into the elevator. Values other than the RSSI that reflect the received signal power can also be adopted, for example, received channel power indicator (RCPI), reference signal received power (RSRP), reference signal received quality (RSRQ), signal-to-noise ratio (SNR), and signal-to-interference-and-noise ratio (SINR).

The algorithm for the training procedure is illustrated in FIG. 34. The elevator door detector module collects RSSI measurements from wireless devices (3402), e.g., commercial Wi-Fi devices with multiple receive antennas over several 20 MHz Wi-Fi channels. For example, with a Wi-Fi device equipped with three receive antennas running on 40 MHz bandwidth (or two 20 MHz Wi-Fi channels), one RSSI measurement includes seven values: one RSSI value for each receive antenna and each 20 MHz Wi-Fi channel that leads to six RSSI values in total, and one RSSI value as the summation of the six RSSI values. Here, denote R = [r_{1,1} r_{2,1} r_{3,1} r_{1,2} r_{2,2} r_{3,2} rₛᵤₘ] as the RSSI measurement matrix, where r_{i,j} stands for the RSSI measurements obtained from receive antenna i and Wi-Fi channel j over T time instances, given by r_{i,j} *=* [r_{i,j}[0], r_{i,j} [1], ..., r_{i,j} [T - 1]].

Considering the instability of the Wi-Fi devices, abnormal RSSI values can be observed in practice, which should be removed before further processing. To overcome the impact of the RSSI outliers, median filtering (3404) is performed for each of the r_{i,j} vector in the RSSI measurement matrix R, and one can denote the resultant RSSI measurement matrix as R_{med}.

To further reduce the high-frequency fluctuations in the RSSI measurements caused by noise, a low-pass filtering (3406) is performed on each of the median filtered RSSI vector r_{i,j}, denoted as r_{i,j,med} of the matrix R_{med}, leading to the RSSI measurement matrix Rₗₚ with RSSI vector given by r_{i,j,lp}.

When the elevator door is kept open or closed, the RSSI measurements would stay around a constant level. On the other hand, abrupt changes in the RSSIs can be observed at the time instance when the elevator door is opening or closing. This implies that the slope of the RSSI values over time would be close to zero when the door is kept open or closed, while the slope of the RSSI values would change greatly when the elevator door is opening or closing. Therefore, the elevator door detector module divides the duration of Rₗₚ into multiple overlapping time windows, and evaluates the slopes (3408) for each vector of Rₗₚ respectively.

For instance, given N measurements for the receive antenna i and Wi-Fi channel j, denoted by ${r}_{i , j , lp}^{′} = \left[{r}_{i , j , lp}^{′}\left[0\right],{r}_{i , j , lp}^{′}\left[1\right],{r}_{i , j , lp}^{′}\left[2\right],…{r}_{i , j , lp}^{′}\left[N-1\right]\right]$, the slope s during the current time window can be estimated using the least-square estimation, denoted as $s = \frac{\left({r}_{i , j , lp}^{′}-\overline{{r}_{i , j , lp}^{′}}\right) {\left(n-\overline{n}\right)}^{T}}{\left(n-\overline{n}\right) {\left(n-\overline{n}\right)}^{T}} ,$ (18) where n = [0,1,2, ..., N - 1] is the time index vector. The slope estimations for each receive antenna and Wi-Fi channel over all time windows are assembled into a slope measurement matrix S consisting of vectors S_{i,j}.

In general, the elevator door opening operation is much more predictable than the elevator door closing operation. For example, people could stop the elevator door from closing by blocking the elevator door, while the opening of the elevator door is mostly uninterruptable. Since the RSSI values would drop when the elevator door opens, a valley can be observed in the slope estimations at the time when the elevator door is opening. Therefore, to use conventional peak detection (3410), the module produce S'=-S as the negative of the actual slope estimation matrix S, which makes the valleys to be detected as peaks.

Peak detection algorithm (3410) is performed on each vector ${S}_{i , j}^{′}$ in the slope measurement matrix S' with criteria such as peak prominence, peak width, and peak persistence. Given that p peaks are detected for ${S}_{i , j}^{′}$, the module selects the peak with the largest prominence from the p peaks. Assume that the peak location is n, the module selects a segment of several seconds on the left of the peak location n in the vector r_{i,j,lp} and evaluates the average RSSI inside that segment as RSSI_{i,j,c}, which is the average RSSI value when the elevator door is closed. Meanwhile, the module selects another segment of several seconds on the right of the peak location n in the vector r_{i,j,lp} and evaluates the average RSSI inside the segment as RSSI_{i,j,o}, which is the average RSSI value when the elevator door is opened. The difference between them is also calculated as RSSI_{i,j,d}. After calculating the RSSI_{i,j,d} values for all receiving antennas i and Wi-Fi channel j, the module picks the (i, j) combination that leads to the largest RSSI_{i,j,d}, i.e., the largest margin between the door opened and door closed states.

Assume that (iₘₐₓ,jₘₐₓ) leads to the largest RSSI_{i,j,d}, the RSSI threshold (3412) is then determined as RSSIₜₕ = (1 - α) RSSI_{iₘₐₓ,jₘₐₓ,c}+αRSSI_{iₘₐₓ,jₘₐₓ,o}, where 0 < α < 1. Notice that, if RSSI_{iₘₐₓ,jₘₐₓ,c} < RSSI_{iₘₐₓ,jₘₐₓ,o}, which means that the RSSI associated with the elevator door closed state is lower than that with the elevator door opened state, the module declares that the training is failed, and a re-training is needed.

The elevator door detector module requires at least one elevator door opening in the duration of the training.

After the training phase, the elevator door detector module can perform real-time door monitoring. The algorithm is shown in FIG. 35. For each incoming RSSI measurement (3502), the module picks the RSSI value from the iₘₐₓ receiving antenna and the iₘₐₓ Wi-Fi channel. Then, it performs median filtering 3504 and low-pass filtering 3506 to mitigate the outliers and high-frequency noise in the RSSI measurements. Assume that the RSSI value after filtering is ${r}_{i , j , lp}^{′} \left[n\right]$ for the n - th RSSI measurement. The module compares ${r}_{i , j , lp}^{′} \left[n\right]$ with the threshold RSSIₜₕ obtained in the training phase (3508), determines the door closed (3514) if ${r}_{i , j , lp}^{′} \left[n\right] > {RSSI}_{th}$, and determines the door opened (3510) if ${r}_{i , j , lp}^{′} \left[n\right] \leq {RSSI}_{th}$.

In practice, the RSSI levels associated with the elevator door open and closed states might change over time, due to the slight changes in the elevator structure, temperature, or the hardware issues. Thus, the module keeps updating the RSSI threshold (3520) based on the slope estimation 3516 and peak detection 3518 as introduced in the training procedure. As long as a peak is detected, the module re-evaluates the RSSI threshold. If the RSSI threshold is valid, then, the module updates *RSSIₜₕ* (3520) and uses it for the next elevator door detection.

### How Much Bandwidth Is Needed

As mentioned earlier in the present teaching, the performance of a TR-based system relies on the ability of resolving the many multipaths naturally existing in the environment. A larger operating bandwidth leads to more resolvable multipaths due to a better time resolution.

However, the spectrum is still a scarce resource with its own cost. So one may need to determine how much bandwidth is needed in a TR-based system, and one metric is to optimize the spectral efficiency of the system. Consider an example of a Time-Reversal Division Multiple Access (TRDMA) system with multiple antennas and varying bandwidths, as disclosed in U.S. Patent No. 13/969,271, filed August 16, 2013, entitled "Time-Reversal Wireless System Having Asymmetric Architecture". The optimal bandwidth for the system is defined as the bandwidth required to achieve the maximum spectral efficiency given the number of users N and backoff factor D. One can first establish an equivalent multi-tap channel model for the system with varying bandwidths based on the real channel measurement in a typical indoor environment. By evaluating the spectral efficiency of the TRDMA system with varying bandwidths and different signature types (e.g., Basic TR signature and Zero-Forcing (ZF) signature), one can find that the optimal bandwidth for TR communication is determined by the number of users N and backoff factor D instead of the signature types. More specifically, the optimal bandwidth for the system increases with D when D is small, while increases with N when D is large.

Even though the optimal bandwidth for a TR system can be obtained through examining the spectral efficiency, a sub-optimal bandwidth can be derived based on the rank condition of the channel matrix, which is much easier to acquire than evaluating the spectral efficiency. Simulation results validate t theoretical analysis and show that the sub-optimal bandwidth is very close to the optimal one when D is small.

The uplink transmission of a typical time-reversal division multiple access system with multiple antennas (TRDMA-MA system) is shown in Fig.36, where N terminal devices (TDs) simultaneously transmit signal to the access point (AP) equipped with M antennas. The emitted signal propagates through the multipath channels ${h}_{i}^{\left(m\right)}$'s and arrives at the AP, where ${h}_{i}^{\left(m\right)}$ represents the multipath channel between the i^{th} TD and the m^{th} antenna at the AP.

To handle the inter-symbol-interference (ISI) due to the multipath channel profile, a backoff factor D is adopted in the system. Regarding the inter-user-interference (IUI) suppression, ${g}_{i}^{\left(m\right)}$'s are the equalizers designed based on the multipath channels ${h}_{i}^{\left(m\right)}$'s as shown in in FIG. 36.

The performance of TR communications inherently depends on the number of resolved independent taps in the channel impulse response (CIR), which is utilized to enable the multiple access of the TDs. Instead of deploying the massive number of antennas like massive MIMO system, TR technique tries to harvest the naturally existing multipaths in the environment with a large bandwidth. In the following, one can first illustrate the relationship between the number of resolved independent multipaths and the system bandwidth. Then, a channel model with varying bandwidths is established for later theoretical analysis in the present teaching.

Suppose there are totally *Kₘₐₓ* independent multipaths from the *i^{th}* TD to the *m^{th}* antenna at the AP, then the channel ${h}_{i}^{\left(m\right)} \left(t\right)$ can be written as ${h}_{i}^{\left(m\right)} \left(t\right) = {\sum }_{k = 1}^{{K}_{max}} {h}_{h , k}^{\left(m\right)} δ \left(t-{τ}_{k}\right) ,$ (19) where ${h}_{i , k}^{\left(m\right)}$ and *τₖ* are the complex channel gain and path delay of the *k^{th}* path, respectively. Without loss of generality, one can assume that *τ*₁ = 0, and as a result, the delay spread of channel is given by *τ_{C} = τ_{Kₘₐₓ}.*

Constrained by the limited bandwidth *W* of practical communication systems, pulse shaping filters are typically used to limit the effective bandwidth of transmission. Generally, the duration of the pulse *Tₚ* is limited by the available bandwidth *W* through the relation *Tₚ* = 1/*W*. Therefore, the equivalent channel response for a system with a limited bandwidth W can be expressed as ${{h}^{˜}}_{i}^{\left(m\right)} \left(t\right) = {\int }_{t - {T}_{p}}^{t} p \left(t-τ\right) {h}_{i}^{\left(m\right)} \left(τ\right) dτ , 0 \leq t \leq {τ}_{C} + {T}_{p}$ (20) From (20), one can see that for those paths whose time differences are less than *Tₚ*, they are mixed together due to the limited bandwidth *W*. In other words, these paths are treated like one path in the equivalent CIR in the system.

According to the analysis, one can consider a channel model as follows ${h}_{i , l}^{\left(m\right)} ∼ CN \left(0, \frac{{e}^{- \frac{α}{L} l}}{{\sum }_{l = 1}^{L} {e}^{- \frac{α}{L} l}}\right) ,$ (21) where *L* is the number of resolved independent taps given the bandwidth W and α is a constant determined by the environment. Note that *L* is determined by the bandwidth through *L =* **f**(*W*) and **f** is a one-to-one mapping given a certain range of *W*, which can be curve fitted by the experiment. The function **f** will be studied with real experiments later. From (21), it is observed that the overall expect channel gain $E \left[{h}_{i}^{\left(m\right)}\right]$ remains constant for the varying *W* and thus *L.* Moreover, the larger the *L,* the smaller the decay for two taps in (21) due to the better time resolution.

For ease of notation, one can find the optimal *L** in the following analysis and the corresponding optimal *W** can be obtained through the inverse mapping of**f**.

Prior to the data transmission, *N* TDs first take turn to transmit an impulse signal, which in practice may be a modified raised-cosine signal depending on the system bandwidth.

The AP estimates the channel response ${h}_{i}^{\left(m\right)}$ of each antenna for the *i^{th}* TD, and one can assume the perfect channel estimation.

Upon acquiring all the ${h}_{i}^{\left(m\right)}$'s of each link, different designed equalizers ${g}_{i}^{\left(m\right)}$'s (e.g. basic TR signature and ZF signature ) can be deployed at the AP side. According to the asymmetric system architecture, these signature waveforms serve as the equalizers in the uplink transmission phase as shown in FIG. 36. $\begin{matrix}{H}_{m , i} = \\ \left(\begin{matrix}{h}_{i}^{\left(m\right)} \left[D\right] & {h}_{i}^{\left(m\right)} \left[D-1\right] & ⋯ & {h}_{i}^{\left(m\right)} \left[1\right] & 0 & ⋯ & ⋯ & 0 \\ {h}_{i}^{\left(m\right)} \left[2D\right] & {h}_{i}^{\left(m\right)} \left[2D-1\right] & ⋯ & ⋯ & {h}_{i}^{\left(m\right)} \left[1\right] & 0 & ⋯ & 0 \\ ⋮ & ⋮ & ⋱ & ⋱ & ⋱ & ⋱ & ⋱ & ⋮ \\ {h}_{i}^{\left(m\right)} \left[L\right] & {h}_{i}^{\left(m\right)} \left[L-1\right] & ⋯ & ⋯ & ⋯ & ⋯ & ⋯ & {h}_{i}^{\left(m\right)} \left[1\right] \\ ⋮ & ⋮ & ⋱ & ⋱ & ⋱ & ⋱ & ⋱ & ⋮ \\ 0 & ⋯ & 0 & {h}_{i}^{\left(m\right)} \left[L\right] & ⋯ & ⋯ & {h}_{i}^{\left(m\right)} \left[L-D+1\right] & {h}_{i}^{\left(m\right)} \left[L-2D\right] \\ 0 & ⋯ & ⋯ & 0 & {h}_{i}^{\left(m\right)} \left[L\right] & ⋯ & {h}_{i}^{\left(m\right)} \left[L-D+1\right] & {h}_{i}^{\left(m\right)} \left[L-D\right]\end{matrix}\right) ,\end{matrix}$ (22)

Denote {**X***ᵢ*} as the sequence of information symbols at the *i^{th}* TD to be transmitted to the AP. To suppress the ISI as well as match the symbol rate with chip rate, a backoff factor D is introduced by inserting (D - 1) zeros between two symbols ,i.e., ${X}^{\left[D\right]} \left[k\right] = \left(\begin{matrix}X \left[k/D\right] , & if \left(k mod D\right) = 0 \\ 0 , & if \left(k mod D\right) \neq 0\end{matrix},\right.$ (23) where (·)^{[*D*]} denotes the *D*-times upsampling. The up-sampled information symbols of the *N* TDs are transmitted out through the multipath channel and are added together at the AP. For instance, the signal received at the *m^{th}* antenna of the AP is represented as follows ${S}_{m} \left[k\right] = {\sum }_{i = 1}^{N} \left({X}_{i}^{\left[D\right]}∗{h}_{i}^{\left(m\right)}\right) \left[k\right] + {n}_{m} \left[k\right]$ (24) where **n***ₘ* is an additive Gaussian noise at the *m^{th}* antenna.

The equalized symbols for the *i^{th}* TD are combined over the M antennas as ${Y}_{i}^{\left[D\right]} \left[k\right] = {\sum }_{m = 1}^{M} \left({\sum }_{i = 1}^{N} \left({X}_{i}^{\left[D\right]}∗{h}_{i}^{\left(m\right)}\right) ∗ {g}_{i}^{\left(m\right)}\right) \left[k\right] + {{n}^{˜}}_{i} \left[k\right] ,$ (25) where {*ñᵢ*[*k*]} is the equivalent AWGN with zero mean and variance *σ*².

Finally, ${Y}_{i}^{\left[D\right]}$ is downsampled with the same factor D, ending up with **Y***ᵢ* given as follows ${Y}_{i} \left[k\right] = {\sum }_{m = 1}^{M} {\sum }_{j = 1}^{N} {\sum }_{l = 1}^{\left(2L-1\right) / D} \left({h}_{j}^{\left(m\right)}∗{g}_{i}^{\left(m\right)}\right) \left[Dl\right] {X}_{i} \left[k-l\right] + {n}_{i} \left[k\right] ,$ (26) where *nᵢ*[*k*] *= n̂ᵢ*[*Dk*]. The information symbols **X***ᵢ* for the *i^{th}* TD is estimated through **Y***ᵢ*.

By replacing the convolution with inner product, (26) can be rewritten as follows $\begin{matrix}{Y}_{i} \left[k\right] & = {\sum }_{m = 1}^{M} \left({H}_{m , i}^{\left(\frac{L}{D}\right)}{g}_{m , i}\right) {X}_{i} \left[k-\frac{L}{D}\right] \left(Signal\right) \\ & + {\sum }_{m = 1}^{M} \left({\sum }_{l = 1 , l \neq L / D}^{\left(2L-1\right) / D} {H}_{m , i}^{\left(l\right)} {g}_{m , i}\right) {X}_{i} \left[k-l\right] \left(ISI\right) \\ & + {\sum }_{m = 1}^{M} {\sum }_{j = 1 , j \neq i}^{N} \left({\sum }_{l = 1}^{\left(2L-1\right) / D} {H}_{m , j}^{\left(l\right)} {g}_{m , i}\right) {X}_{j} \left[k-l\right] \left(IUI\right) \\ & + {n}_{i} \left[k\right] \left(Noise\right)\end{matrix} ,$ (27) where ${H}_{m , i}^{\left(l\right)}$ is the *l^{th}* row of the (2L - 1)/*D* × *L* matrix *H_{m,i}* that is decimated by rows of a Toeplitz matrix as shown in (22).

Consequently, the effective SINR of the *i^{th}* TD can be derived as shown in (28), where $ρ = E \left[{\left|{X}_{i}\left[k\right]\right|}^{2}\right] / {σ}^{2}$ is the signal-to-noise ratio (SNR). ${SINR}_{i} = \frac{ρ E \left[{\left|{\sum }_{m = 1}^{M} \left({H}_{m , i}^{\left(\frac{L}{D}\right)}{g}_{m , i}\right)\right|}^{2}\right]}{ρ E \left[{\sum }_{l = 1 , l \neq L / D}^{\left(2L-1\right) / D} {\left|{\sum }_{m = 1}^{M} \left({H}_{m , i}^{\left(l\right)}{g}_{m , i}\right)\right|}^{2}\right] + ρ E \left[{{\sum }_{j = 1 , j \neq i}^{N} {\sum }_{l = 1}^{\left(2L-1\right) / D} \left|{\sum }_{m = 1}^{M} {H}_{m , j}^{\left(l\right)} {g}_{m , i}\right|}^{2}\right] + 1} .$ (28) Based on (28), the effective SINR of the *i^{th}* TD depends not only on *N and D* but also on L, which is closely related to the system bandwidth.

Based on the channel model as shown in (21), there exists a one-to-one mapping between the bandwidth W and the number of channel taps L. Therefore, the bandwidth plays a significant role in determining the individual spectral efficiency of the TRDMA-MA system as shown in (28).

The spectral efficiency of the *i^{th}* TD in the TRDMA-MA system is defined as ${R}_{i} ≜ \frac{1}{D} log \left(1+{SINR}_{i}\right) ,$ (29) which is an increasing function of *SINRᵢ* given a fixed *D.* Given N and *D,* the optimal *L** that maximizes the spectral efficiency is written as $L * ≜ {{argmaxR}_{i}}_{L} ,$ (30) where *N* TDs are assumed uniformly distributed and thus share the same spectral efficiency. After that, the optimal bandwidth W* can be obtained as $W * = {f}^{- 1} \left(L*\right) ,$ (31) where f is the function that maps the system bandwidth W to the number of resolved independent taps L. The function **f** can be derived with curve fitting on the experiment data, e.g. in FIG. 3, which may vary with different indoor environments.

Even though various signature types can be deployed in TRDMA-MA system that result in different values of spectral efficiency, the signature design method should not affect the *L* and thus the optimal bandwidth *W** for the TRDMA-MA system due to the same number of degree of freedom. Moreover, since there exists a one-to-one mapping between the bandwidth *W* and *L,* one can try to find the optimal *L** as follows.

As an example, one can explore the *L** for TRMDA-MA system with basic TR signature and ZF signatures.

Upon acquiring the CIR between the *i^{th}* TD and the *m^{th}* antenna, the basic TR signature can be obtained as the normalized (by the average channel gain to *M* antennas) complex conjugate of time-reversed CIR, ${g}_{i}^{\left(m\right)} = \frac{{H}_{m , i}^{{\left(\frac{L}{D}\right)}^{†}}}{\sqrt{M}} ,$ (32) where ${H}_{m , i}^{\left(\frac{L}{D}\right)}$ is the time reversed channel and based on (22), ${H}_{m , i}^{\left(\frac{L}{D}\right)} = \left[{h}_{i}^{\left(m\right)}\left[L\right],{h}_{i}^{\left(m\right)}\left[L-1\right],⋯{h}_{i}^{\left(m\right)}\left[1\right]\right] .$ (33)

Based on (32), the expected power of signal, ISI and IUI terms in (28) can be written as follows, $\begin{matrix}{P}_{sig} = \frac{ρ}{M} E \left[{\left({\sum }_{m = 1}^{M} {\left‖{H}_{m , i}^{\left(\frac{L}{D}\right)}\right‖}^{2}\right)}^{2}\right] \\ {P}_{is i} = \frac{ρ}{M} E \left[{\sum }_{l = 1 , l \neq L / D}^{\left(2L-1\right) / D} {\left|{\sum }_{m = 1}^{M} \left({H}_{m , i}^{\left(l\right)}{H}_{m , i}^{\left(\frac{L}{D}\right) †}\right)\right|}^{2}\right] \\ {P}_{iui} = \frac{ρ}{M} E \left[{\sum }_{j = 1 , j \neq i}^{N} {\sum }_{l = 1}^{\left(2L-1\right) / D} {\left|{\sum }_{m = 1}^{M} {H}_{m , j}^{\left(l\right)} {H}_{m , i}^{\left(\frac{L}{D}\right) †}\right|}^{2}\right]\end{matrix} .$ (34)

Assume that the taps of ${h}_{i}^{\left(m\right)}$ and the CIRs of different links are mutually independent. Then, according to the channel model in (21), one can have $\begin{matrix}{P}_{sig} = ρ \left({\sum }_{l = 1}^{L} \frac{{e}^{- \frac{2 α}{L} l}}{{c}^{2}} + M\right) \\ {P}_{isi} = ρ {\sum }_{l = 1 , l \neq L / D}^{\left(2L-1\right) / D} {β}_{l} \\ {P}_{iui} = ρ \left(N-1\right) {\sum }_{l = 1}^{\left(2L-1\right) / D} {β}_{l} ,\end{matrix}$ (35) where $\begin{matrix}c = {\sum }_{l = 1}^{L} {e}^{- \frac{α}{L} l} \\ {β}_{l} = {\sum }_{k = 1}^{Dl} \frac{{e}^{- \frac{α}{L} \left(L-Dl+2k\right)}}{{c}^{2}} .\end{matrix}$

It can be observed that *P_{sig}* increases with the number of transmit antennas M given the fixed number of users N and backoff factor *D.* However, *P_{sig}* decreases with *L,* since the power of each tap becomes much smaller even though the number of terms is larger. Regarding the other terms, *Pᵢₛᵢ* increases with *L* while *Pᵢᵤᵢ* decreases with *L.* Therefore, the main benefit of using a larger bandwidth is to suppress the IUI through resolving a larger number of multipath. On the other hand, a larger L leads to a smaller signal power and a larger ISI. Based on these observations, the spectral efficiency increases with L if the decrease in the IUI dominates the side effect of a larger *L* on both *P_{sig}* and *Pᵢₛᵢ*. Therefore, there would exist an optimal *L** and thus *W** that can achieve the maximum spectral efficiency.

Different from the basic TR signature that is designed based on CIR of each individual TD, ZF signature is designed according to the CIRs of all TDs, i.e., $\begin{matrix}{g}_{i}^{\left(m\right)} = \\ \left(\begin{matrix}{c}_{zf} {Q}_{m}^{†} {\left({Q}_{m}{Q}_{m}^{†}\right)}^{- 1} {e}_{{l}_{i}} , & if {Q}_{m} is full row rank , \\ {c}_{zf} {\left({Q}_{m}^{†}{Q}_{m}\right)}^{- 1} {Q}_{m}^{†} {e}_{{l}_{i}} , & if {Q}_{m} is full column rank ,\end{matrix}\right.\end{matrix}$ (36) where *c_{zf}* is the normalization factor to make the signature unitary power, and **Q***ₘ* is the combined channel matrix of the *N* TDs to the *m^{th}* antenna, i.e., ${Q}_{m} = {\left[{H}_{m , 1}^{†} {H}_{m , 2}^{†} ⋯{H}_{m , N}^{†}\right]}^{†} ,$ (37) and e*_{lᵢ}* is an elementary vector with ${l}_{i} = \left(i-1\right) \left(2\left⌊\frac{L - 1}{D}\right⌋+1\right) + \left⌊\frac{L - 1}{D}\right⌋ + 1 .$ (38)

With the definition of **Q***ₘ* and e*_{lᵢ}* above, one can have ${Q}_{m}^{†} {e}_{{l}_{i}} = {H}_{m , i}^{\left(\frac{L}{D}\right) †} .$ (39)

One may first consider the case of **Q***ₘ* being full-column rank. Under this scenario, the expected power of the intended signal, ISI and IUI in (28) can be derived based on (36) and (39) as follows, $\begin{matrix}{P}_{sig} = {ρc}_{z f}^{2} E \left[{\left|{\sum }_{m = 1}^{M} \left({H}_{m , i}^{\left(\frac{L}{D}\right)}{\left({Q}_{m}^{†}{Q}_{m}\right)}^{- 1}{H}_{m , i}^{\left(\frac{L}{D}\right) †}\right)\right|}^{2}\right] \\ {P}_{isi} = {ρc}_{zf}^{2} E \left[{\sum }_{l = 1 , l \neq L / D}^{\left(2L-1\right) / D} {\left|{\sum }_{m = 1}^{M} \left({H}_{m , i}^{\left(l\right)}{\left({Q}_{m}^{†}{Q}_{m}\right)}^{- 1}{H}_{m , i}^{\left(\frac{L}{D}\right) †}\right)\right|}^{2}\right] \\ {P}_{iui} = {ρc}_{zf}^{2} E \left[{\sum }_{j = 1 j \neq i}^{N} {\sum }_{l = 1}^{\frac{2 L - 1}{D}} {\left|{\sum }_{m = 1}^{M} {H}_{m , j}^{\left(l\right)} {\left({Q}_{m}^{†}{Q}_{m}\right)}^{- 1} {H}_{m , i}^{\left(\frac{L}{D}\right) †}\right|}^{2}\right] .\end{matrix}$ (34) From (40), it can be seen that the interference *Pᵢₛᵢ* and *Pᵢᵤᵢ* cannot be completely canceled when **Q***ₘ* is full column rank. Based on the numerical simulations results shown later, the interference will decrease as **Q***ₘ* tends to be full row rank.

Once **Q***ₘ* becomes full row rank, according to (36), all the interference can be removed. More specifically, ${Q}_{m} {g}_{i}^{\left(m\right)} = {c}_{zf} {Q}_{m} {Q}_{m}^{†} {\left({Q}_{m}{Q}_{m}^{†}\right)}^{- 1} {e}_{{l}_{i}} = {c}_{zf} {e}_{{l}_{i}} .$ (41) In other words, $\left(\begin{matrix}{H}_{m , i}^{\left(l\right)} {g}_{i}^{\left(m\right)} = 0 , & for ∀ i , m and l \neq L / D \\ {H}_{m , j}^{\left(l\right)} {g}_{i}^{\left(m\right)} = 0 , & for ∀ l , m and i \neq j .\end{matrix}\right.$ (42) Therefore, based on (42), one can determine that *Pᵢₛᵢ* = 0 and *Pᵢᵤᵢ* = 0. The signal power becomes ${P}_{sig} = {ρc}_{zf}^{2} {M}^{2} ,$ (43) where ${C}_{zf}^{2} = \frac{1}{M E {\left[{\left({Q}_{m}{Q}_{m}^{†}\right)}^{- 1}\right]}_{{l}_{i} , {l}_{i}}} .$ (44)

From the numerical simulation, (43) first increases with *L* and later saturates. Therefore, the spectral efficiency first increases with *L* and then saturates under the full row rank scenario.

From the above analysis, the optimal *L** is closely related to the rank condition of **Q***ₘ*. To suppress the ISI and IUI, *L** should be close to the *L* that makes **Q***ₘ* either full row rank or most likely to full row rank. This observation can motivate one to find a sub-optimal *L̅*, solely based on the rank condition of **Q***ₘ*, as an approximation of *L**. In the following, one can analyze the sufficient condition of *L* to make **Q***ₘ* full row rank.

As defined in (37), **Q***ₘ* is an $N \left(2\left⌊\frac{L - 1}{D}\right⌋+1\right) \times L$ matrix. Since the taps in each CIR and the CIRs of different TDs are mutually independent, it is reasonable to assume that the rows of **Q***ₘ* are independent. Generally, **Q***ₘ* will be full row rank when $N \left(2\left⌊\frac{L - 1}{D}\right⌋+1\right) \leq L$. Based on the fact that $\left⌊x\right⌋ \leq x$ where *x* is a positive number, one can derive one sufficient condition on *L* to make **Q***ₘ* full row rank, i.e., given *N* and *D,* a sufficient condition on *L* to make **Q***ₘ* full row rank is $\left(\begin{matrix}L & \leq \frac{2 N - DN}{2 N - D} , & for & D & \leq & 2 N , \\ L & \geq \frac{DN - 2 N}{D - 2 N} . & for & D & \geq & 2 N .\end{matrix}\right.$ (45)

One can observe that in order to make **Q***ₘ* full row rank, *L* has an upper bound when *D* is small and a lower bound when *D* is large. Since the interference will be completely canceled when **Q***ₘ* is full row rank, one can propose a sub-optimal *L̅* as the approximation to *L** based on the rank condition, i.e., the optimal *L** can be approximated by *L̅* which satisfies $\overline{L} ≜ min \left(arg{min}_{L}\left(2N\left⌊\frac{L - 1}{D}\right⌋+N-L\right)\right) .$ (46)

According to (46), the sub-optimal *L̅* only depends on the system parameters such as the number of users *N* and the backoff factor *D,* which makes it much easier to obtain than evaluating the spectral efficiency to derive the optimal *L*.* Once the optimal *L** or the sub-optimal *L̅* is derived, the corresponding bandwidth for the system can be obtained according to (31). An example of deriving (31) is shown in the following simulation.

In the simulation, one can first conduct experiments in an indoor environment to validate the relationship between the number of resolvable independent multipaths *L* and the system bandwidth *W*. Then, simulations are conducted to evaluate the optimal *L** and thus the optimal bandwidth of the TRDMA-MA system with both basic TR and ZF signatures.

One may use two universal software radio peripherals (USRPs) as channel sounders to probe the channel in an office, where the TX was located on a channel probing table with 5cm resolution and the RX was placed in the hallway as shown in FIG. 5. One can scan the spectrum in 4.9-5.9GHz with frequency hopping to acquire the CIRs of 10MHz-1GHz bandwidth using transmission power of 100mW.

Based on the measured data, the eigenvalue analysis is utilized to determine the number of resolved independent multipaths for any given bandwidth W. First, one can estimate the covariance matrix of the measured channels *K*_{*h*,*W*} using statistical averaging ${K}_{h , W} = \frac{1}{N} {\sum }_{i = 1}^{N} {h}_{i , W} {h}_{i , W}^{†} ,$ (47) where *h*_{*i*,*W*} is the channel information obtained at location *i* with bandwidth W and *N* = 100. Since *K_{h,W}* is Hermitian and positive definite, there exists a unitary matrix *U* such that ${K}_{h , W} = U Λ {U}^{†} = {\sum }_{i = 1}^{\hat{L}} {λ}_{i , W} {ψ}_{i} {ψ}_{i}^{†} ,$ (48) where *λ*_{1,*W*} ≥ *λ*_{2,*W*} ≥ ··· ≥ *λ_{L̂}_{,W}* and *L̂ = τ_{C}W.*

The experiment results are summarized in FIG. 37. From FIG. 37, one can see that the channel energy is concentrated in a small number of eigenvalues when the bandwidth is small, while spread over a large number of eigenvalues as the bandwidth increases. One can also show in FIG. 38 that the number of significant multipaths in an indoor environment versus the system bandwidth. It can be seen that, with a single antenna, the number of multipaths can approach around 100 as the bandwidth increases to 1GHz. Such degrees of freedom can be further scaled up by deploying more antennas.

Based on FIG. 38, the function f, which maps *W to L,* can be obtained by curve fitting.

In the following, the system with basic TR signature is considered. From previous analysis, one can find that the optimal *L** is closely related to both *D* and *N.* Therefore, in the following, one can evaluate the effect of *D* and *N* separately on *L*.*

First, one can investigate the effect of *D* on *L** given the fixed *N =* 5. One can assume the SNR of system is 20dB. The spectral efficiency of one user is shown in FIG. 39 with *D* = 20 and FIG. 40 with *D* = 4. From these figures, one may see that M has no effect on *L** (i.e. the peak of spectral efficiency appears around the same *L**) even though the spectral efficiency increases with M. The curve of spectral efficiency looks quite distinguishable for large *D* and small *D.* More specifically, the spectral efficiency seems to have an upper bound when *D* is large, i.e., the spectral efficiency saturates after *L* is sufficiently large. On the other hand, there exists a unique *L** when *D* is small. The spectral efficiency decreases when *L > L*.*

Now one can study the more general case by selecting a series of *D.* Since M has no effect on *L*,* the number of antenna is fixed as *M* = 2. The spectral efficiency of individual user with basic TR signature is shown in FIG. 41. From the figure, one can first observe that the spectral efficiency decreases with *D*, which is due to the fact that the term 1/D dominates the improvement of SINR in (28). Then, one can find that the effect of *D* on *L** heavily depends on the value of *D.* On one hand, the *L** increases with *D* when *D* is small, e.g., *D* = 1 → 5. On the other hand, *L** seems independent from *D* when *D* is sufficiently large, e.g., *D* ≥ 20.

In the following, one can explore the effect of *N* on *L*.* As one can know, *L** is independent from *M* and determined by *D* when *D* is small. Therefore, one can consider the system with *M =* 2 with varying *N* for both *D* = 20 (FIG. 42) and *D* = 4 (FIG. 43) in the following simulation. From FIG. 42 and FIG. 43, it can be seen that the spectral efficiency decreases with *N* since the IUI increases with *N.* As shown in FIG. 42, the larger the *N,* the larger the *L** to achieve the maximum spectral efficiency when *D* is large. It is also validated that the *L** is independent of *N* when *D* is small, as shown in FIG. 43.

By summarizing the previous simulation results of the spectral efficiency with basic TR signature, one can observe that *L** is determined by *N* and *D* instead of M. Moreover, when *D* is small, *L** is independent of *N* but increases with *D.* On the other hand, when *D* is large, *L** increases with *N* but independent of *D.* Even though different signature design methods can achieve different spectral efficiencies, the *L** should be independent of the specified signature design methods. Therefore, the conclusion about *L** can also be applied for the ZF signature scenario, which is validated in the following.

As discussed before, one can find some general conclusion about the effect of *D* and *N* on *L** under the basic TR scenario. In the following, it can be demonstrated that the same conclusion is applicable to ZF signature scenario as well.

One can first investigate the effect of *D* on *L** given the fixed *N =* 5. Same as the previous, one can evaluate the spectral efficiency for both large *D* and small *D* with ZF signature. One can assume the SNR of the system is 20dB. The spectral efficiency with ZF signature is shown in FIG. 44 and FIG. 45, where *D* = 20 and *D* = 2, respectively. First, the spectral efficiency increases with M but *L** is independent from M. When *D* is large as shown in FIG. 44, *L* has a lower bound to achieve the maximum spectral efficiency. While *D* is small, there exists a unique *L** as shown in FIG. 45.

Then one can investigate the spectral efficiency under ZF signature with varying *D*. One can fix *M* = 2 and *N* = 5 in the simulation. According to FIG. 46, the *L** to achieve the maximum spectral efficiency increases with *D* given *D* is small. While *D* is sufficiently large, *L** tends to be independent of *D.*

One can explore the effect of *N* on *L** in the following. In the simulation, one can fixed M = 2 and SNR to be 20dB. The spectral efficiency is simulated with varying *N* for both *D* = 20 and *D* = 2 as shown in FIG. 48 and FIG. 49, respectively. From FIG. 48, it is shown that *L** increases with *N* when *D* is sufficiently large. However, *L** becomes independent of *N* when *D* is small as shown in FIG. 49.

From the simulation results for ZF signature, one can find the effect of *D* and *N* on *L** is exactly same as in the basic TR signature scenario. In other words, the *L** has been validated to be independent of the signature types. These findings can be summarized in the following equation, $L * = \left(\begin{matrix}f \left(D\right) , & when D is small \\ g \left(N\right) . & when D is large .\end{matrix}\right.$ (49) where both f and g are the increasing functions.

Even though one can find some useful conclusions about *L** in (49), the spectral efficiency needs to be evaluated to get the exact value of *L**, which is costly in terms of computation. Based on the previous discussion, one can use the rank evaluation as an alternative of computing the spectral efficiency, which provides a sub-optimal approximation of *L**. Although the rank evaluation is more intuitive for ZF signature, it is also applicable for other signature types according to the previous discussion.

Based on (46), the sub-optimal *L̅* only depends on *D* and *N,* which is plotted in FIG. 47. From the figure, it can be seen that the sub-optimal *L̅* is consistent with *L** in terms of (49). By comparing FIG. 47 with FIG. 41 and FIG. 46, *L̅* is quite accurate as an estimation of *L** when *D* is small. When *D* is large, *L̅* becomes a lower bound for *L** as shown in FIG. 42 and FIG. 48. For the system with ZF signature, the spectral efficiency continues to increase with *L* for a while after *L̅,* since the *c_{zf}* in (43) continues to increase with *L* before it saturates.

The sub-optimal *L̅* compared with *L** has more practical meaning. First, the derivation of *L̅* solely depends on *D* and *N* without evaluating the spectral efficiency. Second, the estimation of *L** based on *L̅* is very accurate when *D* is small, which is the typical setting. Once the *L̅* is derived, the sub-optimal bandwidth for the system can be calculated according to (49).

To implement various modules, units, and their functionalities described in the present disclosure, computer hardware platforms may be used as the hardware platform(s) for one or more of the elements described herein (e.g., the components of the system described with respect to any of FIGs. 1-49). The hardware elements, operating systems and programming languages of such computers are conventional in nature, and it is presumed that those skilled in the art are adequately familiar therewith to adapt those technologies to explore object tracking based on time-reversal technology in a rich-scattering environment as described herein. A computer with user interface elements may be used to implement a personal computer (PC) or other type of work station or terminal device, although a computer may also act as a server if appropriately programmed. It is believed that those skilled in the art are familiar with the structure, programming and general operation of such computer equipment and as a result the drawings should be self-explanatory.

The disclosed system can be realized by a specialized system having a functional block diagram illustration of a hardware platform which includes user interface elements. The computer may be a general purpose computer or a special purpose computer. Both can be used to implement a specialized system for the present teaching. This computer may be used to implement any component of the techniques of object tracking based on time-reversal technology in a rich-scattering environment, as described herein. For example, the system in FIG. 8 may be implemented on a computer, via its hardware, software program, firmware, or a combination thereof.

Hence, aspects of the methods of object tracking based on time-reversal technology in a rich-scattering environment, as outlined above, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors or the like, or associated modules thereof, such as various semiconductor memories, tape drives, disk drives and the like, which may provide storage at any time for the software programming.

All or portions of the software may at times be communicated through a network such as the Internet or various other telecommunication networks. Such communications, for example, may enable loading of the software from one computer or processor into another. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

Hence, a machine-readable medium may take many forms, including but not limited to, a tangible storage medium, a carrier wave medium or physical transmission medium. Nonvolatile storage media include, for example, optical or magnetic disks, such as any of the storage devices in any computer(s) or the like, which may be used to implement the system or any of its components as shown in the drawings. Volatile storage media include dynamic memory, such as a main memory of such a computer platform. Tangible transmission media include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a physical processor for execution.

Those skilled in the art will recognize that the present teachings are amenable to a variety of modifications and/or enhancements. For example, although the implementation of various components described above may be embodied in a hardware device, it may also be implemented as a software only solution-e.g., an installation on an existing server. In addition, the object tracking based on time-reversal technology in a rich-scattering environment as disclosed herein may be implemented as a firmware, firmware/software combination, firmware/hardware combination, or a hardware/firmware/software combination.

While the foregoing has described what are considered to constitute the present teachings and/or other examples, it is understood that various modifications may be made thereto and that the subject matter disclosed herein may be implemented in various forms and examples, and that the teachings may be applied in numerous applications, only some of which have been described herein.

## Claims

1. A method, implemented on a machine (104, 106) including at least a processor and a memory communicatively coupled with the processor for tracking a movement of an object in real-time, comprising:
obtaining an initial position of the object prior to a movement of the object;
obtaining at least one wireless signal from a multipath channel that is impacted by the movement of the object;
extracting a time series of channel state information, CSI, for the multipath channel from the at least one wireless signal;
cleaning a phase offset of each of the time series of CSI;
calculating a similarity score based on each pair of consecutive CSIs among the time series of CSI to obtain a plurality of calculated similarity scores, wherein each of the plurality of calculated similarity scores indicates a degree of similarity between a corresponding pair of CSIs;
computing an average similarity score based on the plurality of calculated similarity scores, wherein the average similarity score indicates a degree of spatial resonating decay associated with the movement of the object;
comparing the average similarity score with a reference decay curve to obtain an estimated distance to determine a distance of the movement of the object based on the time series of CSI;
estimating a direction of the movement of the object; and
determining a new position of the object after the movement based on the distance, the direction, and the initial position.

2. The method of claim 1, wherein the object is carrying at least one of:
a transmitter that transmits the at least one wireless signal;
a receiver that receives the at least one wireless signal; and
a sensor configured for direction estimation.

3. The method of claim 1, further comprising:
calculating an additional similarity score based on a first CSI and a last CSI in the time series of CSI;
comparing the additional similarity score with a pre-determined threshold;
determining the distance of the movement of the object to be zero when the additional similarity score exceeds the pre-determined threshold; and
determining the distance of the movement of the object to be the estimated distance when the additional similarity score does not exceed the predetermined threshold.

4. The method of claim 1,
wherein the similarity score is calculated based on at least one of: a TRRS, a cross-correlation, an auto-correlation, an inner product of two vectors, a similarity score, a distance score, a phase correction, a timing correction, a timing compensation, and a phase offset compensation, of a pair of CSIs.

5. The method of claim 1, wherein estimating the direction of the movement of the object comprises:
obtaining a gravity direction of the object from a first sensor,
obtaining rotational information of the object from a second sensor,
determining a coordinate rotation velocity based on the gravity direction and the rotational information,
obtaining a sensor reading interval of the second sensor,
calculating a direction change based on the coordinate rotation velocity and the sensor reading interval, and
estimating the direction of the movement based on the direction change and a previously estimated direction; and
wherein obtaining rotational information of the object from a second sensor comprises obtaining angular velocity of the object from a gyroscope.

6. The method of claim 1, wherein estimating the direction of the movement of the object comprises:
obtaining a first moving distance from a first location straightly to a second location;
obtaining a second moving distance from the second location straightly to a third location;
obtaining a third moving distance from the first location straightly to the third location, wherein at least one of the first, second and third moving distances is determined based on the time series of CSI; and
estimating the direction of the movement of the object based on the first, second and third moving distances according to trigonometry.

7. The method of claim 1, wherein estimating the direction of the movement of the object comprises:
obtaining a plurality of average decay curves of spatial resonating strength within a time window on a plurality of antennas;
determining at least one pattern based on the plurality of average decay curves; and
estimating the direction of the movement of the object based on the at least one pattern.

8. The method of claim 1, wherein:
the at least one wireless signal is received by a receiver through a network that is at least one of: Internet, an Internet-protocol network, and another multiple access network; and
the receiver is associated with a physical layer of at least one of: a wireless PAN, IEEE 802.15.1 (Bluetooth), a wireless LAN, IEEE 802.11 (Wi-Fi), a wireless MAN, IEEE 802.16 (WiMax), WiBro, HiperMAN, mobile WAN, OSM, OPRS, EDOE, HSCSD, iDEN, D-AMPS, IS-95, PDC, CSD, PHS, WiDEN, CDMA2000, UMTS, 30SM, CDMA, TDMA, FDMA, W-CDMA, HSDPA, W-CDMA, FOMA, IxEV-DO, IS-856, TD-SCDMA, OAN, UMA, HSUPA, LTE, 2.50, 30, 3.50, 3.90, 40, 50, 60, 70 and beyond, another wireless system and another mobile system.

9. A system for tracking a movement of an object in real-time, comprising:
a receiver (104, 106) configured for receiving at least one wireless signal from a multipath channel that is impacted by a movement of the object;
a sensor configured for direction estimation; a processor; and
a memory communicatively coupled with the processor, wherein the processor is configured for:
obtaining an initial position of the object prior to a movement of the object;
extracting a time series of channel state information, CSI, for the multipath channel from the at least one wireless signal;
cleaning a phase offset of each of the time series of CSI;
calculating a similarity score based on each pair of consecutive CSIs among the time series of CSI to obtain a plurality of calculated similarity scores, wherein each of the plurality of calculated similarity scores indicates a degree of similarity between a corresponding pair of CSIs;
computing an average similarity score based on the plurality of calculated similarity scores, wherein the average similarity score indicates a degree of spatial resonating decay associated with the movement of the object;
comparing the average similarity score with a reference decay curve to determine a distance of the movement of the object based on the time series of CSI;
estimating**,** using the sensor, a direction of the movement of the object; and
determining a new position of the object after the movement based on the distance, the direction and the initial position.

10. A method, implemented on a machine (104, 106) including at least a processor and a memory communicatively coupled with the processor for tracking a movement of an object in real-time, comprising:
obtaining an initial position of the object prior to a movement of the object;
obtaining at least one wireless signal from a multipath channel that is impacted by the movement of the object;
extracting a time series of channel state information, CSI, according to a sampling period for the multipath channel from the at least one wireless signal;
cleaning a phase offset of each of the time series of CSI,
calculating a similarity score between a most recent CSI in the time series of CSI and each of previous CSIs in the time series of CSI to obtain a time series of similarity scores, wherein each of the time series of similarity scores indicates a degree of similarity between the most recent CSI and a corresponding previous CSI,
determining a curve based on the time series of similarity scores,
identifying a feature point on the curve,
estimating a time period corresponding to the feature point on the curve,
estimating a speed of the movement during the time period, and
determining a distance of the movement of the object based on the speed and the sampling period;
estimating a direction of the movement of the object;
determining a new position of the object after the movement based on the distance, the direction, and the initial position.

11. The method of claim 10, wherein:
the feature point on the curve is identified based on at least one of: a first local peak on the curve, one or more other local peaks on the curve, a first local bottom on the curve, one or more other local bottoms on the curve, and a point having a pre-determined relationship with a local peak or a local bottom on the curve.

12. The method of claim 11, wherein:
the feature point on the curve is identified based on a first local peak on the curve; and
the time period corresponding to the first local peak is estimated based on a similarity score corresponding to the first local peak and two adjacent similarity scores among the time series of similarity scores.

## Patentansprüche

1. Ein Verfahren, das auf einer Maschine (104, 106) implementiert ist, die mindestens einen Prozessor und einen mit dem Prozessor kommunikativ verbundenen Speicher umfasst, zum Verfolgen einer Bewegung eines Objekts in Echtzeit, umfassend:
Ermitteln einer Ausgangsposition des Objekts vor einer Bewegung des Objekts;
Ermitteln mindestens eines Funksignals aus einem Mehrwegkanal, der durch die Bewegung des Objekts beeinflusst wird;
Extrahieren einer Zeitreihe von Kanalzustandsinformationen, CSI, für den Mehrwegkanal aus dem mindestens einen Funksignal;
Bereinigen eines Phasenversatzes jeder der Zeitreihen von CSI;
Berechnen eines Ähnlichkeitswerts auf der Grundlage jedes Paares aufeinanderfolgender CSIs innerhalb der Zeitreihen der CSI, um eine Vielzahl von berechneten Ähnlichkeitswerten zu erhalten, wobei jeder der Vielzahl von berechneten Ähnlichkeitswerten einen Ähnlichkeitsgrad zwischen einem entsprechenden Paar von CSIs anzeigt;
Berechnen eines durchschnittlichen Ähnlichkeitswerts auf der Grundlage der mehreren berechneten Ähnlichkeitswerte, wobei der durchschnittliche Ähnlichkeitswert einen Grad des räumlichen Resonanzabklingens angibt, der mit der Bewegung des Objekts verbunden ist;
Vergleichen des durchschnittlichen Ähnlichkeitswerts mit einer Referenzabklingkurve, um eine geschätzte Entfernung zu erhalten, um eine Bewegungsstrecke des Objekts auf der Grundlage der CSI-Zeitreihe zu bestimmen;
Schätzen einer Bewegungsrichtung des Objekts; und
Bestimmen einer neuen Position des Objekts nach der Bewegung auf der Grundlage der Entfernung, der Richtung und der Ausgangsposition.

2. Verfahren nach Anspruch 1, wobei das Objekt mindestens eines der folgenden Elemente mitführt:
einen Sender, der das mindestens eine Funksignal sendet;
einen Empfänger, der das mindestens eine Funksignal empfängt; und
einen Sensor, der zur Richtungsschätzung ausgelegt ist.

3. Verfahren nach Anspruch 1, das ferner umfasst:
Berechnen eines zusätzlichen Ähnlichkeitswerts auf der Grundlage eines ersten CSI und eines letzten CSI in der Zeitreihe der CSI;
Vergleichen des zusätzlichen Ähnlichkeitswerts mit einem vorgegebenen Schwellenwert;
Festlegen des Bewegungsabstands des Objekts auf Null, wenn der zusätzliche Ähnlichkeitswert den vorgegebenen Schwellenwert überschreitet; und
Bestimmen der Bewegungsentfernung des Objekts als die geschätzte Entfernung, wenn der zusätzliche Ähnlichkeitswert den vorgegebenen Schwellenwert nicht überschreitet.

4. Verfahren nach Anspruch 1,
wobei der Ähnlichkeitswert auf der Grundlage mindestens eines der folgenden Elemente berechnet wird: eines TRRS, einer Kreuzkorrelation, einer Autokorrelation, eines Skalarprodukts zweier Vektoren, eines Ähnlichkeitswerts, eines Abstandswerts, einer Phasenkorrektur, einer Zeitkorrektur, einer Zeitkompensation und einer Phasenversatzkompensation eines Paares von CSIs.

5. Verfahren nach Anspruch 1, wobei das Schätzen der Bewegungsrichtung des Objekts umfasst:
Ermitteln einer Schwerkraftrichtung des Objekts anhand eines ersten Sensors,
Ermitteln von Rotationsinformationen des Objekts anhand eines zweiten Sensors,
Ermitteln einer Koordinatenrotationsgeschwindigkeit auf der Grundlage der Schwerkraftrichtung und der Rotationsinformationen,
Ermitteln eines Sensor-Abtastintervalls des zweiten Sensors,
Berechnen einer Richtungsänderung auf der Grundlage der Koordinatenrotationsgeschwindigkeit und des Sensorabtastintervalls, und
Schätzen der Bewegungsrichtung auf der Grundlage der Richtungsänderung und einer zuvor geschätzten Richtung; und
wobei das Ermitteln von Rotationsinformationen des Objekts anhand eines zweiten Sensors das Ermitteln einer Winkelgeschwindigkeit des Objekts anhand eines Gyroskops umfasst.

6. Verfahren nach Anspruch 1, wobei das Schätzen der Bewegungsrichtung des Objekts umfasst:
Ermitteln einer ersten Bewegungsstrecke von einem ersten Ort geradlinig zu einem zweiten Ort;
Ermitteln einer zweiten Bewegungsstrecke vom zweiten Ort geradlinig zu einem dritten Ort;
Ermitteln einer dritten Bewegungsstrecke von dem ersten Ort in gerader Linie zu dem dritten Ort, wobei mindestens eine der ersten, zweiten und dritten Bewegungsstrecken auf der Grundlage der Zeitreihe von CSI bestimmt wird; und
Schätzen der Bewegungsrichtung des Objekts auf der Grundlage der ersten, zweiten und dritten Bewegungsstrecken gemäß der Trigonometrie.

7. Verfahren nach Anspruch 1, wobei das Schätzen der Bewegungsrichtung des Objekts umfasst:
Ermitteln einer Vielzahl von durchschnittlichen Abklingkurven der räumlichen Resonanzstärke innerhalb eines Zeitfensters an einer Vielzahl von Antennen;
Ermitteln mindestens eines Musters auf der Grundlage der mehreren durchschnittlichen Abklingkurven; und
Schätzen der Bewegungsrichtung des Objekts auf der Grundlage des mindestens einen Musters.

8. Verfahren nach Anspruch 1, wobei:
das mindestens eine Funksignal von einem Empfänger über zumindest eines der folgenden Netzwerke empfangen wird: das Internet, ein Internetprotokoll-Netzwerk und ein anderes Mehrfachzugriffsnetzwerk; und
der Empfänger einer physikalischen Schicht von mindestens einem der folgenden Netzwerke zugeordnet ist: einem drahtlosen PAN, IEEE 802.15.1 (Bluetooth), einem drahtlosen LAN, IEEE 802.11 (Wi-Fi), einem drahtlosen MAN, IEEE 802.16 (WiMax), WiBro, HiperMAN, mobiles WAN, OSM, OPRS, EDOE, HSCSD, iDEN, D-AMPS, IS-95, PDC, CSD, PHS, WiDEN, CDMA2000, UMTS, 30SM, CDMA, TDMA, FDMA, W-CDMA, HSDPA, W-CDMA, FOMA, IxEV-DO, IS-856, TD-SCDMA, OAN, UMA, HSUPA, LTE, 2,50, 30, 3,50, 3,90, 40, 50, 60, 70 und darüber hinaus, ein anderes Funksystem und ein anderes Mobilfunksystem.

9. Ein System zur Verfolgung einer Bewegung eines Objekts in Echtzeit, umfassend:
einen Empfänger (104, 106), der dazu eingerichtet ist, zumindest ein Funksignal von einem Mehrwegkanal zu empfangen, das durch eine Bewegung des Objekts beeinflusst wird;
einen Sensor, der zur Richtungsschätzung ausgelegt ist; einen Prozessor; und
einen Speicher, der kommunikativ mit dem Prozessor gekoppelt ist, wobei der Prozessor eingerichtet ist zum:
Ermitteln einer Ausgangsposition des Objekts vor einer Bewegung des Objekts;
Extrahieren einer Zeitreihe von Kanalzustandsinformationen, CSI, für den Mehrwegkanal aus dem mindestens einen Funksignal;
Bereinigen eines Phasenversatzes jeder der Zeitreihen von CSI;
Berechnen eines Ähnlichkeitswerts auf der Grundlage jedes Paares aufeinanderfolgender CSIs innerhalb der Zeitreihen von CSI, um eine Vielzahl von berechneten Ähnlichkeitswerten zu erhalten, wobei jeder der Vielzahl von berechneten Ähnlichkeitswerten einen Grad der Ähnlichkeit zwischen einem entsprechenden Paar von CSIs angibt;
Berechnen eines durchschnittlichen Ähnlichkeitswerts auf der Grundlage der mehreren berechneten Ähnlichkeitswerte, wobei der durchschnittliche Ähnlichkeitswert einen Grad des räumlichen Resonanzabklingens, der mit der Bewegung des Objekts verbunden ist;
Vergleichen des durchschnittlichen Ähnlichkeitswerts mit einer Referenzabklingkurve, um die Entfernung der Bewegung des Objekts auf der Grundlage der Zeitreihe der CSI zu bestimmen;
Schätzen einer Bewegungsrichtung des Objekts unter Verwendung des Sensors; und
Bestimmen einer neuen Position des Objekts nach der Bewegung auf der Grundlage der Entfernung, der Richtung und der Ausgangsposition.

10. Ein Verfahren, das auf einer Maschine (104, 106) implementiert ist, die mindestens einen Prozessor und einen mit dem Prozessor kommunikativ gekoppelten Speicher, zur Verfolgung einer Bewegung eines Objekts in Echtzeit umfasst, aufweisend:
Ermitteln einer Ausgangsposition des Objekts vor einer Bewegung des Objekts;
Ermitteln mindestens eines Funksignals aus einem Mehrwegkanal, der durch die Bewegung des Objekts beeinflusst ist;
Extrahieren einer Zeitreihe von Kanalzustandsinformationen, CSI, entsprechend einer Abtastperiode für den Mehrwegkanal aus dem mindestens einen Funksignal;
Bereinigen eines Phasenversatzes jeder der Zeitreihen von CSI,
Berechnen eines Ähnlichkeitswerts zwischen der aktuellsten CSI in der Zeitreihe der CSI und jedem der vorhergehenden CSI-Werte in der CSI-Zeitreihe, um eine Zeitreihe von Ähnlichkeitswerten zu erhalten, wobei jede der Zeitreihen von Ähnlichkeitswerten einen Ähnlichkeitsgrad zwischen dem aktuellsten CSI-Wert und einem entsprechenden vorhergehenden CSI-Wert angibt,
Ermitteln einer Kurve auf der Grundlage der Zeitreihe der Ähnlichkeitswerte,
Identifizieren eines Merkmalspunkts auf der Kurve,
Schätzen eines Zeitraums, der dem Merkmalspunkt auf der Kurve entspricht,
Schätzen einer Bewegungsgeschwindigkeit während des Zeitraums, und
Bestimmen einer Bewegungsstrecke des Objekts auf der Grundlage der Geschwindigkeit und des Abtastzeitraums;
Schätzen einer Bewegungsrichtung des Objekts;
Ermitteln einer neuen Position des Objekts nach der Bewegung auf der Grundlage der Strecke, der Richtung und der Ausgangsposition.

11. Das Verfahren nach Anspruch 10, wobei:
der Merkmalspunkt auf der Kurve auf der Grundlage mindestens eines der folgenden Merkmale identifiziert wird: eines ersten lokalen Maximalwerts auf der Kurve, einem oder mehreren weiteren lokalen Maximalwerten auf der Kurve, einem ersten lokalen Tiefpunkt auf der Kurve, einem oder mehrerer weiterer lokaler Tiefpunkte auf der Kurve, und einem Punkt, der eine vorbestimmte Beziehung zu einem lokalen Maximalwert oder einem lokalen Tiefpunkt auf der Kurve aufweist.

12. Verfahren nach Anspruch 11, wobei:
der Merkmalspunkt auf der Kurve anhand eines ersten lokalen Maximalwerts auf der Kurve identifiziert wird; und
der dem ersten lokalen Maximalwert entsprechende Zeitraum auf der Grundlage eines Ähnlichkeitswerts, der dem ersten lokalen Maximalwert entspricht, sowie zweier benachbarter Ähnlichkeitswerte aus der Zeitreihe der Ähnlichkeitswerte geschätzt wird.

## Revendications

1. Procédé, mis en œuvre sur une machine (104, 106) comprenant au moins un processeur et une mémoire couplée de manière communicative au processeur, destiné à suivre le mouvement d'un objet en temps réel, comprenant :
l'obtention d'une position initiale de l'objet avant un déplacement de celui-ci ;
l'obtention d'au moins un signal sans fil provenant d'un canal à trajets multiples affecté par le mouvement de l'objet ;
l'extraction d'une série temporelle d'informations d'état de canal, CSI, pour le canal à trajets multiples à partir dudit au moins un signal sans fil ;
corriger le décalage de phase de chacune des séries chronologiques de CSI ;
calculer un score de similarité sur la base de chaque paire de CSI consécutives parmi les séries chronologiques de CSI afin d'obtenir une pluralité de scores de similarité calculés, dans laquelle chacun des scores de similarité calculés de la pluralité indique un degré de similarité entre un paire correspondante de CSI ;
calculer un score de similarité moyen à partir de la pluralité de scores de similarité calculés, le score de similarité moyen indiquant un degré de décroissance de résonance spatiale associé au mouvement de l'objet ;
comparer le score de similarité moyen à une courbe de décroissance de référence pour obtenir une distance estimée afin de déterminer la distance parcourue par l'objet sur la base de la série chronologique de CSI ;
estimer une direction du déplacement de l'objet ; et
déterminer une nouvelle position de l'objet après le déplacement sur la base de la distance, de la direction et de la position initiale.

2. Procédé selon la revendication 1, dans lequel l'objet est équipé d'au moins l'un des éléments suivants :
un émetteur qui émet le ou les signaux sans fil ;
un récepteur qui reçoit ledit au moins un signal sans fil ; et
un capteur configuré pour l'estimation de la direction.

3. Procédé selon la revendication 1, comprenant en outre :
le calcul d'un score de similarité supplémentaire sur la base d'un premier CSI et d'un dernier CSI dans la série chronologique de CSI ;
la comparaison du score de similarité supplémentaire à un seuil prédéterminé ;
déterminer que la distance du mouvement de l'objet est égale à zéro lorsque le score de similarité supplémentaire dépasse le seuil prédéterminé ; et
déterminer que la distance de déplacement de l'objet correspond à la distance estimée lorsque le score de similarité supplémentaire ne dépasse pas le seuil prédéterminé.

4. Procédé selon la revendication 1,
dans lequel le score de similarité est calculé sur la base d'au moins l'un des éléments suivants : un TRRS, une corrélation croisée, une autocorrélation, un produit scalaire de deux vecteurs, un score de similarité, un score de distance, une correction de phase, une correction de synchronisation, une compensation de synchronisation et une compensation de décalage de phase, d'une paire de CSI.

5. Procédé selon la revendication 1, dans lequel l'estimation de la direction du mouvement de l'objet comprend :
l'obtention d'une direction de gravité de l'objet à partir d'un premier capteur,
l'obtention d'informations de rotation de l'objet à partir d'un deuxième capteur,
déterminer une vitesse de rotation des coordonnées sur la base de la direction de la gravité et des informations de rotation,
l'obtention d'un intervalle d'échantillonnage du deuxième capteur,
calculer un changement de direction en fonction de la vitesse de rotation des coordonnées et de l'intervalle d'échantillonnage du capteur, et
estimer la direction du mouvement en fonction du changement de direction et d'un direction précédemment estimée ; et
dans lequel l'obtention d'informations de rotation de l'objet à partir d'un deuxième capteur comprend l'obtention de la vitesse angulaire de l'objet à partir d'un gyroscope.

6. Procédé selon la revendication 1, dans lequel l'estimation de la direction du mouvement de l'objet comprend :
l'obtention d'une première distance de déplacement entre un premier emplacement et un deuxième emplacement en ligne droite ;
l'obtention d'une deuxième distance de déplacement entre le deuxième emplacement et un troisième ;
l'obtention d'une troisième distance de déplacement en ligne droite entre le premier emplacement et le troisième, dans lequel au moins l'une des première, deuxième et troisième distances de déplacement est déterminée sur la base de la série chronologique de CSI ; et
estimer la direction du mouvement de l'objet sur la base des première, deuxième et troisième distances de déplacement selon des principes trigonométriques.

7. Procédé selon la revendication 1, dans lequel l'estimation de la direction du mouvement de l'objet comprend :
l'obtention d'une pluralité de courbes de décroissance moyennes de l'intensité de résonance spatiale au sein d'une fenêtre temporelle sur une pluralité d'antennes ;
déterminer au moins un profil sur la base de la pluralité de courbes de décroissance moyennes ; et
estimer la direction du mouvement de l'objet à partir de ce au moins un profil.

8. Procédé selon la revendication 1, dans lequel :
le au moins un signal sans fil est reçu par un récepteur via un réseau qui se trouve à au moins l'un des réseaux suivants : Internet, un réseau utilisant le protocole Internet et un autre réseau à accès multiple ; et
le récepteur est associé à une couche physique d'au moins l'un des réseaux suivants : un réseau PAN sans fil, IEEE 802.15.1 (Bluetooth), un réseau LAN sans fil, IEEE 802.11 (Wi-Fi), un réseau MAN sans fil, IEEE 802.16 (WiMax), WiBro, HiperMAN, un réseau WAN mobile, OSM, OPRS, EDOE, HSCSD, iDEN, D-AMPS, IS-95, PDC, CSD, PHS, WiDEN, CDMA2000, UMTS, 30SM, CDMA, TDMA, FDMA, W-CDMA, HSDPA, W-CDMA, FOMA, IxEV-DO, IS-856, TD-SCDMA, OAN, UMA, HSUPA, LTE, 2,50, 30, 3,50, 3,90, 40, 50, 60, 70 et au-delà, un autre système sans fil et un autre système mobile.

9. Système permettant de suivre en temps réel le déplacement d'un objet, comprenant :
un récepteur (104, 106) configuré pour recevoir au moins un signal sans fil provenant d'un canal à trajets multiples qui est affecté par un mouvement de l'objet ;
un capteur configuré pour l'estimation de la direction; un processeur ; et
une mémoire couplée de manière communicative au processeur, dans lequel le processeur est configuré pour :
obtenir une position initiale de l'objet avant un déplacement de celui-ci ;
extraire une série temporelle d'informations d'état de canal, CSI, pour le canal à trajets multiples à partir dudit au moins un signal sans fil ;
corriger le décalage de phase de chacune des séries chronologiques de CSI ;
calculer un score de similarité basé sur chaque paire de CSI consécutives parmi les séries chronologiques de CSI afin d'obtenir une pluralité de scores de similarité calculés, chacun de ces scores indiquant un degré de similarité entre une paire correspondante de CSI ;
calculer un score de similarité moyen à partir de la pluralité de scores de similarité calculés, le score de similarité moyen indiquant un degré de décroissance de la résonance spatiale associé au mouvement de l'objet ;
comparaison du score de similarité moyen avec une courbe de décroissance de référence afin de déterminer la distance parcourue par l'objet sur la base de la série chronologique de CSI ;
estimation, à l'aide du capteur, une direction du mouvement de l'objet ; et déterminer une nouvelle position de l'objet après le mouvement en fonction de la distance, de la direction et de la position initiale.

10. Procédé, mis en œuvre sur une machine (104, 106) comprenant au moins un processeur et une mémoire couplée de manière communicative au processeur pour suivre en temps réel le déplacement d'un objet, comprenant :
l'obtention d'une position initiale de l'objet avant un déplacement de celui-ci ;
l'obtention d'au moins un signal sans fil provenant d'un canal à trajets multiples soumis à le mouvement de l'objet ;
l'extraction, à partir dudit au moins un signal sans fil, d'une série temporelle d'informations d'état de canal, CSI, selon une période d'échantillonnage pour le canal à trajets multiples ;
le nettoyage d'un décalage de phase de chacune des séries chronologiques de CSI,
calculer un score de similarité entre la CSI la plus récente de la série chronologique de CSI et chacune des CSI précédentes de la série chronologique de CSI afin d'obtenir une série chronologique de scores de similarité, dans laquelle chacune des séries chronologiques de scores de similarité indique un degré de similarité entre la CSI la plus récente et une CSI précédente correspondante,
déterminer une courbe sur la base de la série chronologique de scores de similarité,
identifier un point caractéristique sur la courbe,
estimer une période correspondant au point caractéristique sur la courbe,
estimer une vitesse de déplacement pendant cette période, et
déterminer une distance de déplacement de l'objet sur la base de la vitesse et de la période d'échantillonnage ;
estimer une direction du mouvement de l'objet ;
déterminer une nouvelle position de l'objet après le déplacement en fonction de la distance, de la direction et de la position initiale.

11. Procédé selon la revendication 10, dans laquelle :
le point caractéristique sur la courbe est identifié sur la base d'au moins l'un des éléments suivants : un premier pic local sur la courbe, un ou plusieurs autres pics locaux sur la courbe, un premier creux local sur la courbe, un ou plusieurs autres creux locaux sur la courbe, et un point présentant une relation prédéterminée avec un pic local ou un creux local sur la courbe.

12. Procédé selon la revendication 11, dans laquelle :
le point caractéristique sur la courbe est identifié sur la base d'un premier pic local sur la courbe ; et
la période correspondant au premier pic local est estimée sur la base d'un score de similarité correspondant au premier pic local et de deux scores de similarité adjacents parmi la série chronologique de scores de similarité.
